# EUROPEAN PATENT APPLICATION

(11) **EP 4 700 034 A1**
(43) Date of publication of application: **25.02.2026**
(21) Application number: 24382918.1
(22) Date of filing: 19.08.2024
(51) Int. Cl.: C07F 15/00, A61P 35/00, A61K 41/00, A61K 45/06, G01N 33/533

(54) **METAL COMPLEX COMPOUNDS, CONJUGATES AND CONJUGATABLE COMPOUNDS THEREFROM AS WELL AS USES THEREOF AND PROCESSES OF PRODUCING THE SAME**

(71) Applicant: Universitat de Barcelona, 08007 Barcelona (ES)
(72) Inventor: MARCHÁN SANCHO, Vicente, 08028 Barcelona (ES); IZQUIERDO GARCÍA, Eduardo, 08028 Barcelona (ES); ABAD MONTERO, Diego, 08028 Barcelona (ES)
(74) Representative: Hoffmann Eitle

(57) **Abstract**

The present invention relates to new metal complex compounds and their conjugates to tumor-targeting moieties useful as photosensitizers (PS) for photodynamic therapy (PDT), and to new intermediate ligand compounds useful in the preparation of the metal complex compounds.

## Description

### TECHNICAL FIELD

The present invention relates to new metal complex compounds and their conjugates to tumor-targeting moieties useful as photosensitizers (PS) for photodynamic therapy (PDT), and to new intermediate ligand compounds useful in the preparation of the metal complex compounds.

### Background art

A common feature amongst some of the most aggressive solid tumors, including glioblastoma, uveal melanoma, pancreatic, colorectal and breast cancers, is a low oxygen level, a phenomenon that is known as hypoxia. In contrast to normal tissues where the oxygen pressure is usually above 40 mm Hg, the typical oxygen pressure level in the tumor hypoxic area is below 10 mm Hg, which is a result of the rapid tumor cell proliferation and abnormal tumor vasculature. Hypoxia is known to promote tumor angiogenesis, metastasis, and resistance to conventional anticancer therapies such as chemotherapy, radiotherapy and immunotherapy, and it has been shown to correlate with poor prognosis of cancer patients and/or recurrence of the disease.

Glioblastoma is the most common and malignant primary brain tumor in adults, whose standard of care consists of 5-ALA-guided surgery owing to its preferential conversion to Protoporphyrin IX (PpIX) in glioblastoma cells that facilitates intraoperative fluorescence-assisted resection, in combination with radiotherapy and chemotherapy with temozolomide. Unfortunately, despite advances in treatments, prognosis of this disease remains bleak, with a median overall survival rate of 12 to 15 months after being diagnosed (only about 5% of patients survive five years post diagnosis) and, for this reason, glioblastoma as well as other hypoxic tumors are considered unmet medical needs due to the lack of approved drug therapies for the first line treatment. Therefore, finding strategies to overcome hypoxia is paramount in the development of new anticancer drugs.

Photodynamic therapy (PDT) is a clinically-approved technique for the destruction of tumors and/or tumor vasculature, based on the use of a light-responsive drug, referred to as photosensitizer (PS). PDT can also be applied to treat other medical conditions such as actinic keratosis, age-related macular degeneration and some fungal and microbial infections. PDT is delivered in a two-step procedure consisting of local or systemic administration of the PS, at a nontoxic dose, followed by light activation directly in the tumor site to trigger the generation of a variety of highly cytotoxic reactive oxygen species (ROS) that induce tumor cell death. In general, PSs can operate through two main processes: in the Type II mechanism singlet oxygen is sensitized through an energy-transfer process from the excited triplet state, whereas the Type I PDT mechanism is based on electron transfer reactions that generate a variety of different ROS such as superoxide, hydroxyl or hydroperoxyl radicals. While the effectiveness of the Type II mechanism relies heavily on the surrounding oxygen concentration, the Type I mechanism can operate even at low oxygen levels, which offers a good opportunity to address the hypoxia problem in cancer therapy.

Compared to other conventional protocols, PDT exhibits several advantages to treat cancer in a more efficient and selective manner due to the spatiotemporal control provided by light and its noninvasive properties. However, PDT still faces great challenges to become a widespread modality for the treatment of any type of cancer, especially for the treatment of deep-seated hypoxic tumors such as glioblastoma. To date, current marketed PSs based on the tetrapyrrolic scaffold share three main drawbacks: i) dark toxicity, which leads to toxic side effects and limits the dose that can be administered to patients; ii) reduced efficacy in the hypoxic tumor microenvironment; and iii) activation with short wavelengths, which limits tissue penetration and difficult access to large solid tumors. In addition, most PSs based on porphyrins, chlorins and phthalocyanines exhibit several other problems including poor aqueous solubility and prolonged skin photosensitivity, and require of tedious synthetic procedures that generate mixtures of compounds.

Photofrin^{®} and PpIX, both with tetrapyrrolic ring systems, have been approved worldwide for certain cancers. While these two compounds set the standard for PDT, there is ongoing interest in developing new PSs that address some of the limitations of current PSs on clinical use mentioned above. Aqueous-soluble PSs that are prepared in high purity from straightforward syntheses are also highly desirable. Certain metal complex compounds have much to offer in this respect. It is known, that some PSs containing metal ions have advanced to clinical studies, the most promising being padeliporfin (also known as TOOKAD^{®} Soluble and WST11) and TLD-1433. Padeliporfin is a tetrapyrrolic Pd(II) complex compound used as PS in PDT to treat low-risk prostate cancer with vascular-targeted PDT. TLD-1433 is the first Ru(ll) polypyridyl complex compound used as PS in PDT clinical studies to treat nonmuscle invasive bladder cancer. TLD-1433 is a thiophene-containing complex compound disclosed in a family of patent documents by S.A. McFarland with a priority date of 2012. This patent family includes some granted patents, such as EP 2 854 948 B1 and US 9,676,806 B2, both of them claiming PSs which are thiophene-containing complex compounds of several metals (Mn, Mo, Re, Fe, Ru, Os, Co, Rh, Ir, Ni, Pt, and Cu).

Therefore, there is ongoing interest in developing new PSs based on metal complex compounds for PDT which address some of the limitations of current marketed PSs, particularly with operability within the far-red and near infrared region (NIR) of the electromagnetic spectrum and under hypoxic conditions. Although an important attribute of PDT is that cytotoxicity is confined to regions where the PS and light overlap spatiotemporally, PSs exhibiting cancer cell specificity offer an additional advantage to minimize toxic side effects that can occur upon accumulation in surrounding healthy tissues. In this context, PSs conjugated to tumor targeting moieties (TTM) or biomolecules whose receptors are overexpressed in cancer cells compared to healthy cells offer great potential in PDT.

### Summary of the invention

The present invention refers, among other aspects, to conjugatable derivatives of metal complex compounds of formulae I and/or IV. In particular, the present invention provides metal complex compounds of formulae VI, including stereoisomers and E/Z isomers: 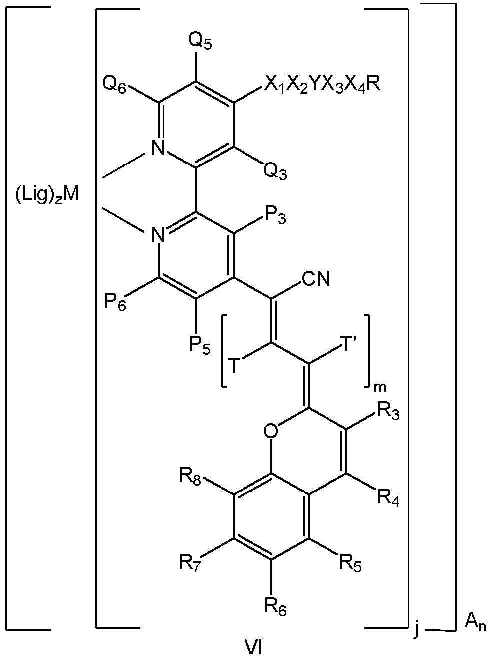
wherein M is a metal cation, and the metal cation M is Ru²⁺;
wherein m = 0, 1, 2, 3 or 4
wherein A is an anion from a pharmaceutically acceptable acid; n being an integer or fractional number whereby the compound of formula VI is electrically neutral; j = 1, 2, or 3; and z = 0, 1, or 2; on the proviso that j + z = 3;
wherein
   X₁, X₂, X₃, X₄ are each one a radical independently selected from the group consisting of H, Ph, (CH₂)ₙ, (O-CH₂-CH₂)ₙ, (CH₂-CH₂-O)ₙ n=1-200
   Y = F, Cl, Br, I, O, S, S-S, NH, C(=O), CH(=O), OC(=O), C(=O)O, NHC(=O),
   C(=O)NH, CN, SC(=O), C(=O)S, NHC(=O)O, OC(=O)NH, NHC(=O)NH,
   C(=O)NH-NH, C(=O)NH-N=CH
   R = N₃, 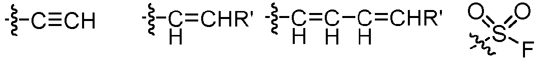 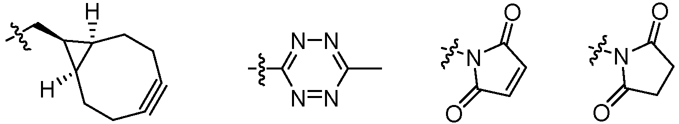 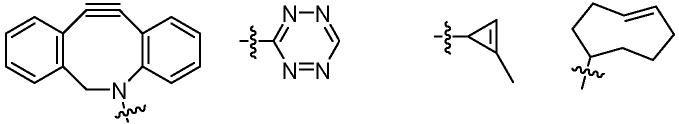
wherein any one of X₁, X₂, X₃, X₄, Y and R can be optionally absent; preferably, wherein any one of X₁, X₂, X₃, X₄ , and Y can be optionally absent thus leaving radical R as mandatory for bioconjugation reactions such as any biorthogonal reaction making use of radical R, including click chemistry; also preferably any one of X₁, X₂, X₃, X₄, Y and R can be optionally absent with the proviso that, when R is absent, at least one of X₁, X₂, X₃, X₄ , or Y is present so to allow the formation of conjugates by, for example, amide, ester, thioester, carbonate, thiocarbonate, hydrazone, oxyme, carbamate and urea bond formation, as well as by Michael addition reactions;
wherein radicals P₃ , P₅ , P₆ , Q₃ , Q₅ , and Q₆, at each occurrence are each one a radical independently selected from the group consisting of: H , (C1-C3)-alkyl , (C3-C6)-cycloalkyl , CH=CHR' , CF₃, CHF₂, CH₂F, CF₂CF₃ , F , CI , Br, I , OR' , C(=O)OR' , O(C=O)R' , C(=O)NR', NR'C(=O)R" , NR'R" , CN , NO₂ , phenyl, mono-substituted phenyl, di-substituted phenyl, and tri-substituted phenyl; pyridine, mono-substituted pyridine, di-substituted pyridine, and tri-substituted pyridine; wherein substituents on the phenyl and pyridine rings are attached to any of the possible substitution positions, and they are independently selected from the group consisting of: F , CI , Br, I , NO₂ , (C1-C3)-alkyl, OH , O[(C1-C3)-alkyl] , CN, CF₃, CHF₂, CH₂F , CF₂CF₃, NH₂, NH[(C1-C3)-alkyl] , and N[(C1-C3)-alkyl]₂; and wherein Q₃ and P₃ can form a C=C bond when Q₃ and P₃ are both CH;
wherein Lig at each occurrence is a bidentate ligand independently selected from the group consisting of ligands of accompanying formulae: Lig 1, Lig 2, Lig 3, Lig 4, Lig 5, Lig 6, Lig 7, Lig 8, Lig 9, Lig 10, Lig 11, Lig 12, and Lig 13; 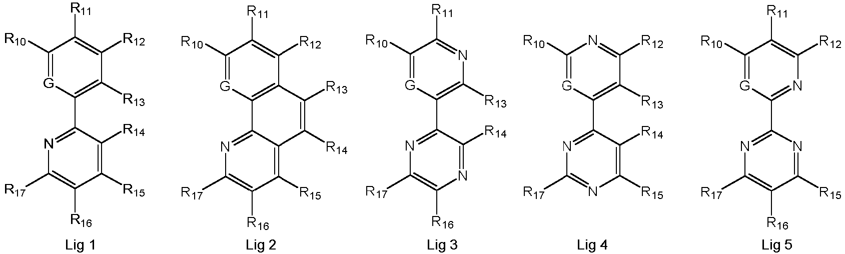 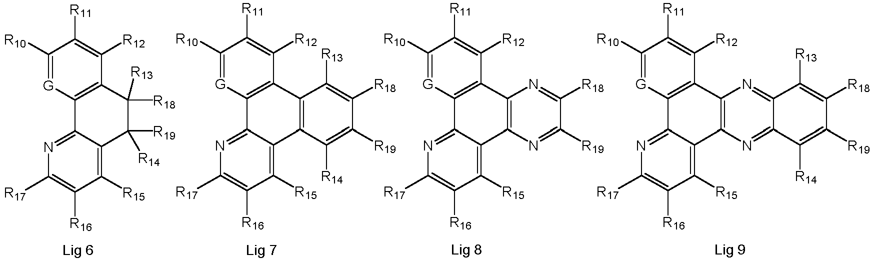 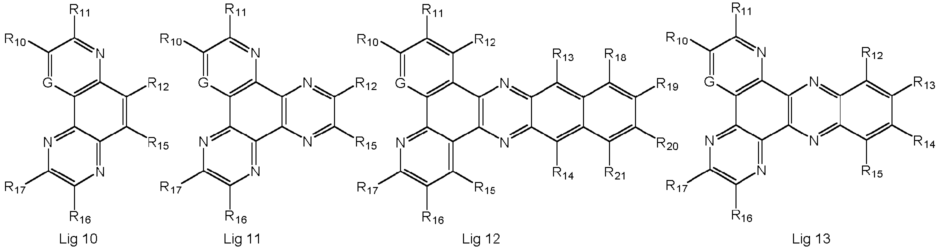
wherein G is either an N atom, or a C atom with one negative charge that is formed *in situ* from a C-H when a H⁺ is lost in the formation of the carbon-metal bond; and wherein R₁₀ , R₁₁ , R₁₂ , R₁₃, R₁₄ , R₁₅ , R₁₆ , R₁₇ , R₁₈ , R₁₉ , R₂₀ and R₂₁ at each occurrence is each one a radical independently selected from the group consisting of: H , (C1-C3)-alkyl , (C3-C6)-cycloalkyl , CH=CHR' , CF₃, CHF₂, CH₂F, CF₂CF₃ , F , CI , Br, I , OR' , C(=O)OR' , O(C=O)R' , C(=O)NR', NR'C(=O)R" , NR'R" , CN , NOz , phenyl, mono-substituted phenyl, di-substituted phenyl, and tri-substituted phenyl; pyridine, mono-substituted pyridine, di-substituted pyridine, and tri-substituted pyridine; wherein substituents on the phenyl and pyridine rings are attached to any of the possible substitution positions, and they are independently selected from the group consisting of: F , CI , Br, I , NO₂ , (C1-C3)-alkyl , OH , O[(C1-C3)-alkyl] , CN , CF₃, CHF₂, CH₂F , CF₂CF₃, NH₂, NH[(C1-C3)-alkyl], and N[(C1-C3)-alkyl]₂;
wherein R₃, R₅ , R₆ , and R₈ at each occurrence are each one a radical independently selected from the group consisting of: H, (C1-C3)-alkyl , (C3-C6)-cycloalkyl , CH=CHR' , CF₃, CHF₂, CH₂F, CF₂CF₃ , F , Cl , Br, I , OR' , C(=O)OR' , O(C=O)R' , C(=O)NR', NR'C(=O)R" , NR'R" , CN , NO₂ , phenyl, mono-substituted phenyl, di-substituted phenyl, and tri-substituted phenyl; pyridine, mono-substituted pyridine, di-substituted pyridine, and tri-substituted pyridine; wherein substituents on the phenyl and pyridine rings are attached to any of the possible substitution positions, and they are independently selected from the group consisting of: F , CI , Br, I , NO₂ , (C1-C3)-alkyl , OH , O[(C1-C3)-alkyl] , CN , CF₃, CHF₂, CH₂F , CF₂CF₃, NH₂ , NH[(C1-C3)-alkyl], and N[(C1-C3)-alkyl]₂;
wherein radical R₇ is H, NO₂ , OR' , NR'R" , N(CH₂COOH)₂ , N(CH₂CH₂SO₃H)₂ , N(CH₂CONHCH₂CH₂NMe₂)₂ , or: an unsubstituted, a mono-(R')-substituted, a di-(R',R")-substituted, or a tri-(R',R",R‴)-substituted radical of each one of the heterocyclic radicals 1-aziridinyl, 1-azetidinyl, 1-pyrrolidinyl, 1-piperidinyl , 4-morpholinyl , or 1-piperazinyl; alternatively R₆ , R₇ and R₈ form together the bicycle system of the accompanying formula, wherein V₁ , V₂ , V₃ , V₄, V₅ , V₆ , V₇ , V₈ , V₉ , V₁₀ , V₁₁ , and V₁₂ are each one a radical independently selected from the group consisting of H and (C1-C3)-alkyl; 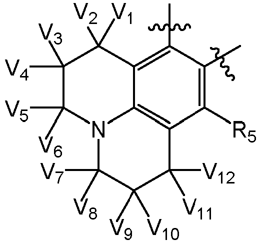
wherein radical R₄ is H , (C1-C3)-alkyl , (C3-C6)-cycloalkyl , CF₃, CHF₂, CH₂F, CF₂CF₃ , F , CI , Br , I , OR' , C(=O)OR' , O(C=O)R' , C(=O)NR' , NR'C(=O)R" , NR'R" , phenyl, a mono-(R')-substituted phenyl, a di-(R', R")-substituted phenyl, a tri-(R', R", R‴)-substituted phenyl, or a radical CE₁E₂E₃ ; wherein: E₁ and E₂ are radicals independently selected from the group consisting of: H , F , CI , Br, I , OR` , NR'R" , NO₂ , (C1-C3)-alkyl , phenyl, and a mono-(R')-substituted phenyl; and E₃ is OH, O[(C1-C3)-alkyl] , or a radical of the accompanying formula wherein p = 0, 1, 2, 3 or 4; and D₁ and D₂ at each occurrence are radicals independently selected from the group consisting of H , O[CH₂]_{q}-X , S[CH₂]_{q}-X , and N[[CH₂]_{q}-X]₂ , being q = 1 or 2, and X = F, Cl, Br or I. 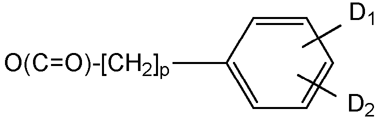
or a radical of the accompanying formula wherein p = 1, 2, 3 or 4; and D3 is a radical independently selected from the group consisting of H , H(C=O) , [(C1-C3)-alkyl](C=O), (OH)₂P(=O)O-CH₂-O(C=O) , (PhO)(OH)P(=O) 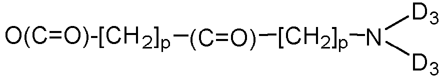
wherein radicals T and T' at each occurrence are each one a radical independently selected from the group consisting of: H and (C1-C3)-alkyl;
wherein when m ≥ 1 (such 1, 2, 3 or more), if radical R₃ is CH₂ it can preferably form a 5 or a 6-membered ring with the T' radical closest to the coumarin backbone provided that T' = CH₂; and
wherein R', R" and R‴ at each one of the above-mentioned occurrences are each one a radical independently selected from the group consisting of: H , F , CI , Br, I , NO₂ , (C1-C3)-alkyl, OH, O[(C1-C3)-alkyl] , NH₂ , NH[(C1-C3)-alkyl], and N[(C1-C3)-alkyl]₂.

Preferably, the complex compound is of formulae VII: 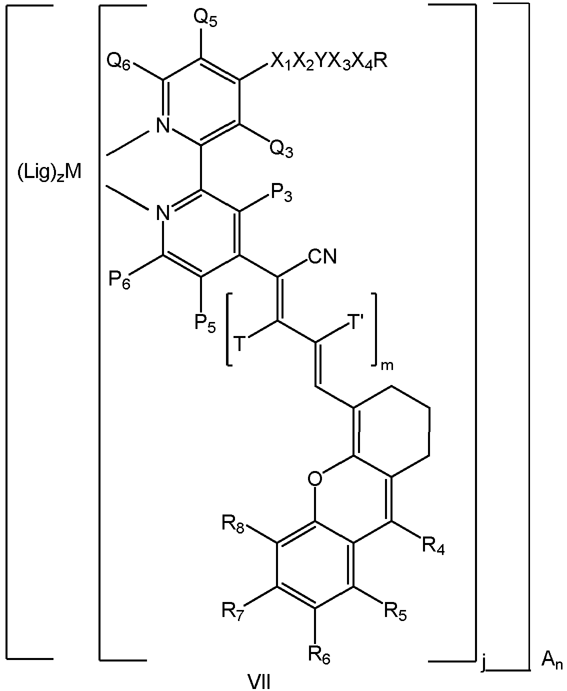 wherein M, A, n, j, z, m, X₁, X₂, X₃, X₄, Y, R, P₃ , P₅ , P₆ , Q₃ , Q₅ , Q₆, T, T', Lig and R₄ to R₈ are as defined above.

More preferably, the compound is selected from the list consisting of: 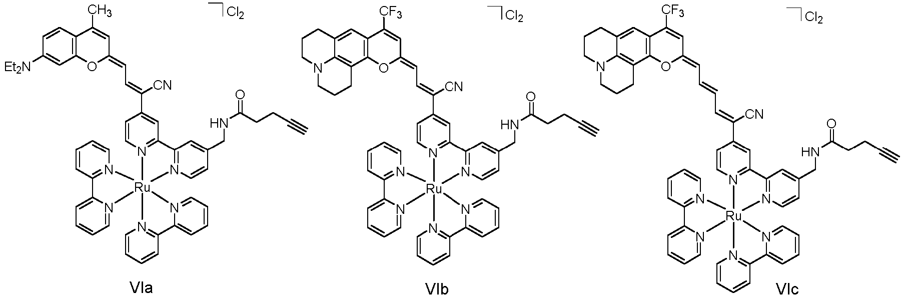 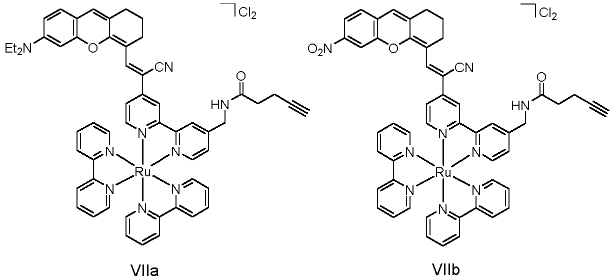 including stereoisomers and E/Z isomers thereof.

In addition, the present invention further provides compounds of formula VI or VII conjugated to TTMs (tumour-targeting moieties), in particular, complex compounds of formula XVI or XVII including stereoisomers and E/Z isomers: 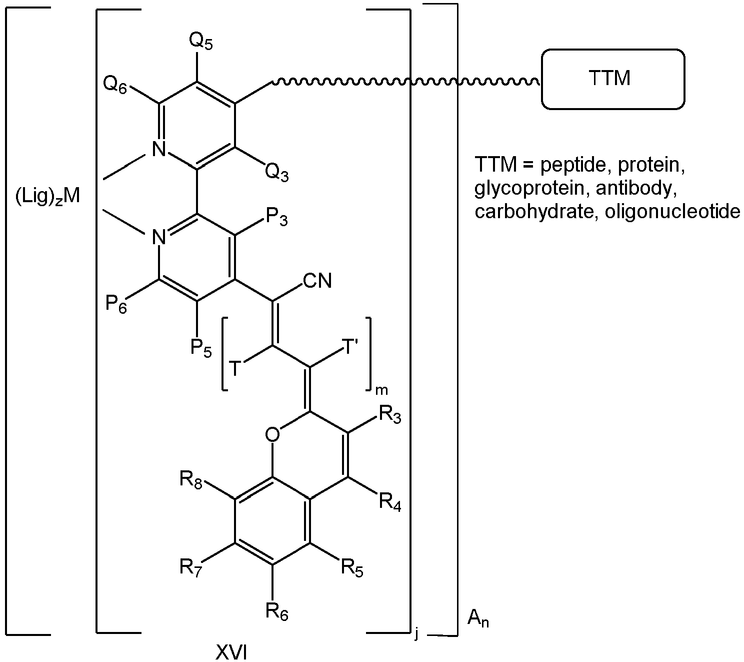 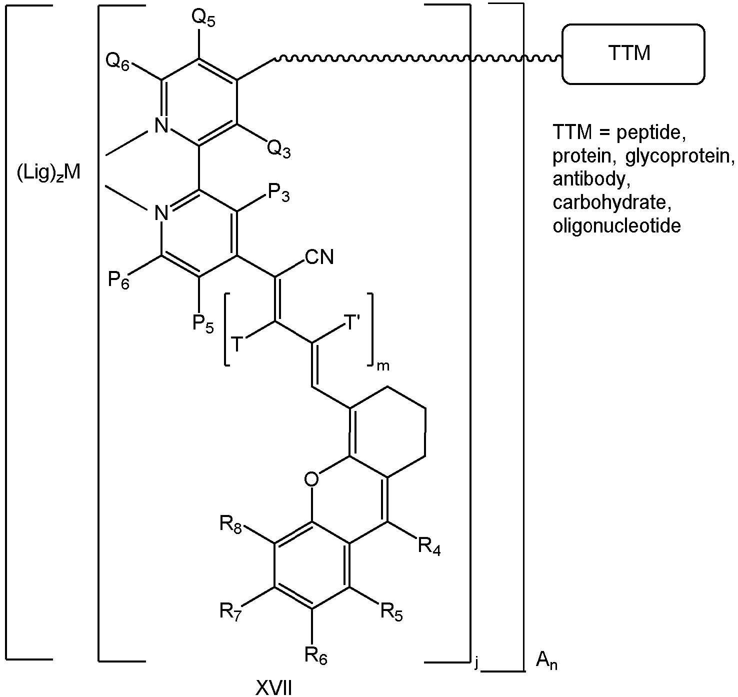 wherein Lig, M, A, n, j, z, m, P₃ , P₅ , P₆ , Q₃ , Q₅ , Q₆, T, T', R₃ (the definition of R₃ is only applicable to formula XVI as formula XVII does not comprise this specific radical), and R₄ to R₈ are as defined above, and wherein TTM is a tumour-targeting moiety or biomolecule selected from the list consisting of a peptide, protein, glycoprotein, antibody, carbohydrate, oligonucleotide or analogues comprising a functional group selected from the list consisting of azide, alkene, 1,4-diene, linear or cyclic alkyne, maleimide, succinimide, tetrazine, ciclopropene, hydrazine, NH₂, COOH, CHO, or SH that can react with the complementary group of the chemical moiety X₁X₂YX₃X₄R of any one of metal complex compounds of formula VI or VII, through any suitable bioconjugation reaction including amide, ester, thioester, carbonate, thiocarbonate, hydrazone, oxyme, carbamate and urea bond formation, Michael additions and cycloaddition reactions. Preferably the bioconjugation reaction is a biorthogonal reaction catalysed or not by copper (click chemistry reaction). The functional group complementary to the reactive group of the chemical moiety X₁X₂YX₃X₄R will be incorporated into the TTM or biomolecule in a suitable position not interfering with its receptor-binding capacity (e.g. in the case of a peptide, protein, glycoprotein or antibody, either at the N- or C-terminal end of the amino acid sequence or through the side chain of a specific amino acid such as aspartic acid, glutamic acid, lysine and cysteine, although non-natural amino acids and linkers may be used for this purpose). It is apparent to the skilled person that the TTM is bound to the structure of formulae VI or VII through its functional group complementary to the reactive group of the chemical moiety X₁X₂YX₃X₄R of chemical formulae VI or VII (although this specific binding structure is not specifically drawn in any of chemical structures XVI or XVII).

Preferably, the complex compound of formula XVI or XVII is selected from the list consisting of:
a. Ru-RGD conjugate (XVIa) 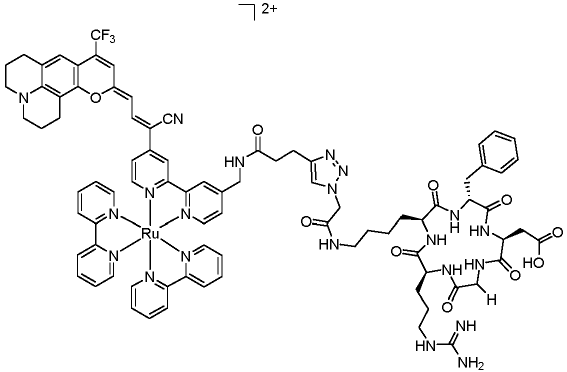
b. Ru-octreotide conjugate (XVIb) 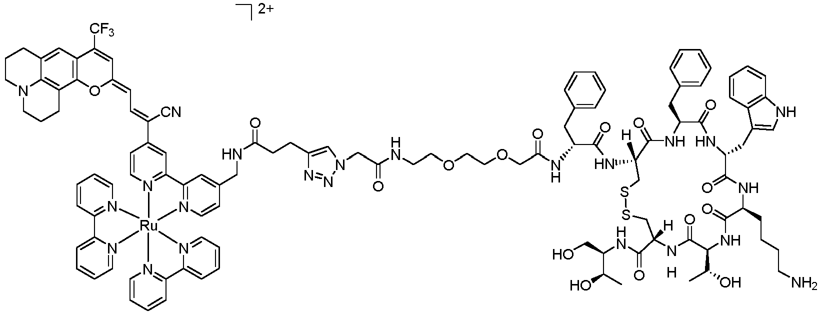
c. Ru-L1MC1R conjugate (XVIc) 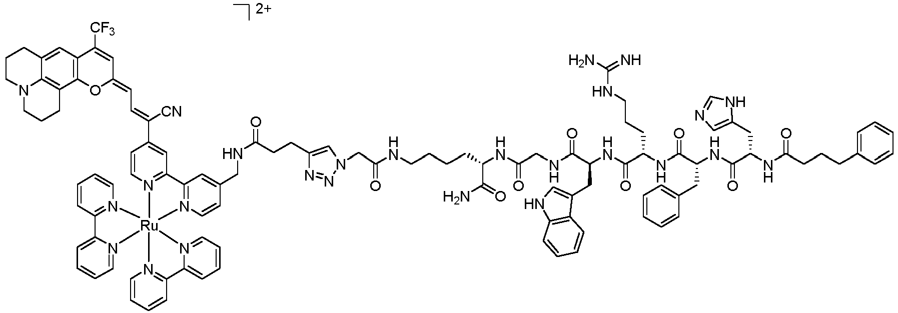
d. Ru-L2MC1R conjugate (XVId) 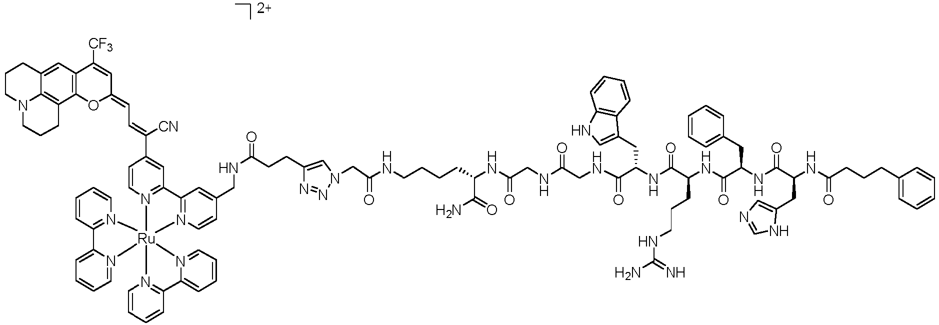
e. Ru-L3MC1R conjugate (XVIe)

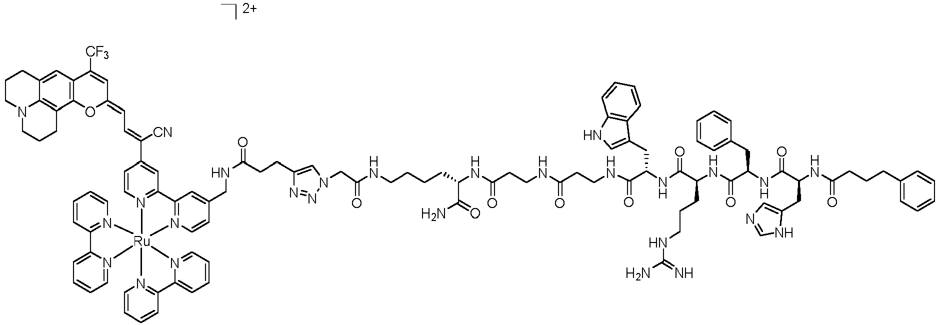

In addition, the present invention provides processes of manufacturing or synthesizing any of compounds VI, VII, XVI or XVII, as well as intermediate compounds that participate in such synthesis.

Moreover, the present invention provides pharmaceutical compositions comprising a therapeutically effective amount of a compound of formulae VI, VII, XVI or XVII as defined above, together with appropriate amounts of excipients, carriers or vehicles. Preferably, the present invention provides for a compound of formulae VI, VII, XVI or XVII as defined above, for use in therapy, preferably for use in human therapy, more preferably for use as photosensitizer in photodynamic therapy of a human condition, such as cancer, a skin condition, a fungal infection, or a microbial infection still more preferably for use as a photosensitizer in photodynamic therapy for the treatment of hypoxic tumors, still more preferably for the treatment of glioblastoma, or uveal melanoma.

### Description of embodiments

A first aspect of the present disclosure relates to the provision of compounds of formula I or IV, including stereoisomers and E/Z isomers,
wherein:
M is a metal cation selected from the group consisting of: Fe²⁺, Ru²⁺, Os²⁺, Co³⁺, Rh²⁺, Rh³⁺, Ir³⁺, Ni²⁺, Pd²⁺, Pt²⁺, and Pt⁴⁺ ;
j = 1, 2, or 3; and z = 0, 1, or 2; on the proviso that: j + z = 2 when M is Ni²⁺, Pd²⁺ or Pt²⁺, and j + z = 3 when M is Fe²⁺, Ru²⁺, Os²⁺, Co³⁺, Rh²⁺, Rh³⁺, Ir³⁺, or Pt⁴⁺ ;
m = 0, 1, 2, 3, or 4;
radicals T and T' at each occurrence are each one a radical independently selected from the group consisting of: H and (C1-C3)-alkyl; 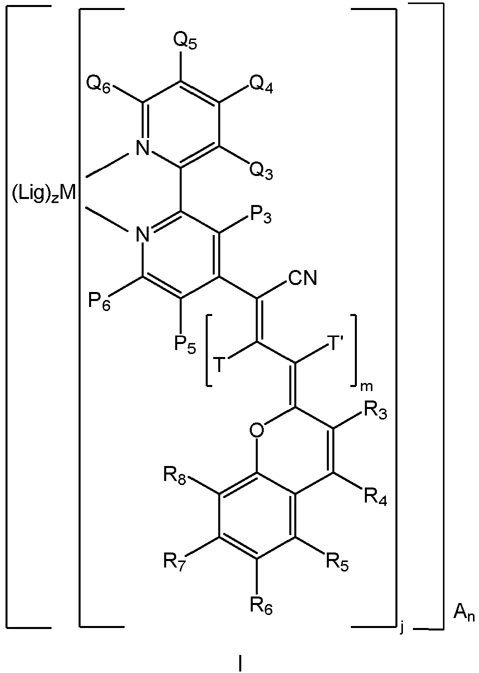 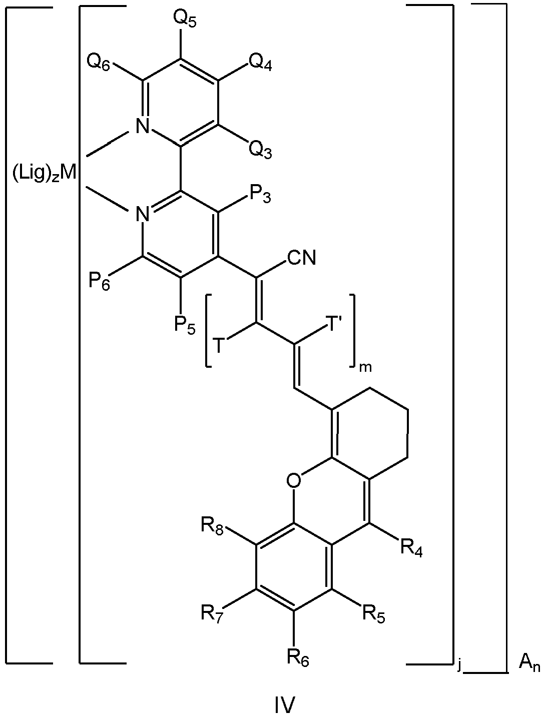
radicals P₃ , P₅ , P₆ , Q₃ , Q₄ , Q₅ , Q₆ , at each occurrence are each one a radical independently selected from the group consisting of: H , (C1-C3)-alkyl , (C3-C6)-cycloalkyl , CH=CHR' , CF₃, CHF₂, CH₂F, CF₂CF₃ , F , CI , Br, I , OR" , C(=O)OR" , O(C=O)R" , C(=O)NR", NR"C(=O)R' , NR"R' , CN , NO₂ , phenyl, mono-substituted phenyl, di-substituted phenyl, and tri-substituted phenyl; pyridine, mono-substituted pyridine, di-substituted pyridine, and tri-substituted pyridine; wherein substituents on the phenyl and pyridine rings are attached to any of the possible substitution positions, and they are independently selected from the group consisting of: F , CI , Br, I , NO₂ , (C1-C3)-alkyl , OH , O[(C1-C3)-alkyl] , CN , CF₃ , CHF₂, CH₂F , CF₂CF₃ , NH₂, NH[(C1-C3)-alkyl] , and N[(C1-C3)-alkyl]₂; and wherein Q₃ and P₃ can form a C=C bond when Q3 and P3 are both CH;
it is noted that, as indicated for some specific embodiments and aspects of the present invention, radical Q₄ can be alternatively selected from the accompanying formula below:

   X₁X₂YX₃X₄R
each of radicals X₁, X₂, Y, X₃, X₄, and R, are defined throughout the present invention.
radicals R₃, R₅ , R₆ , and R₈ at each occurrence are each one a radical independently selected from the group consisting of: H , (C1-C3)-alkyl , (C3-C6)-cycloalkyl , CH=CHR' , CF₃, CHF₂, CH₂F, CF₂CF₃ , F , Cl , Br, I , OR' , C(=O)OR' , O(C=O)R' , C(=O)NR', NR'C(=O)R" , NR'R" , CN , NO₂ , phenyl, mono-substituted phenyl, di-substituted phenyl, and tri-substituted phenyl; pyridine, mono-substituted pyridine, di-substituted pyridine, and tri-substituted pyridine; wherein substituents on the phenyl and pyridine rings are attached to any of the possible substitution positions, and they are independently selected from the group consisting of: F , Cl , Br, I , NO₂ , (C1-C3)-alkyl , OH , O[(C1-C3)-alkyl] , CN, CF₃, CHF₂, CH₂F , CF₂CF₃, NH₂ , NH[(C1-C3)-alkyl], and N[(C1-C3)-alkyl]₂;
radical R₇ is H, NO₂ , OR' , NR'R" , N(CH₂COOH)₂ , N(CH₂CH₂SO₃H)₂ , N(CH₂CONHCH₂CH₂NMe₂)₂ , or: an unsubstituted, a mono-(R')-substituted, a di-(R',R")-substituted, or a tri-(R',R",R‴)-substituted radical of each one of the heterocyclic radicals 1-aziridinyl, 1-azetidinyl, 1-pyrrolidinyl, 1-piperidinyl , 4-morpholinyl , or 1-piperazinyl; alternatively R₆ , R₇ and R₈ form together the bicycle system of the accompanying formula, wherein V₁ , V₂ , V₃ , V₄ , V₅ , V₆ , V₇ , V₅ , V₉ , V₁₀ , V₁₁ , and V₁₂ are each one a radical independently selected from the group consisting of H and (C1-C3)-alkyl; 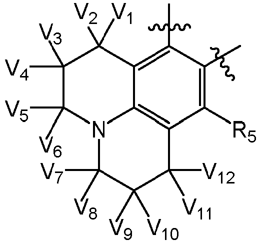
wherein radical R₄ is H , (C1-C3)-alkyl , (C3-C6)-cycloalkyl , CF₃, CHF₂, CH₂F, CF₂CF₃ , F , Cl , Br, I , OR' , C(=O)OR' , O(C=O)R' , C(=O)NR' , NR'C(=O)R" , NR'R" , phenyl, a mono-(R')-substituted phenyl, a di-(R', R")-substituted phenyl, a tri-(R', R", R‴)-substituted phenyl, or a radical CE₁E₂E₃ ; wherein: E₁ and E₂ are radicals independently selected from the group consisting of: H , F , Cl , Br, I , OR` , NR'R" , NO₂ , (C1-C3)-alkyl , phenyl, and a mono-(R')-substituted phenyl; and E₃ is OH, O[(C1-C3)-alkyl] , or a radical of the accompanying formula wherein p = 0, 1, 2, 3 or 4; and D₁ and D₂ at each occurrence are radicals independently selected from the group consisting of H , O[CH₂]_{q}-X , S[CH₂]_{q}-X , and N[[CH₂]_{q}-X]₂ , being q = 1 or 2, and X = F, Cl, Br or I. 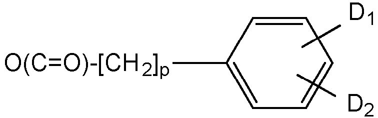
or a radical of the accompanying formula wherein p = 1, 2, 3 or 4; and D₃ is a radical independently selected from the group consisting of H , H(C=O) , [(C1-C3)-alkyl](C=O) , (OH)₂P(=O)O-CH₂-O(C=O) , (PhO)(OH)P(=O) 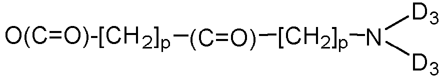
Lig at each occurrence is a bidentate ligand independently selected from the group consisting of ligands of accompanying formulae: Lig 1, Lig 2, , Lig 3, Lig 4, Lig 5, Lig 6, Lig 7, Lig 8, Lig 9, Lig 10, Lig 11, Lig 12, and Lig 13; wherein G is either an N atom, or a C atom with one negative charge that is formed *in situ* from a C-H when a H⁺ is lost in the formation of the carbon-metal bond; and wherein R₁₀ , R₁₁ , R₁₂ , R₁₃ , R₁₄ , R₁₅ , R₁₆ , R₁₇ , R₁₈ , R₁₉ , R₂₀ and R₂₁ at each occurrence is each one a radical independently selected from the group consisting of: H , (C1-C3)-alkyl , (C3-C6)-cycloalkyl , CH=CHR', CF₃, CHF₂, CH₂F, CF₂CF₃ , F , Cl , Br, I , OR' , C(=O)OR' , O(C=O)R' , C(=O)NR', NR'C(=O)R" , NR'R" , CN , NOz , phenyl, mono-substituted phenyl, di-substituted phenyl, and tri-substituted phenyl; pyridine, mono-substituted pyridine, di-substituted pyridine, and tri-substituted pyridine; wherein substituents on the phenyl and pyridine rings are attached to any of the possible substitution positions, and they are independently selected from the group consisting of: F , Cl , Br, I , NO₂ , (C1-C3)-alkyl , OH , O[(C1-C3)-alkyl] , CN , CF₃, CHF₂, CH₂F , CF₂CF₃, NH₂, NH[(C1-C3)-alkyl], and N[(C1-C3)-alkyl]₂;
wherein A is an anion from a pharmaceutically acceptable acid; n being an integer or fractional number whereby the compounds of formula I or IV are electrically neutral; j = 1, 2, or 3; and z = 0, 1, or 2; on the proviso that: j + z = 2 when M is Ni²⁺, Pd²⁺ or Pt²⁺, and j + z = 3 when M is Fe²⁺, Ru²⁺, Os²⁺, Co³⁺, Rh²⁺, Rh³⁺, Ir³⁺, or Pt⁴⁺
wherein R', R" and R‴ at each one of the above-mentioned occurrences are each one a radical independently selected from the group consisting of: H , F , Cl , Br, I , NO₂ , (C1-C3)-alkyl , OH, O[(C1-C3)-alkyl] , NH₂, NH[(C1-C3)-alkyl], and N[(C1-C3)-alkyl]₂.

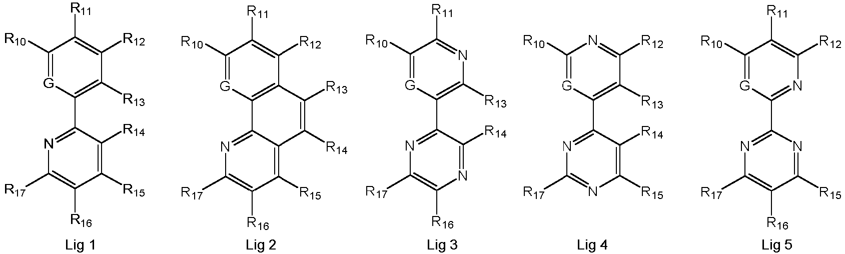

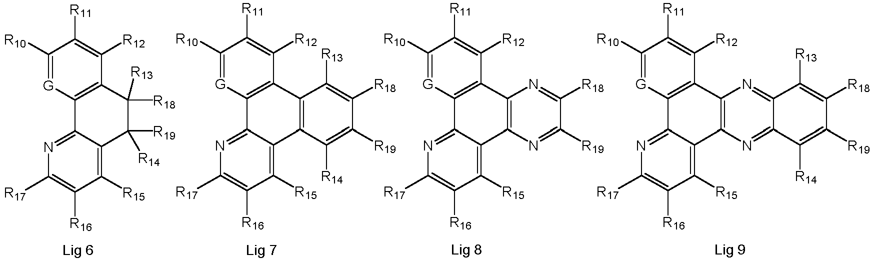

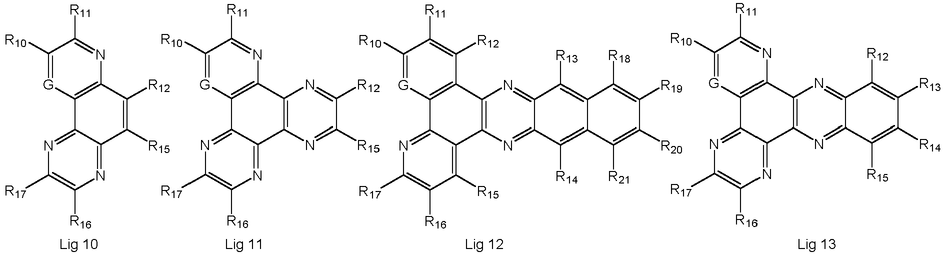

As used herein, the term "stereoisomer" is a general term for all isomers of individual molecules that differ only in the orientation of their atoms in space, and include enantiomers and diastereomers. When the compounds have chiral centers, they can exist in different stereoisomeric forms, such as enantiomeric or diastereomeric forms. Thus, any given compound referred to herein is intended to represent any one of a racemate, one or more enantiomeric forms, and one or more diastereomeric forms. All the stereoisomers, including enantiomers and diastereoisomers of the compounds referred to herein, and mixtures thereof (including racemic mixtures, enantiomerically enriched mixtures, and diastereomerically enriched mixtures), are considered within the scope of the present disclosure. The disclosure further contemplates any E/Z possible isomers and their mixtures.

It is noted that, in a preferred embodiment of the present invention, formula IV can be obtained from formula I, when in formula I m ≥ 1 (such 1, 2, 3 or more), radical R₃ being CH₂ forms a 6-membered ring with the T' radical closest to the coumarin backbone provided that T' = CH₂. It is further noted that, in another preferred embodiment of the present invention, formula V can be obtained from formula II, when in formula II m ≥ 1 (such 1, 2, 3 or more), radical R₃ being CH₂ forms a 6-membered ring with the T' radical closest to the coumarin backbone provided that T' = CH₂.

Generally, complex compounds of formula I or IV can be obtained by reacting -in a solvent or a solvent mixture- the corresponding intermediate compounds of formula II or V (respectively), including the corresponding stereoisomers or *E* / Z isomers thereof, with the corresponding reactant compounds of formula III, wherein m, n, j, z, P₃ , P₅ , P₆ , Q₃ , Q₄ , Q₅ , Q₆ , R₃ , R₄ , R₅ , R₆ , R₇ , R₈ , T , T' , M, Lig and A, are as defined above. As intermediate compounds of formula II or V are novel, another aspect of the present disclosure relates to the provision of these compounds, which are useful in the preparation of complex compounds of formula I or IV.

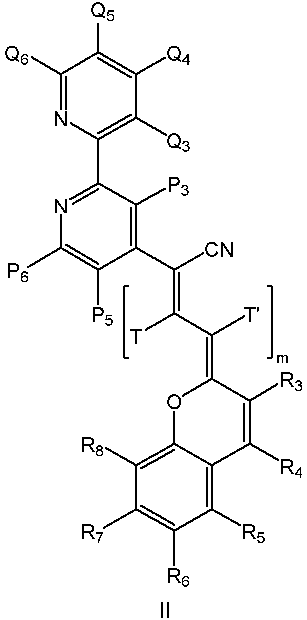

[M(Lig)_{z}]Aₙ III

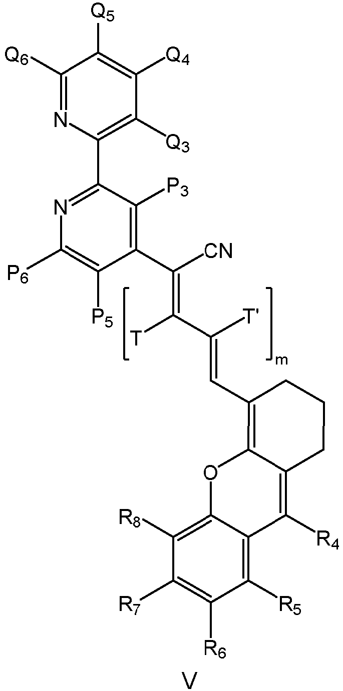

In general, reactant compounds of formula III and intermediate compounds of formula II or V are reacted in molar amount ratios which substantially correspond to the stoichiometry determined by the values of z and j, although an excess of III may be recommended in some cases to increase yield. In some cases, an intermediate compound is obtained with a different anion A', and a final anion-exchange reaction (e.g. with an ion-exchange resin) is carried out.

In particular embodiments of complex compounds of formula I or IV, the anion A is Cl⁻, Br, PF₆⁻, PF₄⁻, BF₄⁻ , ClO₄⁻, CF₃SO₃⁻, SO₄²⁻, CF₃COO⁻ , acetate, formate, and oxalate. In particular embodiments A is Cl⁻.

In particular embodiments of complex compounds of formula I or IV, j = 1, z = 2, and the metal cation M is Fe²⁺, Ru²⁺, Os²⁺, Co³⁺, Rh²⁺, Rh³⁺, Ir³⁺, or Pt⁴⁺ . In particular embodiments, the metal cation M is Ru²⁺.

In particular embodiments of complex compounds of formula I or IV, the bidentate ligand Lig is Lig 1; and each one of the radicals R₁₀ , R₁₁ , R₁₂ , R₁₃ , R₁₄ , R₁₅ , R₁₆ , R₁₇ , R₁₈ , R₁₉ , R₂₀ and R₂₁ at each occurrence is H, methyl or phenyl. In particular embodiments, G is N. In particular embodiments, the bidentate ligand Lig is 2,2'-bipyridine (bpy).

In particular embodiments, some complex compounds of formula I or IV are obtained by reaction between intermediate compounds of formula II or V and reactant compounds of formula III, wherein M = Ru²⁺ , A = Cl⁻ , z = 2 , j = 1, n = 2 , Lig = 2,2'-bipyridine (bpy), the solvent mixture is ethanol or a mixture of ethanol and water, and the molar amount ratio of compound III to compound II or V is 1.0 or slightly higher.

In particular embodiments of complex compounds of formula I or IV, each one of the radicals P₃ , P₅ , P₆ , Q₃ , Q₄ , Q₅ , Q₆ , R₃ (the definition of R₃ is only pertinent for formula I as formula IV fails to comprise this radical), R₅ , R₆ , and R₈ at each occurrence is H or (C1-C3)-alkyl; and the radical R₇ is N[(C1-C3)-alkyl)]₂ or NO₂ ; alternatively the radicals R₆ , R₇ and R₈ form together the bicycle system of the formula shown above, wherein each one of V₁ , V₂ , V₃ , V₄ , V₅ ,V₆ ,V₇ ,V₈ ,V₉ ,V₁₀ ,V₁₁ , and V₁₂ is H.

In particular embodiments of complex compounds of formula I or IV, the radical R₄ is H, (C1-C3)-alkyl , CF₃, or CH(CH₃)O(C=O)-[CH₂]₂-(C=O)-CH₂-NH₂,

It is noted that, in a preferred embodiment of the present invention, formula IV can be obtained from formula I, when in formula I m ≥ 1 (such 1, 2, 3 or more), radical R₃ being CH₂ forms a 6-membered ring with the T' radical closest to the coumarin backbone provided that T' = CH₂. It is further noted that, in another preferred embodiment of the present invention, formula V can be obtained from formula II, when in formula II m ≥ 1 (such 1, 2, 3 or more), radical R₃ being CH₂ forms a 6-membered ring with the T' radical closest to the coumarin backbone provided that T' = CH₂.

In particular embodiments m = 0, 1 or 2. In particular embodiments, the complex compounds of formula I or IV have one of the accompanying formulas la, Ib, Ic, Im, In, lo, Iq, IVa or IVb:

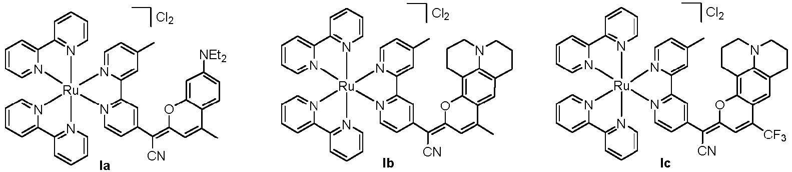

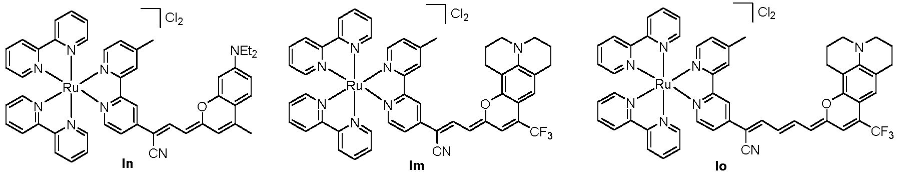

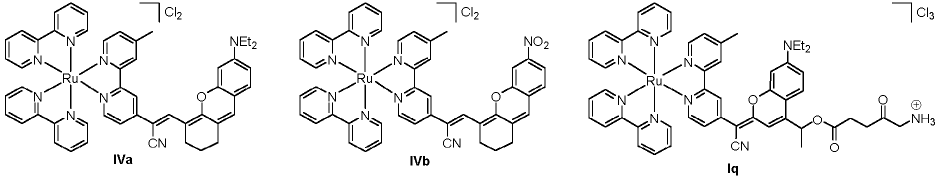

In particular embodiments the intermediate compounds of formula II (IIa, IIb, IIc, IIe, IIm, IIn, IIo, IIq, Ilr) or V (Va or Vb) have one of the accompanying formulas Ila, IIb, IIc, IIe, IIm, Iln, IIo, Ilq, IIr, Va or Vb.

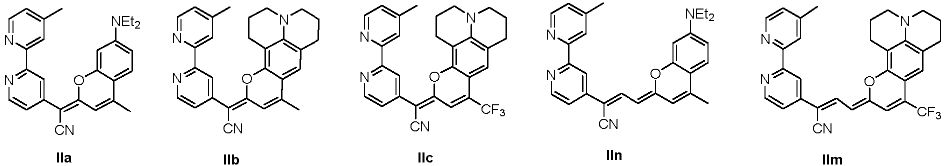

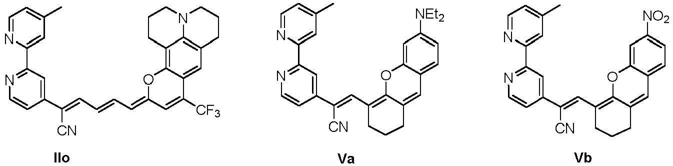

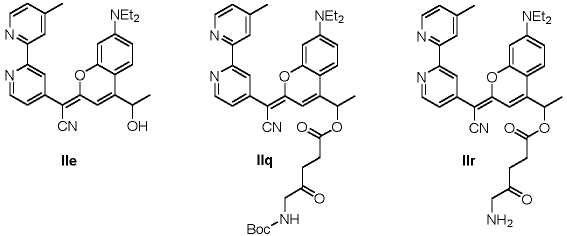

In a preferred embodiment of the first aspect of the present invention, optionally in combination with any previous or subsequent embodiments of the invention, the chemical compounds of formulae I and/or IV can be made conjugatable compounds to allow conjugation to any TTM compound or biomolecule such as peptides, proteins, glycoproteins, antibodies, carbohydrates or oligonucleotides. For such purpose, radical Q₄ of any of chemical compounds I and/or IV can be replaced by the following structural moiety: X₁X₂YX₃X₄R, wherein
X₁, X₂, X₃, X₄ are each one a radical independently selected from the group consisting of H, Ph, (CH₂)ₙ, (O-CH₂-CH₂)ₙ, (CH₂-CH₂-O)ₙ n=1-200
Y = F, Cl, Br, I, O, S, S-S, NH, C(=O), CH(=O), OC(=O), C(=O)O, NHC(=O),
C(=O)NH, CN, SC(=O), C(=O)S, NHC(=O)O, OC(=O)NH, NHC(=O)NH,
C(=O)NH-NH, C(=O)NH-N=CH
R = N₃, 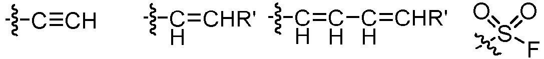 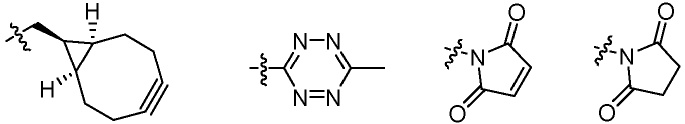 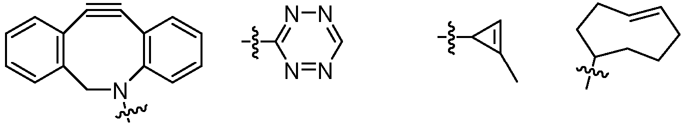
wherein any one of X₁, X₂, X₃, X₄ , Y and R can be optionally absent; preferably, wherein any one of X₁, X₂, X₃, X₄, and Y can be optionally absent thus leaving radical R as mandatory for bioconjugation reactions such as any biorthogonal reaction making use of radical R, including click chemistry; also preferably any one of X₁, X₂, X₃, X₄ , Y and R can be optionally absent with the proviso that at least one of X₁, X₂, X₃, X₄ , or Y is present so to allow the formation of conjugates by, for example, amide, ester, thioester, carbonate, thiocarbonate, hydrazone, oxyme, carbamate and urea bond formation, as well as by Michael addition reactions. That is, just as an explanatory example, if X₁ is consider absent then the formula above would be X₂YX₃X₄R; if X₂ would be absent then the formula would be X₁YX₃X₄R; if both X₂ and X₃ are deemed to be absent then the formula would be X₁YX₄R. If all of radicals X₁, X₂, X₃, X₄ and Y are absent then only R would be present.

That is, another preferred embodiment of the first aspect of the invention, optionally in combination with any previous or subsequent embodiment of the invention, refers to a metal complex compound having any of formulae I and/or IV, wherein radical Q₄ has been replaced as indicated above by the chemical moiety X₁X₂YX₃X₄R. Preferably, this specific embodiment refers to metal complex compounds having chemical formulae VI including stereoisomers and E/Z isomers: 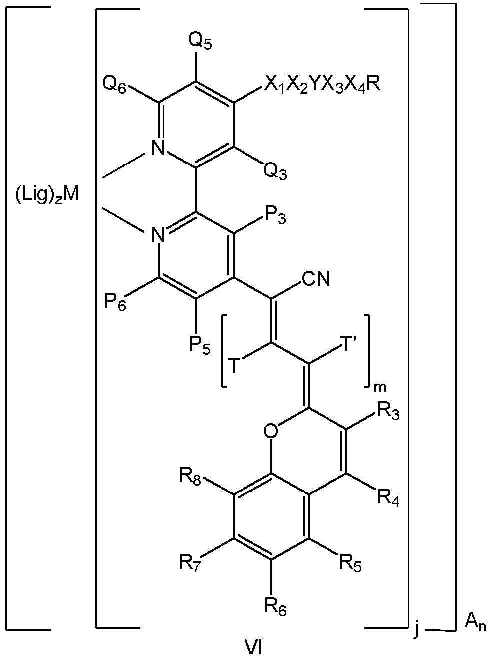
wherein M, A, n, j, z, X₁, X₂, X₃, X₄, Y, R, P₃ , P₅ , P₆ , Q₃ , Q₅ , and Q₆ are as defined above;
wherein m = 0, 1, 2, 3 or 4
wherein R₃, R₄, R₅, R₆, R₇ and R₈ are as defined above;
wherein Lig is as defined above; and
wherein radicals T and T' are as defined above.

It is noted that when, in formula VI above, m ≥ 1 (such 1, 2, 3 or more), if radical R₃ is CH₂ it can preferably form a 6-membered ring with the T' radical closest to the coumarin backbone provided that T' = CH₂. In this sense, another preferred embodiment of the first aspect of the invention, refers to a complex chemical compound of formula VII including stereoisomers and *E* / *Z* isomers: 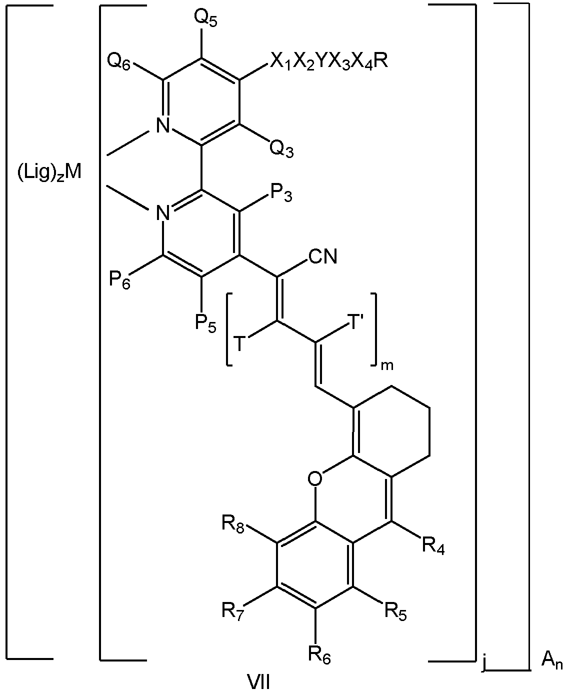 wherein M, Lig, A, n, j, z, m, X₁, X₂, X₃, X₄, Y, R, P₃ , P₅ , P₆ , Q₃ , Q₅ , Q₆, T, T' and R₄ to R₈ are as defined above.

It is noted that a much preferred Lig is Lig 1.

Preferred compounds of any of chemical compounds VI or VII according to the present invention are selected from the list consisting of: 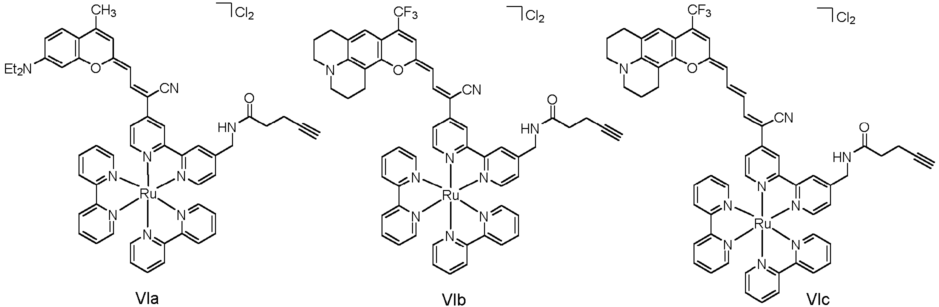 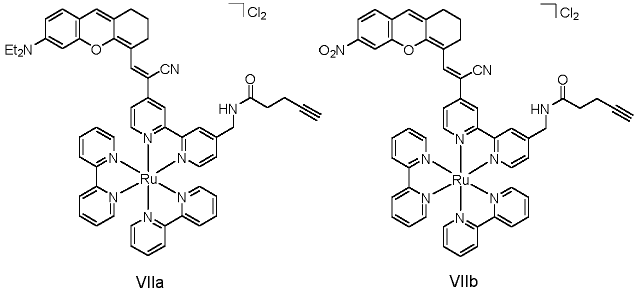 including stereoisomers and *E* / *Z* isomers thereof.

Notwithstanding the above, as indicated in example 3, the metal complex compounds of formulae I and/or IV can also be made conjugatable compounds to allow conjugation to TTM or biomolecule compounds by substituting any of positions R₁₂ or R₁₅ of Lig 1 with the following structural moiety: X₁X₂YX₃X₄R, wherein X₁, X₂, X₃, X₄, Y, and R are as defined above. That is, another preferred embodiment of the first aspect of the invention, optionally in combination with any of the previous embodiments of the first aspect of the invention, refers to a metal complex compound having any of formulae I and/or IV, wherein any one or both radicals R₁₂ and/or R₁₅ of Lig 1 have been replaced as indicated above by the chemical moiety X₁X₂YX₃X₄R. It is thus noted that metal complex compounds of formulae I and/or IV can be made conjugatable compounds to allow conjugation to TTM or biomolecule compounds by substituting any of positions Q₄ , R₁₂ and/or R₁₅, the possibility of modifying one, two or three of these positions with the structural moiety: X₁X₂YX₃X₄R is envisaged by the present invention. Preferably, a specific embodiment refers to metal complex compounds having chemical formulae XIII or XIV: 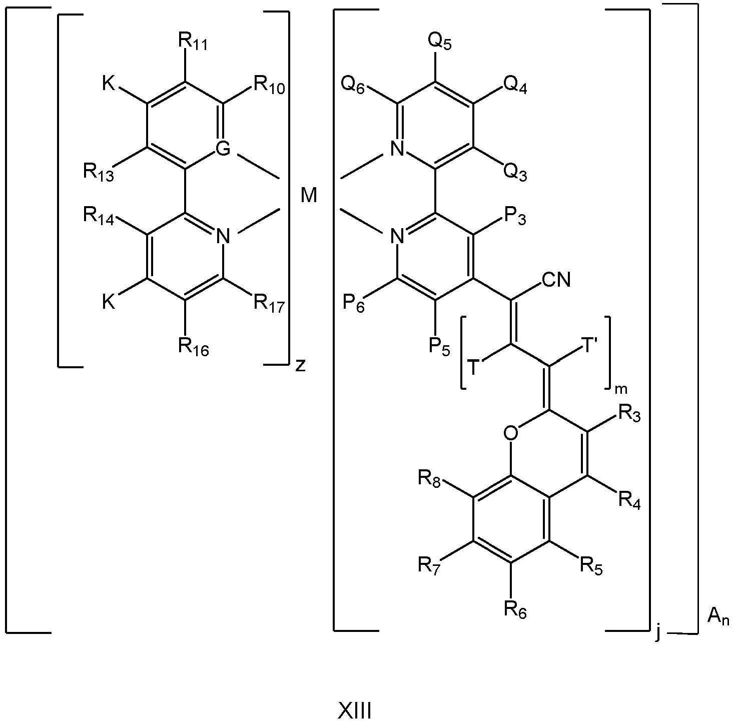
wherein A is an anion from a pharmaceutically acceptable acid; n being an integer or fractional number; whereby the compounds of formula XIII are electrically neutral; z = 1 or 2, j = 1 or 2, on the proviso that: j + z = 2 when M is Ni²⁺, Pd²⁺ or Pt²⁺, and j + z = 3 when M is Fe²⁺, Ru²⁺, Os²⁺, Co³⁺, Rh²⁺, Rh³⁺, Ir³⁺, or Pt⁴⁺ ; m = 0, 1, 2, 3 or 4;
wherein K radicals can be independently selected either from the structural moiety X₁X₂YX₃X₄R or from radicals R₁₂ or R₁₅ as defined above, wherein at least one of the K radicals of all of the bipyridine units (Lig1) present in formula XIII, is the structural moiety X₁X₂YX₃X₄R;
wherein G is either an N atom, or a C atom with one negative charge that is formed *in situ* from a C-H when a H⁺ is lost in the formation of the carbon-metal bond; and
wherein, R₁₀, R₁₁, R₁₃, R₁₄, R₁₆ and R₁₇ are as defined above, and wherein R₁₃ and R₁₄ can form a C=C bond when R₁₃ and R₁₄ are both CH. 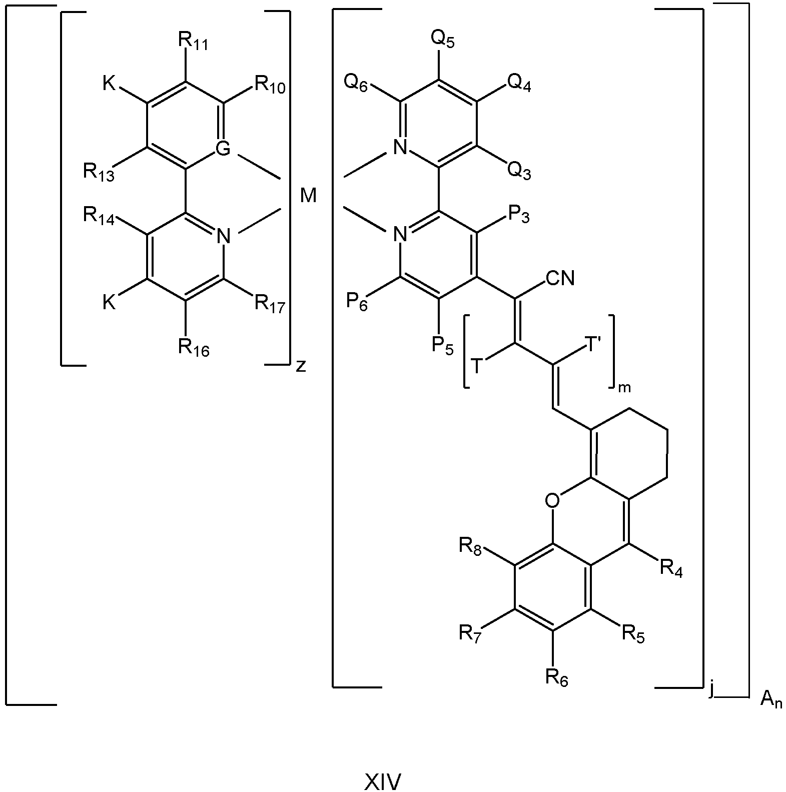
wherein A is an anion from a pharmaceutically acceptable acid; n being an integer or fractional number; whereby the compounds of formula XIII are electrically neutral; z = 1 or 2, j = 1 or 2, on the proviso that: j + z = 2 when M is Ni²⁺, Pd²⁺ or Pt²⁺, and j + z = 3 when M is Fe²⁺, Ru²⁺, Os²⁺, Co³⁺, Rh²⁺, Rh³⁺, Ir³⁺, or Pt⁴⁺ ; m = 0, 1, 2, 3 or 4;
wherein K radicals can be independently selected either from the structural moiety X₁X₂YX₃X₄R or from radicals R₁₂ or R₁₅ as defined above, wherein at least one of the K radicals of all of the bipyridine units (Lig1) present in formula XIII, is the structural moiety X₁X₂YX₃X₄R;
wherein G is either an N atom, or a C atom with one negative charge that is formed *in situ* from a C-H when a H⁺ is lost in the formation of the carbon-metal bond; and
wherein, R₁₀, R₁₁, R₁₃, R₁₄, R₁₆ and R₁₇ are as defined above, and wherein R₁₃ and R₁₄ can form a C=C bond when R₁₃ and R₁₄ are both CH.

It is noted that, in a preferred embodiment of the present invention, formula XIV can be obtained from formula XIII, when in formula XIII m ≥ 1 (such 1, 2, 3 or more), radical R₃ being CH₂ forms a 6-membered ring with the T' radical closest to the coumarin backbone provided that T' = CH₂.

In another preferred embodiment, apart from any of radicals R₁₂ or R₁₅ of any of formulae XIII and/or XIV, also radical Q₄ can be replaced as indicated above by the chemical moiety X₁X₂YX₃X₄R.

On the other hand, it is noted that any of chemical compounds VI or VII can be preferably synthesized by using the synthetic route indicated in example 2 of the present invention. For such purpose, the intermediate compounds of chemical formulae VIII and IX are of particular interest: 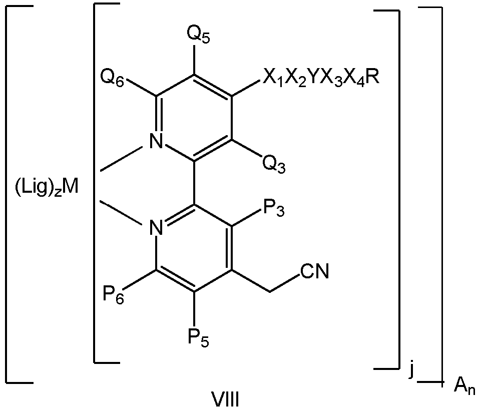 wherein Lig, M, A, n, j, z, X₁, X₂, X₃, X₄, Y, R, P₃ , P₅ , P₆ , Q₃ , Q₅ , and Q₆, are as defined above. 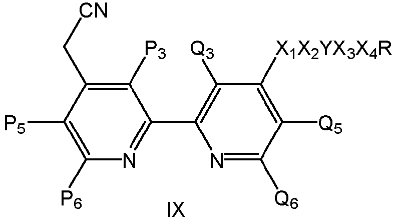 wherein X₁, X₂, X₃, X₄, Y, R, P₃ , P₅ , P₆ , Q₃ , Q₅ , and Q₆ are as defined above;

A preferred compound of any of chemical compounds of formula VIII, including stereoisomers and E/Z isomers, according to the present invention is:

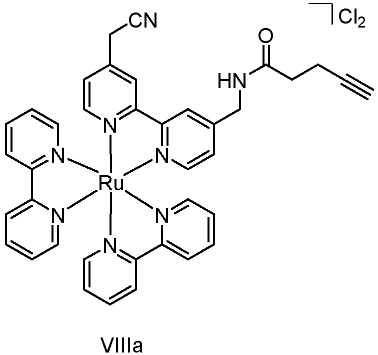

Therefore, in a second aspect of the invention, the present invention refers to any of chemical compounds of chemical formulae VIII or IX, preferably Villa, including stereoisomers and *E* / Z isomers.

Furthermore, it is noted that any of chemical compounds XIII or XIV can be preferably synthesized by using the synthetic route indicated in example 3 of the present invention. For such purpose, the intermediate compounds of chemical formulae XV are of particular interest: 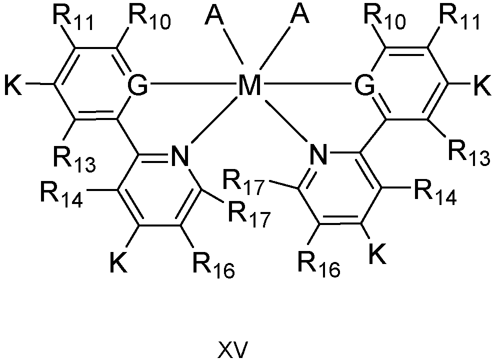 wherein M is as defined above;
wherein K radicals can be independently selected either from the structural moiety X₁X₂YX₃X₄R or from radicals R₁₂ or R₁₅ as defined above, wherein at least one of the K radicals is the structural moiety X₁X₂YX₃X₄R;
A is as defined above, however, in the context of formula XV, is usually a CI atom;
wherein G is either an N atom, or a C atom with one negative charge that is formed *in situ* from a C-H when a H⁺ is lost in the formation of the carbon-metal bond; and
wherein, R₁₀, R₁₁, R₁₃, R₁₄, R₁₆ and R₁₇ are as defined above, and wherein R₁₃ and R₁₄ can form a C=C bond when R₁₃ and R₁₄ are both CH.

Therefore, in a third aspect of the invention, the present invention refers to any of chemical compounds of chemical formulae VIII, IX or XV including stereoisomers and E/Z isomers.

On the other hand, a fourth aspect of the invention refers to a process for manufacturing the intermediate complex compound of chemical formula VIII, wherein the process comprises the steps of:
a. Dissolving a ligand compound of formula IX 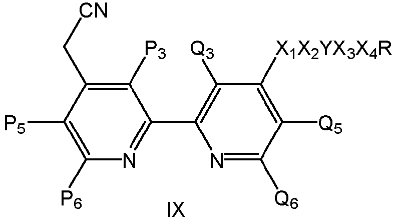 wherein X₁, X₂, X₃, X₄, Y, R, P₃ , P₅ , P₆ , Q₃ , Q₅ , and Q₆ are as defined above;
   with a compound of formula [M(Lig)_{z}]Aₙ in a solvent or solvent mixture under a normal or an inert atmosphere to carry out a coordination reaction to form the complex compound of formula VIII, in which the reactant compounds of formulae IX and [M(Lig)_{z}]Aₙ are reacted in preferably molar amount ratios; and
b. optionally filtering, concentrating and/or purifying the resulting complex compound of formula VIII.

In a preferred embodiment of this aspect of the invention, the Lig is Lig 1, and G is an N atom.

It is noted that the solvent or solvent mixture is preferably selected from, but not limited to, the list consisting of: water, alcoholic solvents (e.g. methanol, ethanol, isopropanol), acetonitrile, tetrahydrofurane, dioxane, dimethylsulfoxide, *N,N*-dimethylformamide and *N-*methylpyrrolidone, and wherein the inert atmosphere may be selected from nitrogen or argon. It is further noted that the coordination reaction of step a) preferably takes place during a time range of from 2 to 72 h at a temperature of from 60°C to 100°C. Alternatively, microwave irradiation may be used to reduce the reaction time and/or the temperature.

Furthermore, a fifth aspect of the invention refers to a process for manufacturing complex compounds of formulae VI: 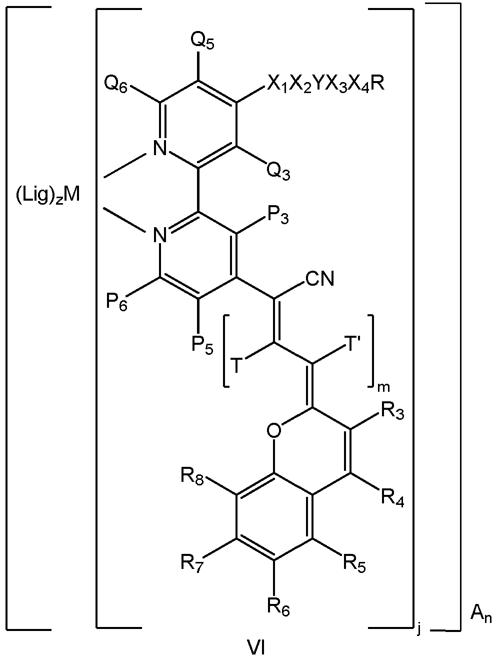 wherein all of the substituents are as defined above; and
wherein the process for manufacturing complex compounds of formulae VI comprises the steps of:
a. contacting a complex compound of formula VIII as defined above, with a coumarin of formula X, or XI, wherein m and radicals R₃, R₄, R₅, R₆, R₇, R₈, T and T' are as defined above, and preferably a base (e.g. piperidine) in a solvent or solvent mixture under a normal or an inert atmosphere, and carrying out a condensation reaction to form the complex compound of formula VI; and
b. optionally filtering, concentrating and/or purifying the complex compound of formula VI; 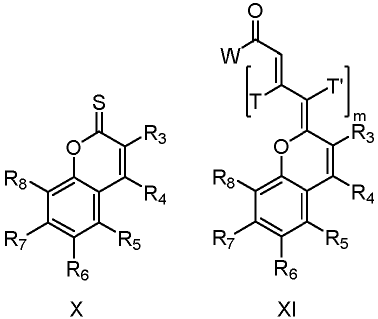 wherein W is selected from any of: H, (C1-C3)-alkyl, CF₃, or CF₂CF₃

In a preferred embodiment of this aspect of the invention, the Lig is Lig 1, and G is an N atom. In another preferred embodiment of this aspect of the invention, the solvent or solvent mixture is preferably selected from the list consisting of water, alcoholic solvents (e.g. methanol, ethanol, isopropanol), acetonitrile, tetrahydrofurane, dioxane, dimethylsulfoxide, N,N-dimethylformamide and *N*-methylpyrrolidone, and wherein the inert atmosphere is preferably selected from nitrogen or argon. Preferably, the condensation reaction of step a) takes place during a time range of from 2 to 72 h at a temperature of from 0°C to 100°C. Alternatively, microwave irradiation may be used to reduce the reaction time and/or the temperature.

Furthermore, a sixth aspect of the invention refers to a process for manufacturing complex compounds of formulae VII: 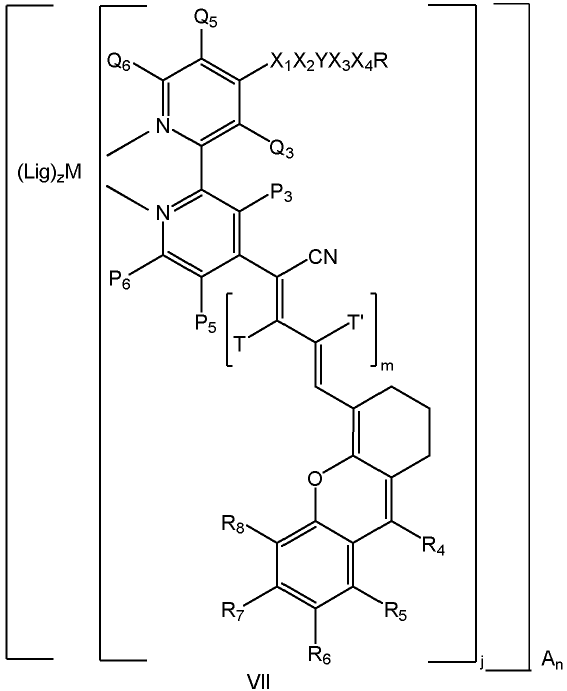 wherein A, n, j, z, m, X₁, X₂, X₃, X₄, Y, R, P₃ , P₅ , P₆ , Q₃ , Q₅ , Q₆, T, T', Lig and R₄ to R₈ are as defined above; and
wherein the process for manufacturing complex compounds of formulae VII comprises the steps of:
a. contacting a complex compound of formula VIII as defined above, with a coumarin of formula XII, wherein m and radicals R₃, R₄, R₅, R₆, R₇, R₈, T, T' and Ware as defined above, and preferably a base (e.g. piperidine) in a solvent or solvent mixture under a normal or an inert atmosphere, and carrying out a condensation reaction to form the complex compound of formula VII; and 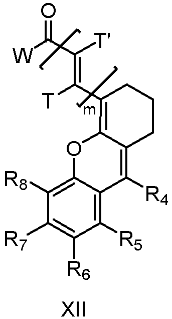
b. optionally filtering, concentrating and/or purifying the complex compound of formula VIII.

In a preferred embodiment of this aspect of the invention, the Lig is Lig 1, and G is an N atom.

In another preferred embodiment of this aspect of the invention, the solvent or solvent mixture is preferably selected from the list consisting of water, alcoholic solvents (e.g. methanol, ethanol, isopropanol), acetonitrile, tetrahydrofurane, dioxane, dimethylsulfoxide, N,N-dimethylformamide and *N*-methylpyrrolidone, and wherein the inert atmosphere is preferably selected from nitrogen or argon. Preferably, the condensation reaction of step a) takes place during a time range of from 2 to 72 h at a temperature of from 0°C to 100°C. Alternatively, microwave irradiation may be used to reduce the reaction time and/or the temperature.

A seventh aspect of the invention refers to a process for manufacturing metal complex compounds of formulae XIII and/or XIV wherein the process comprises the steps of:
a. contacting a complex compound of formula XV with a coumarin of formula II or V, wherein radicals K, R₁₀, R₁₁, R₁₃, R₁₄, R₁₆, R₁₇, Q₃ , Q₄ , Q₅, Q₆, P₃, P₅ , P₆ , R₃, R₄, R₅, R₆, R₇, R₈, T and T' are as defined above, in a solvent or solvent mixture under a normal or an inert atmosphere, and carrying out a coordination reaction to form the metal complex compound of formulae XIII or XIV, or alternatively intermediate metal complex compounds of formulae XIII' and XIV' containing a K' radical; and
b. optionally reacting the chemical moiety K' of an intermediate complex compound of formulae XIII' or XIV' with the suitable reagents and conditions to provide metal complex compounds of formulae XIII or XIV containing the chemical moiety K, and
c. optionally filtering, concentrating and/or purifying the complex compound of formulae XIII and XIV.

An eighth aspect of the invention refers to a method for preparing complex compounds of formula XVI or XVII, wherein these compounds are conjugated to a TTM or biomolecule, which comprises the steps of:
a. reacting a peptide, protein, glycoprotein, antibody, carbohydrate or oligonucleotide bearing a suitable functional group and linker with the complementary group of the chemical moiety X₁X₂YX₃X₄R of metal complex compounds of formula VI or VII defined above, preferably via a bioorthogonal reaction, catalysed or not by copper, in a solvent or solvent mixture under a normal o inert atmosphere, preferably in a controlled pH, and, when required, using a coupling reagent or additive; and
b. optionally filtering, concentrating and/or purifying the complex compound of formula XVI or XVII. 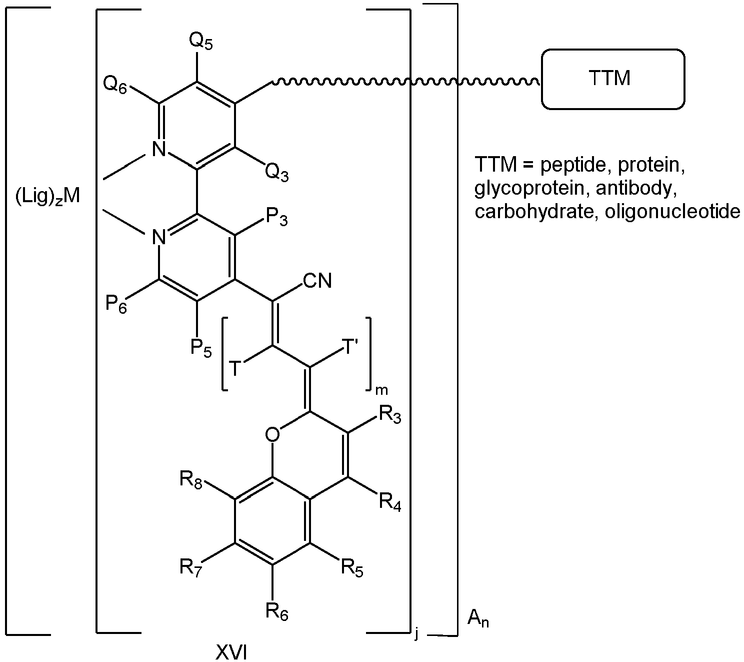 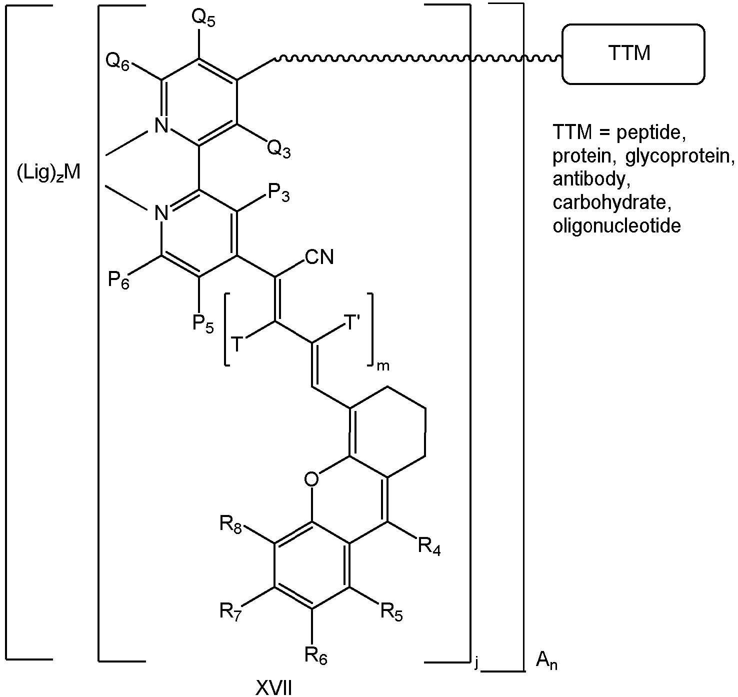
wherein M, A, n, j, z, m, P₃ , P₅ , P₆ , Q₃ , Q₅ , Q₆, T, T', Lig and R₃ to R₈ are as defined above.

A ninth aspect of the present invention, refers to a complex compound of formulae XVI or XVII including stereoisomers and *E* / *Z* isomers: 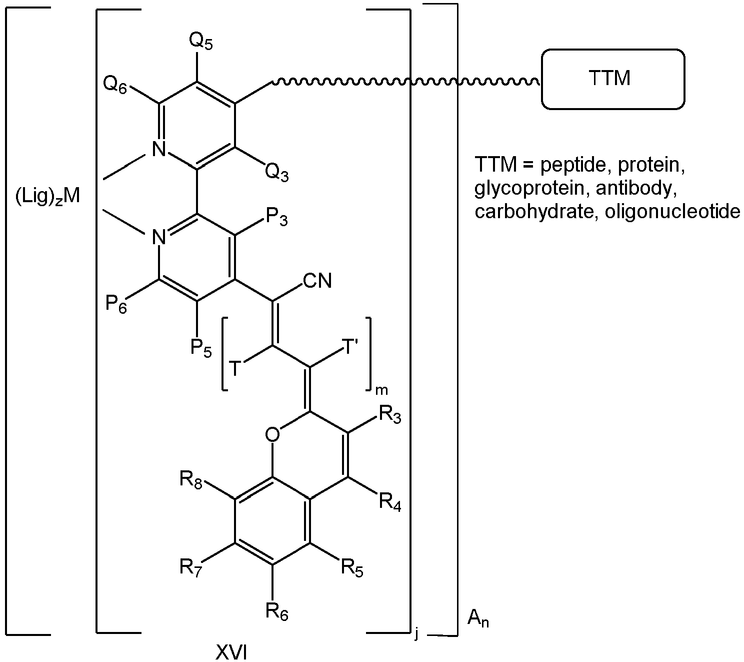 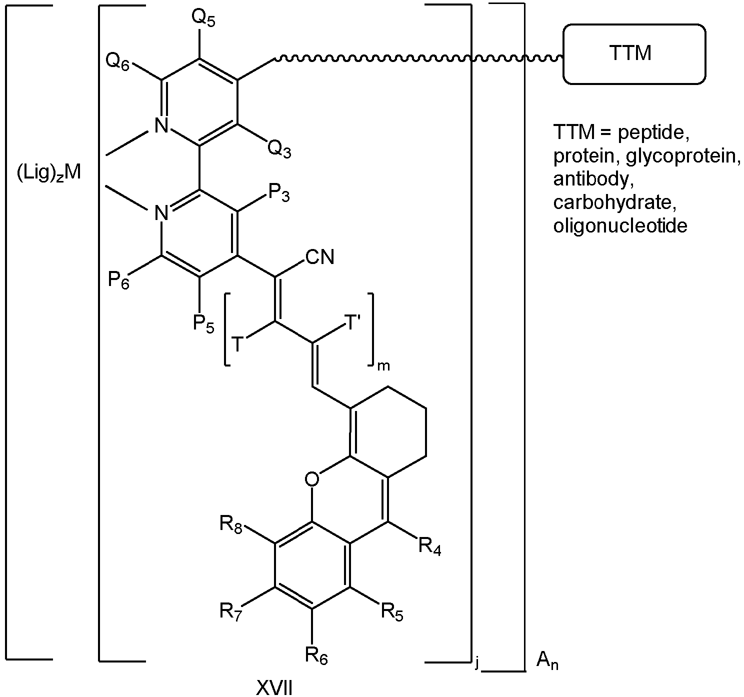 wherein M, A, n, j, z, m, P₃ , P₅ , P₆ , Q₃ , Q₅ , Q₆, T, T', Lig and R₃ to R₈ are as defined above; and wherein TTM is a peptide, protein, glycoprotein, antibody, carbohydrate or oligonucleotide bearing a suitable functional group (e.g. azide, alkene, 1,4-diene, linear or cyclic alkyne, maleimide, succinimide, tetrazine, ciclopropene, hydrazine, NH₂, COOH, CHO, SH) attached to a suitable position through an appropriate linker and that reacts with the complementary group of the chemical moiety X₁X₂YX₃X₄R of complex compounds of formula VI or VII, preferably selected from the list consisting of octreotide, RGD-containing peptide sequences, MC1R peptide ligands and Trastuzumab and biosimilars.

In a preferred embodiment of the ninth aspect of the present invention, said compound is selected from the list consisting of:
a. Ru-RGD conjugate (XVIa) 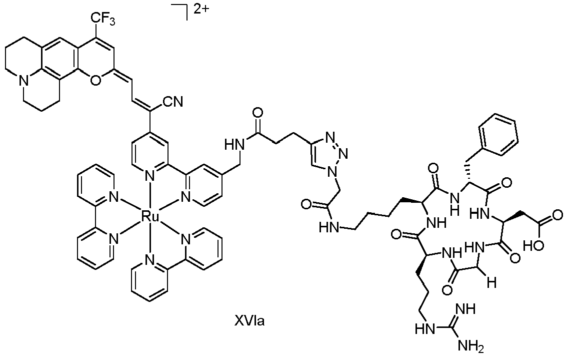
b. Ru-octreotide conjugate (XVIb) 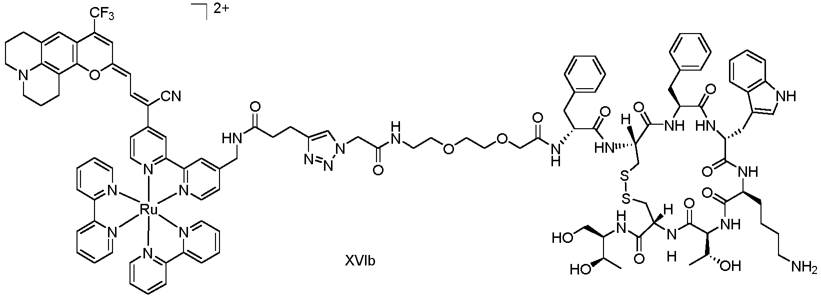
c. Ru-L1MC1R conjugate (XVIc) 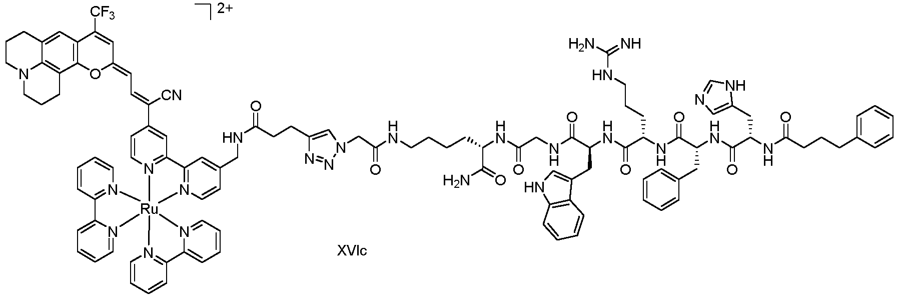
d. Ru-L2MC1R conjugate (XVId) 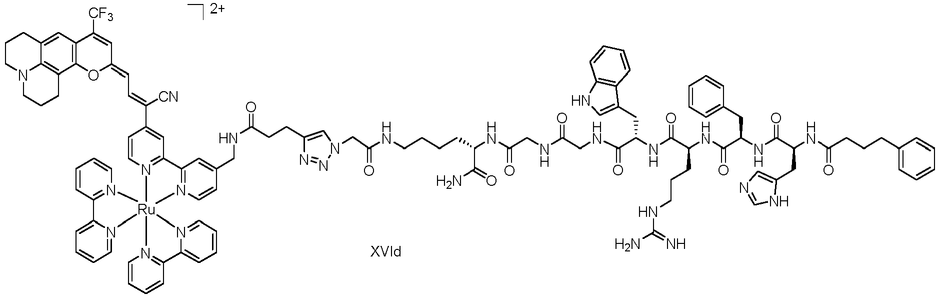
e. Ru-L3MC1R conjugate (XVIe) 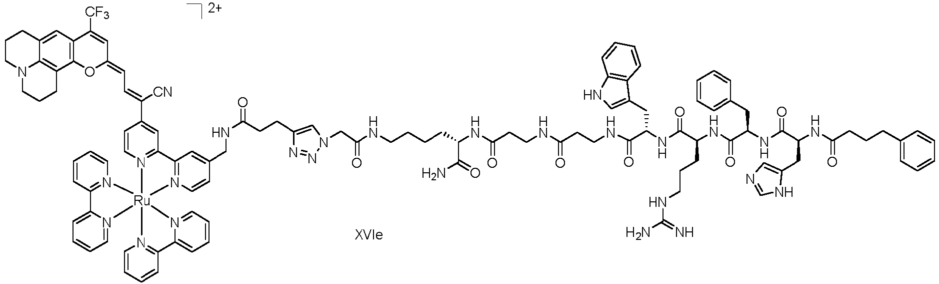
including stereoisomers and E/Z isomers.

A tenth aspect of the present invention refers to any one of peptides L1MC1R, L2MC1R and L3MC1R or any combination thereof, required for the preparation of conjugates XVIc, XVId and XVIe:

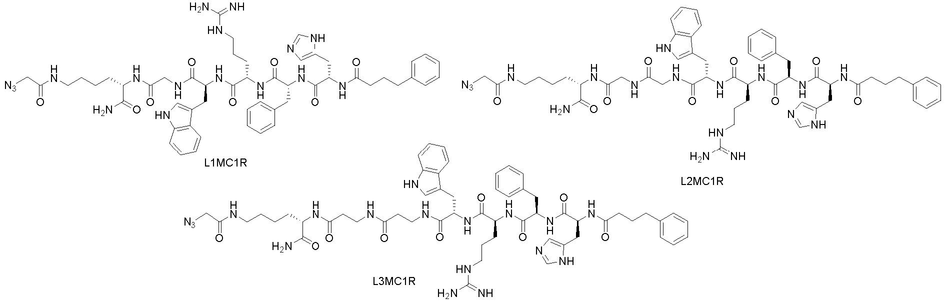

In a preferred embodiment of the tenth aspect of the invention, the present invention is directed to any one of peptides L1MC1R, L2MC1R and L3MC1R as such, or to any composition containing or comprising the same, including pharmaceutical compositions.

An eleventh aspect of the present invention refers to a method for preparing complex compounds of formula XVIII or XIX, wherein these compounds are conjugated to a TTM or biomolecule, which comprises the steps of
a. reacting a peptide, protein, glycoprotein, antibody, carbohydrate or oligonucleotide bearing a suitable functional group and linker with the complementary group of the chemical moiety X₁X₂YX₃X₄R of complex compounds of formula XIII or XIV defined above, preferably via a bioorthogonal reaction, catalysed or not by copper, in a solvent or solvent mixture under a normal o inert atmosphere, in a controlled or not pH, and, when required, using a coupling reagent or additive; and
b. optionally filtering, concentrating and/or purifying the complex compound of formula XVIII or XIX. 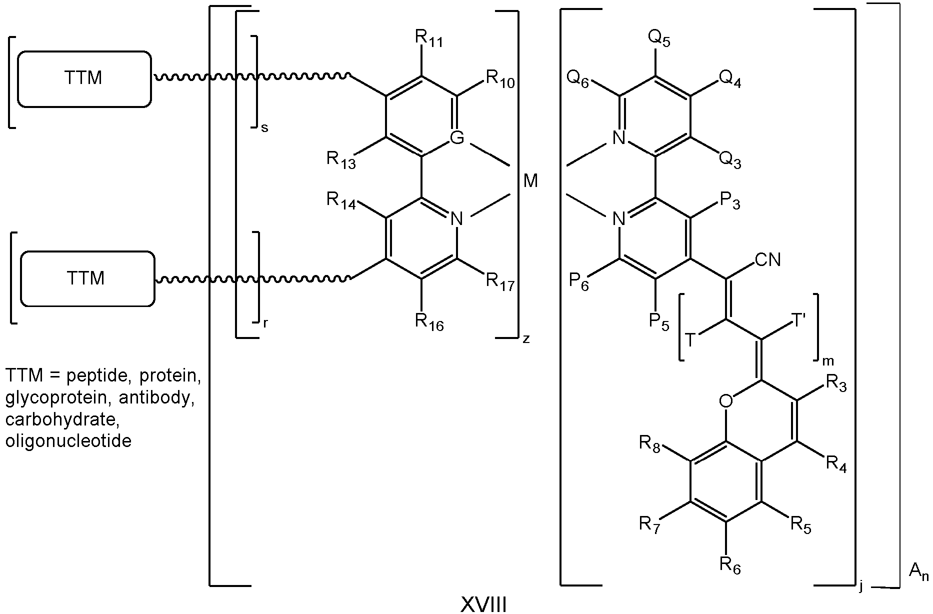
   wherein M, A, n, j, z, m, P₃ , P₅ , P₆ , Q₃ , Q₄ , Q₅ , Q₆, T, T', R₃ to R₈, R₁₀, R₁₁, R₁₃, R₁₄, R₁₆ and R₁₇ are as defined above;
   whereby when z = 1, r and s can be independently selected between 0 and 1
   whereby when z = 2, r, r', s and s' can be independently selected at each bipyridine unit between 0 and 1; then, r+r+s+s' can be 0, 1, 2, 3 or 4 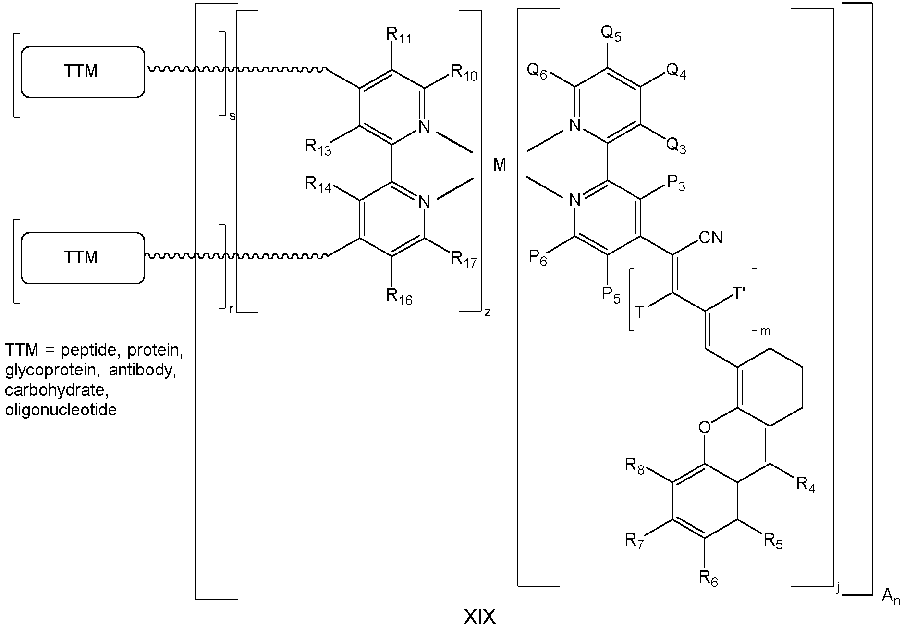
   wherein M, A, n, j, z, m, P₃ , P₅ , P₆ , Q₃ , Q₄ , Q₅ , Q₆, T, T', R₃ to R₈, R₁₀, R₁₁, R₁₃, R₁₄, R₁₆ and R₁₇ are as defined above;
   whereby when z = 1, r and s can be independently selected between 0 and 1
   whereby when z = 2, r, r', s and s' can be independently selected at each bipyridine unit between 0 and 1; then, r+r+s+s' can be 0, 1, 2, 3 or 4

It is noted that although chemical structures XVIII or XIX are drawn having two TTMs per Lig, it will become apparent for the skilled person that each bipyridine unit can have one or two TTMs or none as long as at least one bipyridine unit has at least one TTM.

A further aspect of the present invention refers to a complex compound of formula XVIII or XIX including stereoisomers and *E* / *Z* isomers.

As illustrated by the results shown in the description of embodiments section (Tables 1 and 2), metal complex compounds of formula I or IV are useful as PSs in anticancer PDT. For example, complex compounds of formula Ic and Im show very good phototoxic activity against two human glioblastoma cancer cell lines, HCB-GIC20 and U87-MG, under hypoxic conditions (5% O₂) and upon irradiation with light within the phototherapeutic window, being particularly relevant those obtained in the glioblastoma-initiating cells (HCB-GIC20) isolated from glioblastoma biopsies obtained at Hospital Clinic Barcelona (Barcelona, Spain). These results indicate that the compounds of the present invention are especially useful as a photosensitizer in photodynamic therapy for the treatment of highly aggressive hypoxic tumours such as glioblastoma, uveal melanoma and other hypoxic tumours such as pancreas, breast and colorectal cancer.

Thus, another aspect of the present disclosure relates to the provision of pharmaceutical compositions comprising a therapeutically effective amount of a complex compound of formula I or IV, as well as any compound of formulae VI, VII, XIII, XIV, XVI, XVII, XVIII, and/or XIX as well as any further specific compound identified throughout the present invention, together with appropriate amounts of excipients, carriers or vehicles.

Complex compounds of formula I are virtually non-toxic in dark conditions at therapeutic dosage. For example, complex compounds Ic and Im are non-toxic in dark conditions with IC₅₀ values 181.70 µM and 271.17 µM, respectively, in U87-MG glioblastoma cells, but become highly cytotoxic after irradiation with monochromatic visible light. As shown in Tables 1 and 2, IC₅₀ values for compound Ic were found in the low submicromolar range upon irradiation with 660 nm far-red light (0.6 µM in U87-MG cells and 0.13 µM in patient-derived glioblastoma HCB-GIC20 cells) with very good PI values (>303 and >330, respectively). Similarly, complex compound of formula Im exhibited very good activity against HCB-GIC20 cells upon irradiation at 660 nm (IC₅₀ = 0.48 µM, PI = 214). By contrast, the IC₅₀ values for PpIX, a PS that is in clinical development against glioblastoma, are much higher in HCB-GIC20 cells (e.g. 2.45 µM, PI = 60, 660 nm).

Compound of formula Im shows a good phototoxicity profile with highly penetrating NIR light (760 nm), especially against the patient-derived glioblastoma HCB-GIC20 cells (IC₅₀ = 2.82 µM, PI = 36), whereas PpIX was deemed completely non-active under the same experimental conditions (IC₅₀ = 159.6 µM, PI = 1). Such results illustrate that compounds of formula I or IV are useful for the PDT treatment of deep-seated hypoxic tumours by using light within the phototherapeutic window far-red to NIR.

Aspects of the present disclosure relate to metal complex compounds of formula I or IV, as well as any compound of formulae VI, VII, XIII, XIV, XVI, XVII, XVIII, and/or XIX as well as any further specific compound identified throughout the present invention, as for use in human therapy, particularly for use as photosensitizer in photodynamic therapy of a human condition; more specifically the human condition is a cancer, a skin condition, a fungal infection or a microbial infection. More particular, for use as a photosensitizer in photodynamic therapy for the treatment of aggressive hypoxic tumours such as glioblastoma, uveal melanoma and other hypoxic tumours such as pancreas, breast and colorectal cancer. These aspects also relate to preparation processes of medicaments for photodynamic therapy in humans of cancer, skin conditions, fungal infections or microbial infections, comprising the use of complex compounds of formula I or IV, preferably in the form of nanoformulations or their conjugates to tumor-targeting moieties or biomolecules. In other words, it can be said that complex compounds of formula I or IV are useful in methods of treatment by photodynamic therapy, of cancer, skin conditions, fungal infections or microbial infections in humans.

Although an important attribute of PDT is that cytotoxicity is confined to regions where the PS and light overlap spatiotemporally, PSs exhibiting cancer cell specificity offer an additional advantage to minimize toxic side effects that can occur upon accumulation in surrounding healthy tissues. Therefore, as indicated throughout the text, new conjugatable metal complex compounds have been described in the present invention, as well as their conjugates to tumour targeting moieties, which represents an important milestone in the field of PDT. The availability of PSs based on conjugated metal complex compounds with cancer cell specificity will obviously position photodynamic therapy as a reference treatment modality of hypoxic tumours.

Throughout the present disclosure and claims the word "comprise" and variations of the word, are not intended to exclude other technical features, additives, components, or steps. Furthermore, the word "comprise" encompasses the case of "consisting of". Additional objects, advantages and features will become apparent to those skilled in the art upon examination of the disclosure, or may be learned by practice of the disclosure. The following examples are provided by way of illustration, and they are not intended to be limiting.

### Examples

### Example 1. General synthetic routes for the preparation of metal complex compounds of formulae I and IV and of ligand compounds of formulae II and V.

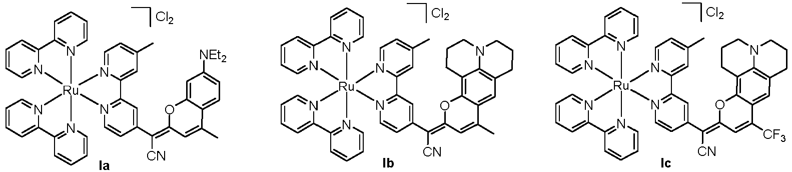

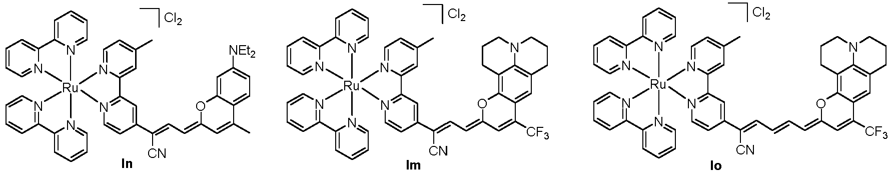

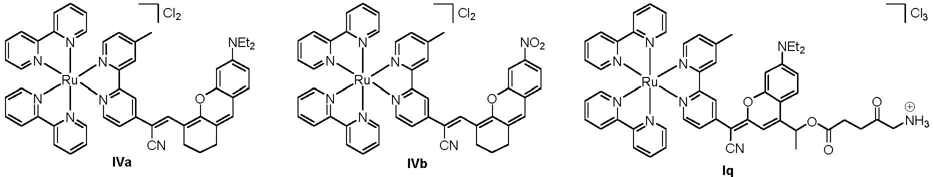

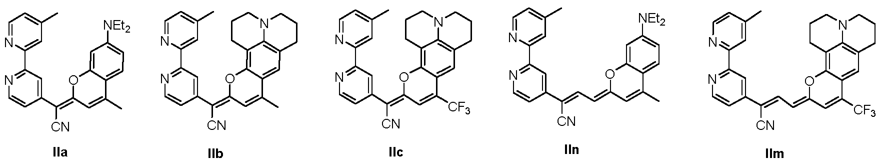

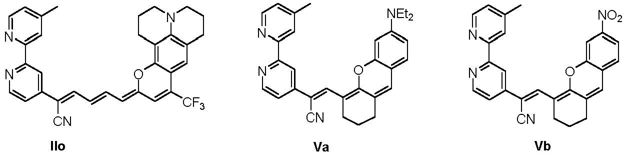

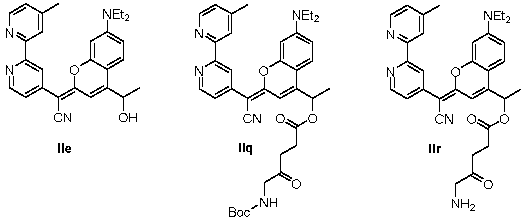

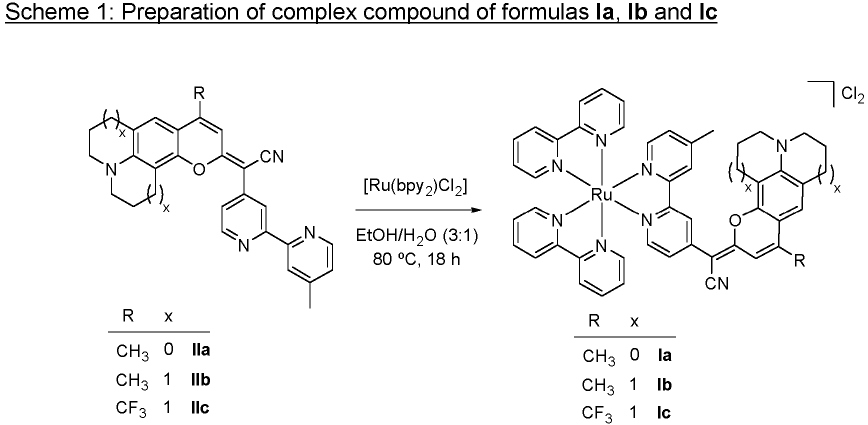

Complex compound of formula **Ia**. Ligand compound of formula **IIa** (31 mg, 0.073 mmol) and [Ru(bpy)₂Cl₂] (37 mg, 0.076 mmol) were dissolved in 3 mL of a 3:1 (v/v) solution of EtOH/H₂O. The reaction mixture was stirred overnight at 80 °C and analyzed by HPLC-MS to confirm the formation of the product. The solution mixture was evaporated to dryness and the resulting aqueous solution was lyophilized and the product was isolated by silica column chromatography starting with hexanes and increasing the eluent polarity with DCM first (0-100%) and then with MeOH (0-20%). 41 mg of a red-maroon solid were obtained (yield: 89%), identified as complex compound of formula la. TLC: Rf (35% MeOH in DCM) 0.4. HR-ESI MS (ESI): *m*/*z* 418.1265 calc. for [C₄₇H₄₂N₈ORu]²⁺: 418.1257.

Complex compound of formula **Ib**. Ligand compound of formula **IIb** (34 mg, 0.076 mmol) and [Ru(bpy)₂Cl₂] (38 mg, 0.078 mmol) were dissolved in 3 mL of a 3:1 (v/v) solution of EtOH/H₂O. The reaction mixture was stirred overnight at 80 °C and analyzed by HPLC-MS to confirm the formation of the product. The solvent was evaporated to dryness and the product was isolated by silica column chromatography starting with hexanes and increasing the eluent polarity with DCM first (0-100%) and then with MeOH (0-20%). 46 mg of a dark-maroon solid were obtained (yield: 65%), identified as complex compound of formula lb. TLC: Rf (1:9 MeOH/DCM) 0.5. HR-ESI MS (ESI): *m*/*z* 430.1258, calc. for [C₄₉H₄₂N₈ORu]²⁺: 430.1257.

Complex compound of formula **Ic**. Ligand compound of formula **IIc** (31.6 mg, 0.063 mmol) and [Ru(bpy)₂Cl₂] (37.4 mg, 0.077 mmol) were dissolved in 2 mL of a 3:1 (v/v) solution of EtOH/H₂O. The reaction was stirred overnight at 80 °C and analyzed by HPLC-MS to confirm the formation of the product. The solvent was evaporated to dryness and the product was isolated by silica column chromatography starting with hexanes and increasing the eluent polarity with DCM first (0-100%) and then with MeOH (0-16%). 51.4 mg of a dark-purple solid were obtained, identified as complex compound of formula Ic (yield: 87%). TLC: Rf (30% MeOH in DCM) 0.4. HR-ESI MS (ESI): *m*/*z* 457.1102 calc. for [C₄₉H₃₉N₈OF₃Ru]²⁺: 457.1115.

### General procedure for the synthesis of complex compounds of formulae Im, In, Io, IVa and IVb.

The corresponding ligand compound of formula **IIm, IIn, IIo, Va** or **Vb** and [Ru(bpy)₂Cl₂] (1.1 eq) were dissolved in a 1:1 (v/v) mixture of EtOH/H₂O and the reaction mixture was stirred for 24-48 h at 80-90 °C under an argon atmosphere. The reaction was followed by HPLC-MS. Once the total conversion was reached, the solvent was evaporated to dryness and the product was purified by silica column chromatography with DCM and increasing the eluent polarity with MeOH. Compounds were obtained as a racemic mixture of the chloride salt and characterized by HR-ESI MS.

**Im**: HR-ESI MS (ESI): *m*/*z* 470.1193 calc. for [C₅₁H₄₁F₃N₈ORu]²⁺: 470.1199.

**In:** HR-ESI MS (ESI): *m*/*z* 431.1336 calc. for [C₄₉H₄₄N₈ORu]²⁺: 431.1336.

**Io**: HR-ESI MS (ESI): *m*/*z* 483.1282 calc. for [C₅₃H₄₃F₃N₈ORu]²⁺: 483.1272.

**IVa:** HR-ESI MS (ESI): *m*/*z* 444.1420 calc. for [C₅₁H₄₄N₈ORu]²⁺: 444.1414.

**IVb:** HR-ESI MS (ESI): *m*/*z* 431.0972 calc. for [C₄₇H₃₆N₈O₃Ru]²⁺: 431.0972.

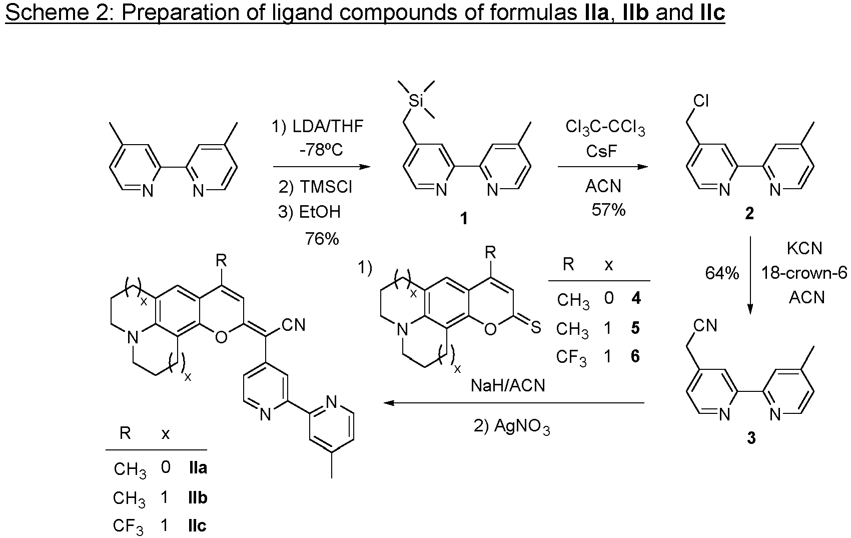

4-Methyl-4'-((trimethylsilyl)methyl)-2,2'-bipyridine, of formula **1.** A solution of 4,4'-dimethyl-2,2'-bipyridine (2 g, 10.86 mmol) in anhydrous THF (80 mL) was added dropwise via cannula to a cold (-78 °C) solution of LDA in THF (12 mL, 11.95 mmol) under an Ar atmosphere. The resulting maroon mixture was stirred for 1 h at -78 °C. Then, trimethylsilyl chloride (2 mL, 11.95 mmol) was added to the crude, which turned to blue and, exactly 10 seconds later, 10 mL of absolute ethanol were added carefully, which caused the solution to become yellow. The crude was transferred to a separatory funnel containing a saturated solution of NaHCO₃ (200 mL) and extracted with DCM (3x150 mL). The combined organic phases were washed with brine (150 mL), dried over anhydrous Na₂SO₄, filtered and evaporated to dryness to obtain 2.12 g of the title compound, as a yellow solid (yield: 76%), which was used without further purification.

4-(Chloromethyl)-4'-methyl-2,2'-bipyridine, of formula **2.** 4-Methyl-4'-((trimethylsilyl)methyl)-2,2'-bipyridine (2.12 g, 7.80 mmol), hexachloroethane (7.39 g, 31.2 mmol) and cesium fluoride (4.76 g, 31.2 mmol) were dissolved in anhydrous ACN (120 mL) under an Ar atmosphere and the resulting solution was stirred at 60 °C for 3.5 h. The reaction mixture was partitioned between 50 mL of H₂O and 50 mL of AcOEt and transferred to a separatory funnel. The aqueous phase was extracted with ethyl acetate (3x50 mL) and the combined organic phases were washed with brine (50 mL), dried over anhydrous Na₂SO₄, filtered and evaporated to dryness. The product was purified by column chromatography (silica gel, 0-10% MeOH in DCM) to obtain 1.01 g of the title compound, as a yellow solid (yield: 57%). TLC: Rf (1:9 MeOH/DCM) 0.6. LR-ESI MS (ESI): *m*/*z* 218.8 calc. for [C₁₂H₁₁N₂Cl+H]⁺: 218.1.

2-(4'-Methyl-f2,2'-bipyridin1-4-yl) acetonitrile, of formula **3.** 4-(Chloromethyl)-4'-methyl-2,2'-bipyridine (1 g, 4.57 mmol), 18-crown-6 (26.8 mg, 0.09 mmol) and KCN (3.39 g, 36.5 mmol) were dissolved in 100 mL of ACN and stirred overnight at room temperature. HPLC-MS analysis of the crude revealed that the product was barely formed. Then, 214 mg of 18-crown-6 (0.81 mmol) were added and the solution was stirred overnight at 50 °C. After confirmation by HPLC-MS that the reaction had finished, the reaction mixture was evaporated to dryness, re-dissolved in deionized water (100 mL) and extracted with DCM (3x100 mL). The combined organic phases were dried over anhydrous Na₂SO₄, filtered and evaporated to dryness. After purification by column chromatography (silica gel, 50-100% AcOEt in hexanes), 615 mg of the title compound were obtained as a white solid (yield: 64%). TLC: Rf (AcOEt 100%) 0.7. HR-ESI MS (ESI): *m*/*z* 210.1026 calc. for [C₁₃H₁₁N₃+H]⁺: 210.1025.

Thiocoumarin of formula **4.** 7-Diethylamino-4-methylcoumarin (1.72 g, 7.42 mmol) and Lawesson's reagent (1.65 g, 4.08 mmol) were dissolved in toluene (40 mL) and heated at 100 °C for 12 h. After evaporation under reduced pressure, the dark residue was purified by column chromatography (silica gel, DCM) to give the thiocoumarin of formula **4,** as an orange solid (1.70 g, 92%): TLC Rf (DCM) 0.50. HRMS (ESI-TOF) *m*/*z* [M+H]⁺ calcd. for C₁₄H₁₈NOS 248.1109, found 248.1099.

Thiocoumarin of formula **5.** 2,3,6,7-Tetrahydro-9-methyl-1*H*,5*H*-quinolizino[9,1-*gh*]coumarin (2 g, 7.8 mmol) and Lawesson's reagent (1.89 g, 4.7 mmol) were dissolved in 100 mL of toluene and stirred overnight at 100 °C. The dark-green crude was evaporated to dryness and the product was isolated by column chromatography (silica gel, 0-70% DCM in hexanes). 1.44 g of the thiocoumarin of formula **5** as an orange solid were obtained (yield: 68%). TLC: Rf (100% DCM) 0.8. HR-ESI MS (ESI): *m*/*z* 272.1104 calc. for [C₁₆H₁₇NOS+H]⁺: 272.1102.

Thiocoumarin of formula **6.** 2,3,6,7-Tetrahydro-9-trifluoromethyl-1*H*,5*H*-quinolizino[9,1-gh]coumarin (1 g, 3.23 mmol) and Lawesson's reagent (791 mg, 1.94 mmol) were dissolved in toluene (50 mL). The yellow solution was stirred overnight at 100 °C. The dark-red solution was evaporated to dryness and the thiocoumarin of formula 6 was isolated through column chromatography (silica-gel, 0-40% DCM in hexanes). 1.14 g of a golden maroon solid were obtained (yield: 95%). Golden maroon solid. TLC: Rf (100% DCM) 0.9. HR-ESI MS (ESI): *m*/*z* 326.0821 calc. for [C₁₆H₁₄F₃NOS+H]⁺: 326.0816.

Ligand compound of formula **IIa**. To a solution of sodium hydride (568 mg of a 60% dispersion in mineral oil, 14.15 mmol) and compound **3** (712 mg, 3.40 mmol) in anhydrous acetonitrile (300 mL), 7-(*N*,*N*-diethylamino-4-methyl-2-thiocoumarin (703 mg, 5.66 mmol) was added. The orange solution was stirred for 3 h. Then, silver nitrate (962 mg, 4.98 mmol) was added and the reaction mixture was stirred for 2 h at room temperature. The dark-maroon solution was analyzed by HPLC-MS to confirm the formation of the desired product in the crude, which was evaporated under reduced pressure. The product was isolated by silica column chromatography starting with hexanes and increasing the eluent polarity with DCM first (0-100%) and then with MeOH (0-10%). 451 mg of an orange/red solid were obtained (yield: 37%), identified as ligand compound of formula **IIa**. TLC: Rf (1:9 MeOH/DCM) 0.5. HR-ESI MS (ESI): *m*/*z* 423.2181 calc. for [C₂₇H₂₆N₄O+H]⁺: 423.2179

Ligand compound of formula **IIb**. To a solution of NaH (38 mg of a 60% dispersion in mineral oil, 1.58 mmol) and compound **3** (46 mg, 0.22 mmol) in anhydrous ACN (12 mL), thiocoumarin of formula 5 (50 mg, 0.18 mmol) was added. The maroon solution was stirred at 50 °C for 3 h. Then, silver nitrate (71 mg, 0.41 mmol) was added and the crude was stirred for 2 h at room temperature. The product was isolated by silica column chromatography starting with hexanes and increasing the eluent polarity with DCM first (0-100%) and then with MeOH (0-10%). 20 mg of an orange/brown solid were obtained (yield: 20%), identified as ligand compound of formula **IIb**. TLC: Rf (5% MeOH/DCM) 0.5. HR-ESI MS (ESI): *m*/*z* 447.2186 calc. for [C₂₉H₂₆N₄O+H]⁺: 447.2179.

Ligand compound of formula **IIc**. To a solution of NaH (18 mg of a 60% dispersion in mineral oil, 0.77 mmol) and compound **3** (41 mg, 0.18 mmol) in anhydrous ACN (35 mL), thiocoumarin of formula 6 (50 mg, 0.15 mmol) was added. The maroon solution was stirred for 3 h. Then, silver nitrate (56 mg, 0.32 mmol) was added and the crude was stirred for 2 h at room temperature. The product was isolated by silica column chromatography starting with hexanes and increasing the eluent polarity with DCM first (0-100%) and then with MeOH (0-20%). 58 mg of bright red solid were obtained (yield: 75%), identified as ligand compound of formula **IIc**. Bright red solid. TLC: Rf (5% MeOH/DCM) 0.5. HR-ESI MS (ESI): *m*/*z* 501.1889 calc. for [C₂₉H₂₃F₃N₄O+H]⁺: 501.1887.

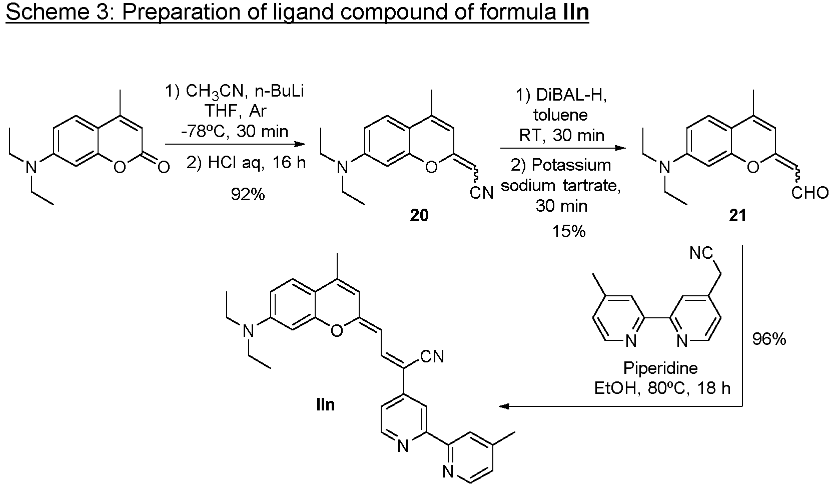

Nitrile coumarin of formula **20.** To a cold solution (-78 °C) of CH₃CN (2.42 mL, 34.6 mmol) in anhydrous THF (40 mL), n-BuLi (13.8 mL, 2.5 M in hexanes, 34.6 mmol) was added under an Ar atmosphere. The resulting transparent and colorless solution was stirred for 15 min at -78 °C, time after which the appearance of a white suspension was observed. Then, a solution of 7-(diethylamino)-4-methyl-2H-chromen-2-one coumarin (2.008 g, 8.65 mmol) in anhydrous THF (30 mL) was slowly added and the reaction mixture was stirred for 30 min at -78 °C under Ar. The resulting white mixture was quenched by addition of saturated aqueous NH₄Cl (35 mL) while still at -78 °C, and a white solid precipitated. The mixture was warmed to room temperature and extracted with AcOEt (3x50 mL). The combined organic phases were evaporated to dryness. To the resulting orange crude oil, 0.5 M aqueous HCl (200 mL) was added and the mixture stirred vigorously for 16 h. The solution turned into brown color and a yellow solid precipitated. The precipitate was dissolved adding 50 mL of DCM and the mixture was extracted with DCM (3x100 mL), dried over anhydrous MgSO₄, filtered and evaporated to dryness under reduced pressure to obtain a brown solid. The crude mixture was purified by silica column chromatography (0-22% AcOEt in hexanes). 2.01 g of the title compound, as bright orange solid were obtained (yield: 92%). Orange solid. TLC: Rf (50% AcOEt in hexanes) 0.61. LR-ESI MS (ESI): *m*/*z* 255.2 calc. for [C₁₆H₁₈N₂O+H]⁺: 255.1492.

Coumarin aldehyde of formula **21.** Nitrile coumarin of formula 20 (500 mg, 1.97 mmol) was dissolved in anhydrous toluene (25 mL) under an Ar atmosphere and the mixture was let stir for 10 min (orange solution). Then, 1 M toluene solution of DIBALH was added (3.0 mL, 2.95 mmol) and the reaction mixture stirred for 30 min at room temperature under an Ar atmosphere. The crude was cooled in an ice-bath and acetone (10 mL) was added for decomposition of the excess reagent. After addition of saturated potassium sodium tartrate (40 mL), the crude was extracted with DCM (3 x 50 mL) and the organic phases washed with brine (50 mL), dried over anhydrous MgSO4, filtered and evaporated to dryness under reduced pressure. The compound was purified by silica column chromatography with hexanes increasing the polarity with DCM first (0-100%) and with MeOH then (0-0.5%). 78 mg of the title compound, as a yellow solid, were obtained (yield: 15%). TLC: Rf (5% MeOH in DCM) 0.52. LR-ESI MS (ESI): *m*/*z* 258.2 calc. for [C₁₆H₁₉NO₂+H]⁺: 258.1489.

Ligand compound of formula **IIn**. To a solution of the coumarin aldehyde of formula 21 (47 mg, 0.18 mmol) in absolute ethanol (10 mL), piperidine (84 mg, 1.0 mmol) and 2-(4'-methyl-[2,2'-bipyridin]-4-yl)acetonitrile (44 mg, 0.20 mmol) were added. The reaction mixture was stirred at 80 °C overnight. After evaporation to dryness under reduced pressure, the compound was purified by silica column chromatography with hexanes increasing the polarity with DCM first (0-100%) and with MeOH then (0-2%). 79 mg of a violet solid, identified as the ligand compound of formula IIn, were obtained (yield: 96%). TLC: Rf (5% MeOH in DCM) 0.38. HR-ESI MS (ESI): *m*/*z* 449.2336 calc. for [C₂₉H₂₈N₄O+H]⁺: 449.2336.

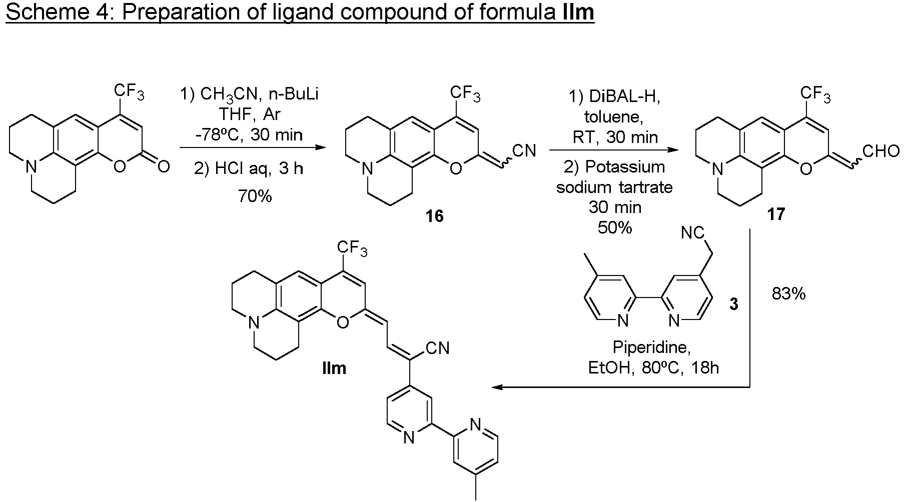

Nitrile coumarin of formula **16.** To a cold solution (-78 °C) of CH₃CN (0.7 mL, 13.4 mmol) in anhydrous THF (40 mL), n-BuLi (5.2 mL, 2.5 M in hexanes, 13 mmol) was added under an Ar atmosphere. The resulting transparent and colorless solution was stirred for 15 min at -78 °C, time after which the appearance of a white suspension was observed. Then, a solution of 2,3,6,7-tetrahydro-9-trifluoromethyl-1H,5H-quinolizino[9,1-gh] coumarin (1.023 g, 3.31 mmol) in anhydrous THF (35 mL) was slowly added and the reaction mixture was stirred for 30 min at -78 °C under Ar. The resulting orange mixture was quenched by addition of saturated aqueous NH₄Cl (35 mL) while still at -78 °C, and a white solid precipitated. The mixture was warmed to room temperature and extracted with AcOEt (3x50 mL). The combined organic phases were evaporated to dryness. To the resulting crude oil, 0.5 M aqueous HCl (200 mL) was added and the mixture stirred vigorously for 3 h. The solution color turned from orange to brown. This solution was extracted with AcOEt (3x80 mL), dried over anhydrous MgSO₄, filtered and evaporated to dryness under reduced pressure. The crude mixture was purified by flash chromatography silica gel, 0-15% AcOEt in hexanes. 760 mg of the title compound, as bright orange solid were obtained (yield: 70%). Orange solid. TLC: Rf (25% AcOEt in hexanes) 0.38. HR-MS (ESI): *m*/*z* 332.1209 calc. for [C₁₈H₁₅F₃N₂O+H]⁺: 333.1215.

Coumarin aldehyde of formula **17.** Nitrile coumarin of formula 16 (300 mg, 0.90 mmol) was dissolved in anhydrous toluene (35 mL) under an Ar atmosphere and the mixture was let stir for 10 min (orange solution). Then, 1 M toluene solution of DIBALH was added (1.35 mL, 1.35 mmol) and the reaction mixture stirred for 30 min at room temperature under an Ar atmosphere (the color changed to maroon). The crude was cooled in an ice-bath and acetone (4 mL) was added for decomposition of the excess reagent (the color changed from maroon to dark red). After addition of saturated potassium sodium tartrate (25 mL), the crude was extracted with AcOEt (3 x 25 mL) and the organic phases washed with water (25 mL) and brine (25 mL), dried over anhydrous MgSO4, filtered and evaporated to dryness under reduced pressure. The compound was purified by silica column chromatography with hexanes increasing the polarity with DCM first (0-100%) and with MeOH then (0-0.5%). 150 mg of the title compound, as a red solid, were obtained (yield: 50%). TLC: Rf (5% MeOH in DCM) 0.74. HR-MS (ESI): *m*/*z* 336.1206 calc. For [C₁₈H₁₆F₃NO₂+H]⁺: 336.1211.

Ligand compound of formula **IIm.** To a solution of the coumarin aldehyde of formula 17 (50 mg, 0.15 mmol) in absolute ethanol (7 mL), piperidine (70 mg, 0.83 mmol) and 2-(4'-methyl-[2,2'-bipyridin]-4-yl)acetonitrile (46 mg, 0.22 mmol) were added. The reaction mixture (dark maroon) was stirred at 80 °C overnight. After evaporation to dryness under reduced pressure, the compound was purified by silica column chromatography with hexanes increasing the polarity with DCM first (0-100%) and with MeOH then (0-2%). 66 mg of a violet solid, identified as the ligand compound of formula **IIm,** were obtained (yield: 83%). TLC: Rf (5% MeOH in DCM) 0.4. HR-ESI MS (ESI): *m*/*z* 527.2077 calc. for [C₃₁H₂₅F₃N₄O+H]⁺: 527.2053.

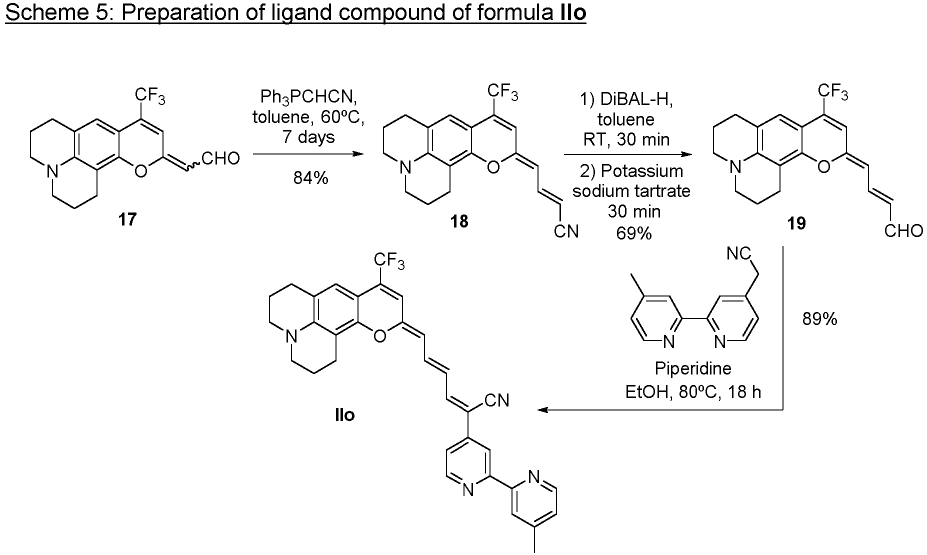

Nitrile coumarin of formula **18**. To a solution of the coumarin aldehyde formula 17 (207 mg, 0.62 mmol) in toluene (15 mL), (triphenylphosphoranylidene)acetonitrile (850 mg, 2.82 mmol) was added. The solution was degassed with Ar and then the vial was sealed, and the mixture was stirred at 60 °C for 7 days. Once the starting aldehyde was totally consumed, deionized water (H₂O) was added to the crude and then extracted with AcOEt (3 x 30 mL). The combined organic phases were dried over anhydrous MgSO₄, filtered, and evaporated to dryness under reduced pressure and the crude was purified by silica column chromatography with hexanes increasing the polarity with AcOEt (0-12%). 184 mg of the title compound, as a red solid, were obtained (yield: 84%). TLC: Rf (50% AcOEt in hexanes) 0.90. HR-MS (ESI): *m*/*z* 359.1366 calc. for [C₂₀H₁₇F₃N₂O+H]⁺: 359.1357.

Coumarin aldehyde of formula **19.** Nitrile coumarin of formula 18 (103 mg, 0.29 mmol) was dissolved in anhydrous THF (30 mL) under an Ar atmosphere and the mixture was cooled in an ice bath. Then, 1 M toluene solution of DIBALH was added (3.0 mL, 2.95 mmol) and the reaction mixture stirred for 30 min at room temperature under an Ar atmosphere. The crude was cooled in an ice-bath and acetone (10 mL) was added for decomposition of the excess reagent. After addition of saturated potassium sodium tartrate (25 mL) and let stir for 30 min, the crude was extracted with DCM (3 x 50 mL) and the organic phases washed with brine (50 mL), dried over anhydrous MgSO4, filtered and evaporated to dryness under reduced pressure. The compound was purified by silica column chromatography with hexanes increasing the polarity with DCM first (0-100%) and with MeOH then (0-0.5%). 78 mg of the title compound, as a yellow solid, were obtained (yield: 69%). TLC: Rf (5% MeOH in DCM) 0.52. HR-MS (ESI): *m*/*z* 362.1362 calc. for [C₂₀H₁₇F₃N₂O+H]⁺: 362.1359.

Ligand compound of formula **IIo.**To a solution of the coumarin aldehyde of formula 19 (32 mg, 0.088 mmol) in absolute ethanol (10 mL), piperidine (45 mg, 0.531 mmol) and 2-(4'-methyl-[2,2'-bipyridin]-4-yl)acetonitrile (23 mg, 0.11 mmol) were added. The reaction mixture was stirred at 80 °C overnight. After evaporation to dryness under reduced pressure, the compound was purified by silica column chromatography with hexanes increasing the polarity with DCM first (0-100%) and with MeOH then (0-1%). 44 mg of a violet solid, identified as the ligand compound of formula **IIo,** were obtained (yield: 89%). TLC: Rf (5% MeOH in DCM) 0.59. LR-MS (ESI): *m*/*z* 553.25 calc. for [C₃₃H₂₇F₃N₄O+H]⁺: 553.2210.

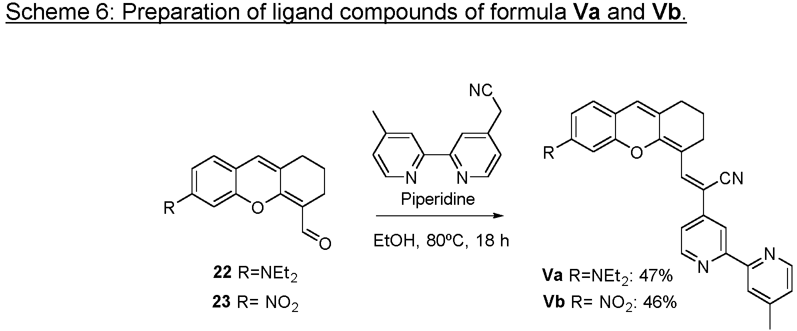

Ligand compound of formula **Va.** To a solution of the coumarin aldehyde of formula 22 (92 mg, 0.32 mmol) in absolute ethanol (10 mL), piperidine (166 mg, 2.0 mmol) and 2-(4'-methyl-[2,2'-bipyridin]-4-yl)acetonitrile (68 mg, 0.32 mmol) were added. The reaction mixture was stirred at 80 °C overnight. After evaporation to dryness under reduced pressure, the compound was purified by silica column chromatography with hexanes increasing the polarity with DCM first (0-100%) and with MeOH then (0-1%). 73 mg of a violet solid, identified as the ligand compound of formula **Va,** were obtained (yield: 47%). TLC: Rf (5% MeOH in DCM) 0.59. HR-MS (ESI): *m*/*z* 475.2495 calc. for [C₃₁H₃₀N₄O+H]⁺: 475.2492.

Ligand compound of formula **Vb.** To a solution of the coumarin aldehyde of formula 23 (40 mg, 0.155 mmol) in absolute ethanol (8 mL), piperidine (79 mg, 0.932 mmol) and 2-(4'-methyl-[2,2'-bipyridin]-4-yl)acetonitrile (33 mg, 0.155 mmol) were added. The reaction mixture was stirred at 80 °C overnight. After evaporation to dryness under reduced pressure, the compound was purified by silica column chromatography with hexanes increasing the polarity with DCM first (0-100%) and with MeOH then (0-2%). 32 mg of a brown solid, identified as the ligand compound of formula Vb, were obtained (yield: 46%). TLC: Rf (5% MeOH in DCM) 0.30. LR-MS (ESI): *m*/*z* 449.07 calc. for [C₂₇H₂₀N₄O₃+H]⁺: 449.1608.

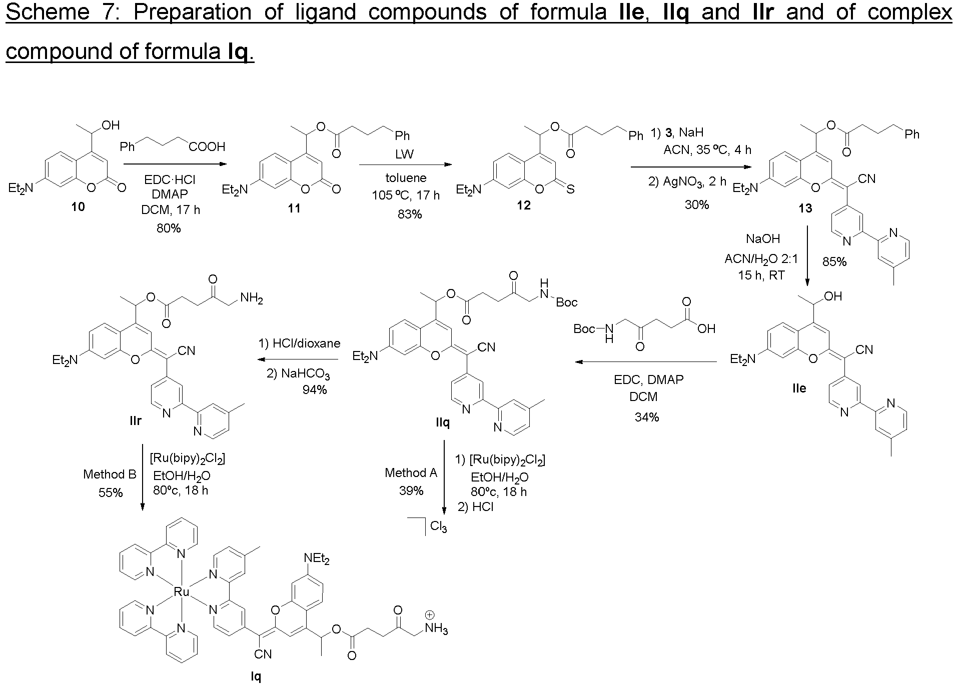

Compound of formula 11. A mixture of the coumarin of formula 10 (1.60 g, 6.12 mmol), phenylbutyric acid (1.50 g, 9.15 mmol), EDC (1.75 g, 9.15 mmol) and 4-dimethylaminopyridine (DMAP) (1.12 g, 9.15 mmol) was cooled at 0 °C under an argon atmosphere and then dissolved in DCM (100 mL). The mixture was stirred at 0 °C for 15 minutes and then 17 h at room temperature. Then, the solution was washed with saturated NH₄Cl (2 x 100 mL), 5% aqueous NaHCOs (1 × 100 mL, 2 × 50 mL) and deionized water (100 mL). The organic layer was dried over anhydrous MgSO₄, filtered and evaporated under reduced pressure. The product was isolated by silica column chromatography (silica gel, 50-100% DCM in hexanes, 1-3% MeOH in DCM) to give 2 g of the title compound as a yellow/orange solid (yield: 80%). TLC: Rf (DCM) 0.6. HR-ESI MS (ESI): *m*/*z* 408.2169 calc. for [C₂₅H₂₉NO₄+H]⁺: 408.2175.

Compound of formula **12.** Lawesson's reagent (1.54 g, 3.81 mmol) was added to a solution of compound of formula 11 (1.94 g, 4.75 mmol) in toluene (60 mL) under an Ar atmosphere. The mixture was stirred at 105 °C in the dark overnight. A color change was observed from dark brown to pale yellow color. After removal of the solvent under reduced pressure, the product was isolated by column chromatography (silica gel, 100-30 % hexanes in DCM) to give 1.67 g of the title compound as an orange solid (yield: 83 %). TLC: R_{f} (DCM)= 0.78. HR-ESI MS (ESI): *m*/*z* 424.1936 calc. for [C₂₅H₂₉NO₃S+H]⁺: 424.1946.

Compound of formula **13.** First, sodium hydride (68.3 mg of a 60% dispersion in mineral oil, 1.71 mmol) and 2-(4'-methyl-[2,2'-bipyridin]-4-yl)acetonitrile (formula **3)** (357 mg, 1.71 mmol) were dissolved in anhydrous ACN (30 mL) and stirred for 15 min at 35 °C under an argon atmosphere. Then, a solution of the coumarin of formula **12** (362 mg, 0.85 mmol) in anhydrous ACN (15 mL) and added to the previous flask and the resulting reaction mixture was stirred for a further 4 h in the dark at 35 °C under an argon atmosphere. After 4 hours, AgNO₃ (363 mg, 2.14 mmol, 2.5 equiv.) was added and it was stirred for another 2 h. A color change was observed from brown to dark red/maroon color. After evaporation of the solvent under reduced pressure, the product was isolated by silica column chromatography starting with a 1:1 mixture of DCM and hexanes and increasing the eluent polarity with DCM by 10 until 100% and then with MeOH (0-1%) in DCM. 155 mg of an orange/red solid were obtained (yield: 30%), identified as the compound of formula **13.** TLC: R_{f} (10% MeOH in DCM)= 0.4. HR-ESI MS (ESI): *m*/*z* 599.3014 calc. for [C₃₈H₃₈N₄O₃+H]+: 599.3022.

Ligand compound of formula **IIe.** To a solution of the ligand compound of formula 13 (41.7 mg, 0.070 mmol) in 2:1 (v/v) ACN/H₂O (7 mL), 10% NaOH (6.65 mL, 0.070 mmol) was added and the reaction mixture was stirred overnight at room temperature in the dark. Reaction mixture color changed from orange/yellow to a bright orange with solid particles. After evaporation to dryness under reduced pressure, the crude was purified by flash chromatography (DCM in hexanes (50-100%) and then MeOH in DCM (0-10%), Puriflash system, Silica column PF-DLE-F0012) affording 26.9 mg of an orange/red solid (yield: 85%), identified as the ligand compound of formula **IIe.** TLC: Rf (10% MeOH in DCM) 0.57. HR-ESI MS (ESI): *m*/*z* 452.56 calc. for [C₂₉H₂₈N₄O₂+H]⁺: 453.2291.

Ligand compound of formula **IIq.** 4-Dimethylaminopyridine (DMAP) (20 mg, 0.164 mmol) and 1-ethyl-3-(3'-dimethylaminopropyl)carbodiimide hydrochloride (EDC HC!) (38 mg, 0.198 mmol) were sequentially added to an ice-cooled solution of *N*-Boc-5-aminolevulinic acid (38 mg, 0.164 mmol) in DCM (10 mL) under an argon atmosphere. After the mixture was stirred for 15 min at 0 °C, a solution of compound of formula IIe (61 mg, 0.135 mmol) in DCM (5 mL) was added dropwise at the same temperature. Then, the ice bath was removed, and the resulting mixture was further stirred for 16 h at room temperature. After completion of the reaction (TLC, HPLC), the volatiles were concentrated under reduced pressure and the resulting crude was purified by flash column chromoatography (silica gel, MeOH in DCM 0-5%) to afford 31 mg of an orange/red solid (yield: 34%), identified as the ligand compound of formula **IIq.** LR-MS (ESI): *m*/*z* [M+H]⁺ calcd for C₃₈H₄₃N₅O₆ 666.33, found 666.57.

Ligand compound of formula **IIr.** To an ice-cooled solution of ligand compound of formula Ilq (15 mg, 0.022 mmol) in anhydrous THF (2 mL) was added 4N HCl in dioxane (675 µL, 2.70 mmol), and the resulting mixture was allowed to warm to room temperature and further stirred for 5 h. After completion of the reaction (HPLC-MS), the reaction mixture was neutralized with 10% (w/v) aq. NaHCOs (10 mL), diluted with water (20 mL), extracted with DCM (3 x 20 mL), and the combined organic extracts were dried over anhydrous MgSO₄, filtered and concentrated under reduced pressure to give the crude ligand compound of formula **IIr** (12 mg, yield: 94%) as an orange/red solid, which was used in the following step without any further purification. LR-MS (ESI): *m*/*z* [M+H]⁺ calcd for C₃₃H₃₅N₅O₄ 566.28, found 566.45.

Complex compound of formula **Iq.** Method A: Ligand compound of formula Ilq (16 mg, 0.024 mmol) and [Ru(bpy)₂Cl₂] (10 mg, 0.021 mmol) were dissolved in 4 mL of a 3:1 (v/v) solution of EtOH/H₂O under an Ar atmosphere. The reaction mixture was stirred overnight at 80 °C and analyzed by HPLC-MS to confirm the formation of the product. The reaction mixture was evaporated to dryness and the residue was taken up in anhydrous THF (2 mL), treated with 4N HCl in dioxane (600 µL, 2.37 mmol) at 0 °C, allowed to warm to room temperature and further stirred for 5 h. After completion of the reaction (HPLC-MS), the reaction mixture was neutralized with 10% (w/v) aq. NaHCO₃ (5 mL), diluted with water (20 mL), extracted with DCM (3 x 20 mL), and the combined organic extracts were dried over anhydrous MgSO₄, filtered and concentrated under reduced pressure to give the crude. Method B: Ligand compound of formula IIr (11 mg, 0.019 mmol) and [Ru(bpy)₂Cl₂] (9 mg, 0.019 mmol) were dissolved in 4 mL of a 3:1 (v/v) solution of EtOH/H₂O under an Ar atmosphere. The reaction mixture was stirred overnight at 80 °C and analyzed by HPLC-MS to confirm the formation of the product. The reaction mixture was evaporated to dryness to give the crude. In both methods, the crude was purified by preparative HPLC (C18 column; mobile phase, A: 0.1% HCOOH in H₂O, B: 0.1% HCOOH in ACN; gradient: 10 to 80% B in 30 min) and the desired complex compound of formula Iq was obtained as a maroon solid (Method A: 8 mg, yield: 39%; Method B: 10 mg, yield: 55%). Analytical HPLC (C18 column; mobile phase, A: 0.1% HCOOH in H₂O, B: 0.1% HCOOH in ACN; gradient: 5 to 100% B in 5 min): Rt = 2.14 min. LR-MS (ESI): *m*/*z* [M]²⁺ calcd for C₅₃H₅₁N₉O₄Ru²⁺ 489.65, found 489.60.

### Example 2. Conjugatable metal complex compounds of formulae VI, VII and VIII.

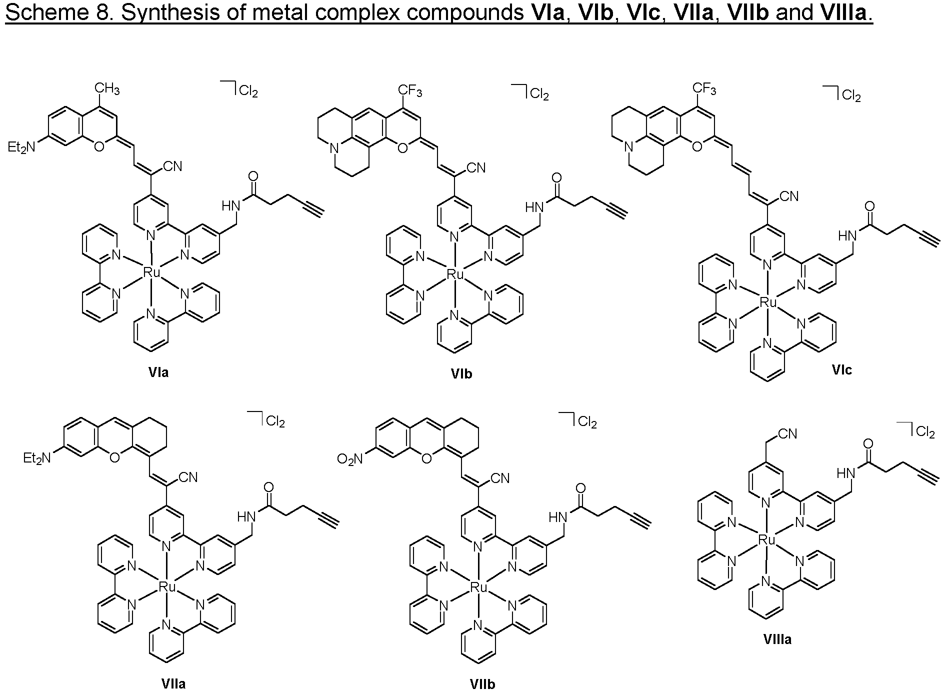

Complex compound of formula **VIIIa.** Compound **26** (25 mg, 0.080 mmol) and [Ru(bpy)₂Cl₂] (40 mg, 0.080 mmol) were dissolved in EtOH (5 mL) and the reaction mixture was stirred overnight at 80 °C. The solvent was evaporated to dryness and the product was purified by silica column chromatography starting with ACN and increasing the eluent polarity with NaCl 0.1 M (0-15%) 46 mg of a dark red solid were obtained (yield: 70%). LR-ESI MS (ESI): m/z 358.9 calc. for [C₃₈H₃₂N₈ORu]²⁺: 359.09

General procedure for the synthesis of complex compounds of formulae **VIa, VIb, VIc, Vila** and **VIIb** . Complex compound of formula **VIIIa,** the required coumarin aldehyde of formula **17, 19, 21, 22** or **23** (1 mol equiv.) and piperidine (3 mol equiv.) were dissolved in absolute EtOH. The reaction mixture was stirred at 80 °C for 3 h under an argon atmosphere. The reaction was followed by HPLC-MS. Once the total conversion was reached, the solvent was evaporated to dryness and the product was purified by silica column chromatography with DCM and increasing the eluent polarity with MeOH or alternatively with ACN and increasing the eluent polarity with aqueous NaCl 0.1 M (0-15%). Compounds were obtained as a racemic mixture of the chloride salt and characterized by LR-ESI MS.
**VIa:** LR-ESI MS (ESI): *m*/*z* 478.53 calc. for [C₅₄H₄₉N₉O₂Ru]²⁺ : 478.65
**VIb:** LR-ESI MS (ESI): *m*/*z* 517.25 calc. for [C₅₆H₄₆F₃N₉O₂Ru]²⁺: 517.64
**VIc:** LR-ESI MS (ESI): *m*/*z* 530.31 calc. for [C₅₈H₄₈F₃N₉O₂Ru]²⁺: 530.65
Vila: LR-ESI MS (ESI): *m*/*z* 491.33 calc. for [C₅₆H₅₁N₉O₂Ru]²⁺: 491.66
Vllb: LR-ESI MS (ESI): *m*/*z* 478.75 calc. for [C₅₂H₄₁N₉O₄Ru]²⁺: 478.62

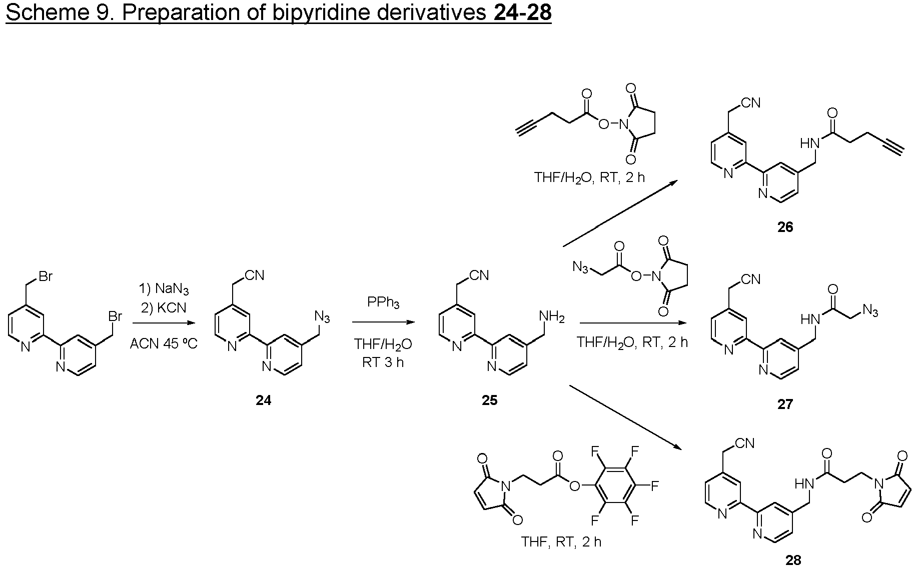

Compound of formula **24.** 4,4'-bis(bromomethyl)-2,2'-bipyridine (500 mg, 1.46 mmol) and sodium azide (85 mg, 1.32 mmol) were dissolved in ACN (20 mL) and stirred at 45 °C for 2 h. Next the crude was filtered and evaporated to dryness under reduced pressure to provide a solid that was dissolved in a 4:1 (v/v) DCM/MeOH mixture and washed with brine. The organic phase was dried over anhydrous MgSO₄, filtered and evaporated to dryness under reduced pressure. The resulting solid, KCN (475 mg, 7.31 mmol) and KI (243 mg, 1.46 mmol) were dissolved in ACN (20 mL) and mixture was stirred at 45 °C for 16 h. Then, the crude was filtered and evaporated to dryness under reduced pressure. The resulting solid was dissolved in a 4:1 (v/v) DCM/MeOH mixture and washed with brine. The organic phase was dried over anhydrous MgSO₄, filtered and evaporated to dryness under reduced pressure to afford a reddish oil (253 mg) containing a 1:1 mixture of **24** and 4,4'-bis(azidomethyl)-2,2'-bipyridine which was used without further purification in the next step. LR-ESI MS (ESI): *m*/*z* 251.0 calc. for [C₁₃H₁₀N₆+H]⁺: 251.10.

Compound of formula **25.** The previous crude containing compound **24** was dissolved in a 3:1 (v/v) THF/H₂O mixture and reacted over triphenylphosphine, polymer-bound (1.2 g, 1.4-2.0 mmol/g) for 3 h at RT. Next, the polymer was removed by filtration and the crude was evaporated to dryness under reduced pressure to afford a reddish oil (215 mg) corresponding to a 1:1 mixture of **25** and [2,2'-bipyridine]-4,4'-diyldimethanamine, which was used without further purification in the next step. LR-ESI MS (ESI): *m*/*z* 225.1 calc. for [C₁₃H₁₂N₄+H]⁺. 225.11.

Compound of formula 26. The previous crude containing compound **25** (73 mg), 4-pentynoic acid succinimidyl ester (127 mg, 0.326 mmol) and DIPEA (42 mg, 0.326 mmol) were dissolved in a 1:1 (v/v) THF/H₂O mixture and stirred at RT for 2 h. The crude was evaporated to dryness under reduced pressure, and compound **26** was purified by silica column chromatography with hexanes increasing the polarity with DCM first (0-100%) and then with MeOH then (0-5%). 26 mg of the title compound, as a yellowish solid, were obtained (yield: 26%). LR-ESI MS (ESI): *m*/*z* 305.1 calc. for [C₁₈H₁₆N₄O+H]⁺: 305.14.

Compound of formula **27.** Crude containing compound **25** (132 mg), azido acetic acid succinimidyl ester (233 mg, 1.18 mmol) and DIPEA (152 mg, 1.18 mmol) were dissolved in a 1:1 (v/v) THF/H₂O mixture and stirred at RT for 2 h. The crude was evaporated to dryness under reduced pressure, and compound **27** was purified by silica column chromatography with hexanes increasing the polarity with DCM first (0-100%) and then with MeOH (0-5%). 40 mg of the title compound, as a red solid, were obtained (yield: 11%). LR-ESI MS (ESI): *m*/*z* 308.1 calc. for [C₁₅H₁₃N₇O+H]⁺: 308.12.

Compound of formula **28.** The previous crude containing compound **25** (40 mg), maleimido propionic acid pentafluorophenol ester (118 mg, 0.303 mmol) and DIPEA (39 mg, 0.303 mmol) were dissolved in THF and stirred at RT for 2 h. The crude was evaporated to dryness under reduced pressure, and compound **28** was purified by silica column chromatography with hexanes increasing the polarity with DCM first (0-100%) and then with MeOH then (0-5%). 15 mg of the title compound, as a yellowish, were obtained (yield: 22%). LR-ESI MS (ESI): *m*/*z* 376.1 calc. for [C₂₀H₁₇N₅O₃+H]⁺: 376.14

### Example 3. Conjugatable metal complex compounds of formulae XIII and XIV.

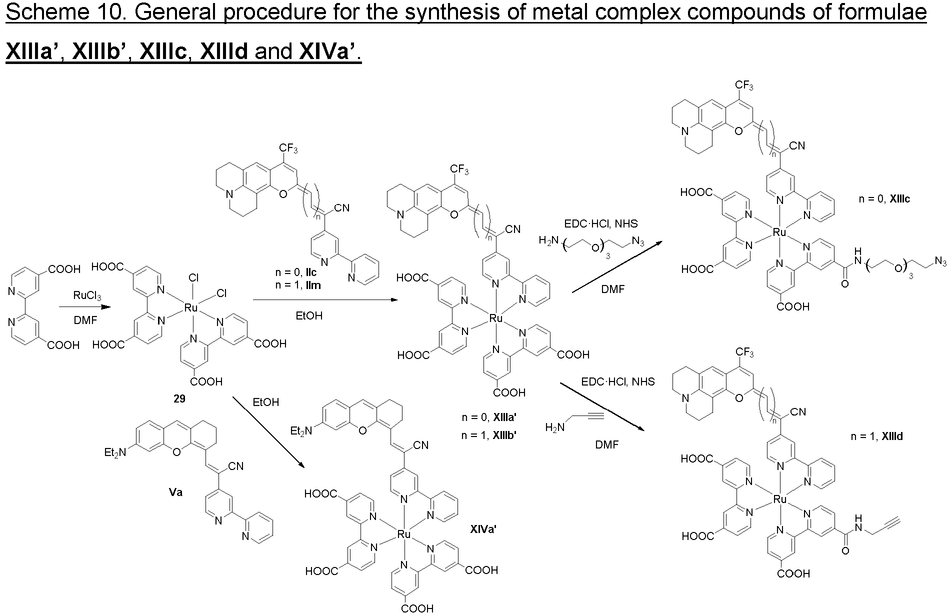

Compound of formula **29.** To a stirred solution of 2,2'-bipyridine-4,4'-dicarboxylic acid (540 mg, 2.22 mmol, 2.0 equiv.) in degassed DMF (10 mL) under an argon atmosphere, RuCl₃.H₂O (250 mg, 1.11 mmol, 1.0 equiv.) was added, and the resulting mixture was stirred at 110°C for 24 hours. Then, DMF was removed under reduced pressure, and the residue was dissolved in acetone. This solution was stored at 4 °C for 2 days, and the formed precipitate was collected by filtration and washed with three portions of cold acetone, yielding compound of formula 1 as a dark red solid (320 mg, yield: 44%). LR-MS (ESI) *m*/*z* calcd. for C₂₄H₁₅Cl₂N₄O₈Ru⁻ [M-H]⁻: 660.9; found: 660.8.

Complex compound of formula **Xllla'.** Compound **IIc** (31 mg, 0.063 mmol, 1.05 equiv.) and compound **29** (40 mg, 0.060 mmol, 1.0 equiv.) were dissolved in EtOH (5 mL) and the reaction mixture was stirred overnight at 85 °C. The solvent was evaporated to dryness and the product was purified by silica column chromatography starting with ACN and increasing the eluent polarity with NaCl 0.1 M (0-15%) to obtain complex compound of formula **XIIIa'** as a dark purple solid (38 mg, yield: 55%). LR-MS (ESI) *m*/*z* calcd. for C₅₂H₃₇F₃N₈O₉Ru²⁺ [M]²⁺: 538.1; found: 538.1.

Complex compound of formula **XIIIb**'. Compound **IIm** (33 mg, 0.064 mmol, 1.05 equiv.) and compound **29** (40 mg, 0.060 mmol, 1.0 equiv.) were dissolved in EtOH (5 mL) and the reaction mixture was stirred overnight at 85 °C. The solvent was evaporated to dryness and the product was purified by silica column chromatography starting with ACN and increasing the eluent polarity with NaCl 0.1 M (0-12%) to obtain complex compound of formula **XIIIb'** as a dark green solid (30 mg, yield: 42%). LR-MS (ESI) *m*/*z* calcd. for C₅₄H₃₉F₃N₈O₉Ru²⁺ [M]²⁺: 551.1; found: 551.3.

Complex compound of formula **XIVa'.** Compound **Va** (29.5 mg, 0.064 mmol, 1.05 equiv.) and compound **29** (40 mg, 0.060 mmol, 1.0 equiv.) were dissolved in EtOH (5 mL) and the reaction mixture was stirred overnight at 85 °C. The solvent was evaporated to dryness and the product was purified by silica column chromatography starting with ACN and increasing the eluent polarity with NaCl 0.1 M (0-12%) to obtain complex compound of formula **XIVa'** as a dark green solid (25 mg, yield: 40%). LR-MS (ESI) *m*/*z* calcd. for C₅₄H₄₄N₈O₉Ru²⁺ [M]²⁺: 525.1; found: 524.9.

Complex compound of formula **XIIIc.** To a stirred solution of compound **XIIIa'** (20 mg, 0.017 mmol, 1.0 equiv.), *N*-hydroxysuccinimide (NHS) (3 mg, 0.026 mmol, 1.5 equiv.) and 1-ethyl-3-carbodiimide hydrochloride (EDC HC!) (5 mg, 0.026 mmol, 1.5 equiv.) in DMF (8 mL) was added 11-azido-3,6,9-trioxaundecan-1-amine (5 mg, 0.022 mmol, 1.25 equiv.). After stirring overnight at rt, the resulting mixture was concentrated under vacuum to give a residue which was purified by silica gel column chromatography (DCM/MeOH: 0-50%) to give complex **XIIIc** as a dark purple solid (11 mg, yield: 47% yield). LR-MS (ESI) m/z calcd. for C₆₀H₅₃F₃N₁₂O₁₁Ru²⁺ [M]²⁺: 638.2; found: 638.1.

Complex compound of formula **XIIId**. To a stirred solution of compound **XIIIb'** (20 mg, 0.017 mmol, 1.0 equiv.), NHS (3 mg, 0.026 mmol, 1.5 equiv.) and EDC·HCl (5 mg, 0.026 mmol, 1.5 equiv.) in DMF (8 mL) was added propargyl amine (1 mg, 0.020 mmol, 1.25 equiv.). After stirring overnight at rt, the resulting mixture was concentrated under vacuum to give a residue which was purified by silica gel column chromatography (DCM/MeOH: 0-50%) to give complex Xllld as a dark green solid (15 mg, yield: 73% yield). LR-MS (ESI) m/z calcd. for C₅₇H₄₂F₃N₉O₈Ru²+ [M]²⁺: 569.6; found: 569.4.

### Example 4. Examples of conjugated metal complex compounds according to the invention.

Although PDT can be considered an inherently selective approach due to the spatiotemporal control provided by light, PSs exhibiting cancer cell specificity offer an additional advantage to minimize toxic side effects that can occur upon accumulation in surrounding healthy tissues. In this context, receptor-binding peptides whose receptors are overexpressed in cancer cells compared to healthy cells offer great potential in targeted anticancer strategies. We have exemplified the present invention by synthesizing 5 conjugated metal complex compounds by using different peptides whose receptors are overexpressed in the cell membrane of different tumour cells.

Firstly, as described in example 5, once the phototoxic activity of the metal complexes compounds Ic and Im was validated *in vitro* against glioblastoma cells under hypoxic conditions upon irradiation with light of the phototherapeutic window (660 nm far-red light and 760 nm NIR light), we focused on the conjugation of the compounds to targeting ligands to confer them specificity towards glioblastoma cells. αᵥβ₃ belongs to the integrin subtypes that recognize the tripeptide sequence Arg-Gly-Asp (RGD) found in many extracellular matrix (ECM) proteins, including fibronectin or vitronectin, and is abundantly expressed in high-grade brain tumors. A plethora of studies highlighted the role of αᵥβ₃ in sustaining a high proliferative rate, migrative and invasive properties of glioblastoma, as well as promoting angiogenesis. Therefore, conjugation of photosensitizers based on metal complex compounds to RGD-containing peptides will deliver them specifically to glioblastoma cells, which would facilitate *in vivo* evaluation.

Secondly, since somatostatin subtype 2 receptor (SSTR2) is highly expressed in a wide variety of human tumors, including breast cancer, we have also conjugated the PSs to Octreotide (OCT), an FDA-approved cyclooctapeptide exhibiting high affinity and specificity towards SSTR2.

Thirdly, a selected lead PS has been conjugated to peptide-based ligands with high affinity and specificity for the melanocortin 1 receptor (MC1R), which is highly expressed in uveal melanoma cells, another example of hypoxic tumor.

Glioblastoma and uveal melanoma are considered unmet medical needs because there are no approved drug therapies for the first line treatment and despite advances in treatments, prognosis of both diseases remains bleak.

### General procedure for the preparation of conjugates between metal complex compounds and receptor-binding peptides

The required azido-peptides **RGD, OCT, L1MC1R, L2MC1R** and **L3MC1R** were assembled on a solid support following Fmoc/tBu methodology and purified by reversed-phase HPLC after acidic cleavage and deprotection.
Azido peptide **RGD:** c(RGFfK[COCH₂N₃])
   LR-ESI MS (ESI): *m*/*z* 687.6 calc. for [C₂₉H₄₂N₁₂O₈+H]⁺: 687.33, [C₈₅H₈₈F₃N₂₁O₁₀Ru+2H]²⁺: 344.17
Azido peptide **OCT:** N₃-CH₂-CONH-CH₂-CH₂-O-CH₂-CH₂-O-CH₂-CO-D-Phe-Cys-Phe-D-Trp-Lys-Thr-Cys-Thr-ol [S-S]
   LR-ESI MS (ESI): *m*/*z* 624.4 calc. for [C₅₇H₇₆N₁₄O₁₄S₂+2H]²⁺: 624.27.
Azido peptide **L1MC1R:** 4-Phenylbutyric-His-D-Phe-Arg-Trp-Gly-Lys(CO-CH₂-N₃)-NH₂
   LR-ESI MS (ESI): *m*/*z* 529.9 calc. for [C₅₂H₆₇N₁₇O₈+2H]²⁺: 529.78.
Azido peptide **L2MC1R:** 4-Phenylbutyric-His-D-Phe-Arg-Trp-Gly-Gly-Lys(CO-CH₂-N₃)-NH₂
   LR-ESI MS (ESI): *m*/*z* 558.4 calc. for [C₅₄H₇₀N₁₈O₉+2H]²⁺: 558.29.
Azido peptide **L3MC1R:** 4-Phenylbutyric-His-D-Phe-Arg-Trp-β-Ala-β-Ala-Lys(CO-CH₂-N₃)-NH₂
   LR-ESI MS (ESI): *m*/*z* 572.4 calc. for [C₅₆H₇₄N₁₈O₉+2H]²⁺: 572.30.

For the bioconjugation reaction, complex compound of formula **VIb** (1.0 mg, 1 µmol), the corresponding azido-peptide **RGD, OCT, L1MC1R, L2MC1R** or **L3MC1R** (0.5 µmol) and THPTA (3.6 mg, 10 µmol) were dissolved in a 1:1 (v/v) mixture H₂O/MeOH (400 µL). Then, lutidine (2 µL, 20 µmol) and [Cu(ACN)₄]PF₆ (5.1 mg, 10 µmol) were subsequently added. The reaction mixture was stirred at RT for 16 h. The solvent was evaporated to dryness and the Ru-peptide conjugate was purified by reversed phase HPLC. The title compound was obtained as dark green solid after lyophilization.

Ru-RGD conjugate **(XVIa).** LR-ESI MS (ESI): *m*/*z* 860.1, 574.2, 431,0 calc. for [C₈₅H₈₈F₃N₂₁O₁₀Ru]²⁺: 860.80, [C₈₅H₈₈F₃N₂₁O₁₀Ru+H]³⁺: 574.20, [C₈₅H₈₈F₃N₂₁O₁₀Ru+2H]⁴⁺: 430.90.

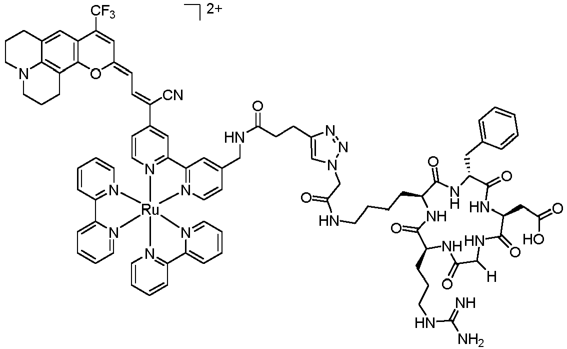

Ru-OCT conjugate **(XVIb).** LR-ESI MS (ESI): *m*/*z* 760.9, 571.2 calc. for [C₁₁₃H₁₂₄F₃N₂₃O₁₆RuS₂+H]³⁺:761.27, [C₁₁₃H₁₂₄F₃N₂₃O₁₆RuS₂+2H]⁴⁺: 571.20.

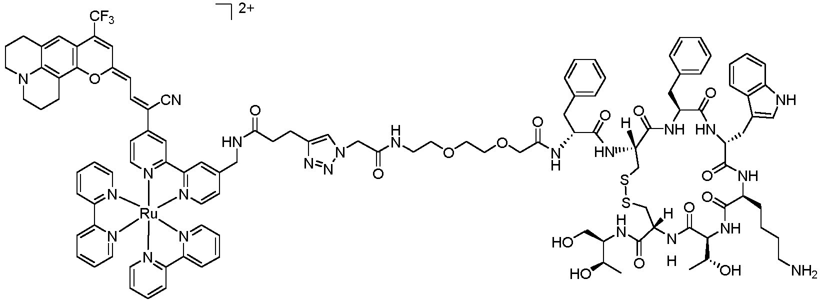

Ru-L1MC1R conjugate **(XVIc).** LR-ESI MS (ESI): *m*/*z* 697.9, 523.8 calc. for [C₁₀₈H₁₁₃F₃N₂₆O₁₀Ru+H]³⁺:698.27, [C₁₀₈H₁₁₃F₃N₂₆O₁₀Ru+2H]⁴⁺: 523.96.

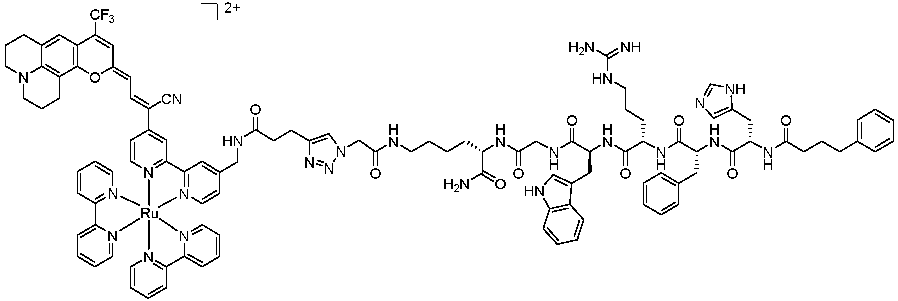

Ru-L2MC1R conjugate **(XVId).** LR-ESI MS (ESI): *m*/*z* 717.5, 538.0 calc. for [C₁₁₀H₁₁₆F₃N₂₇O₁₁Ru+H]³⁺:717.28, [C₁₁₀H₁₁₆F₃N₂₇O₁₁Ru+2H]⁴⁺: 538.21.

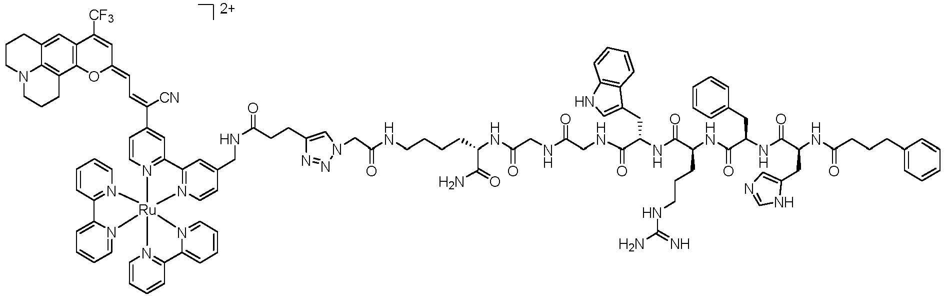

Ru-L3MC1R conjugate **(XVIe).** LR-ESI MS (ESI): *m*/*z* 726.5, 544.7 calc. for [C₁₁₂H₁₂₀F₃N₂₇O₁₁Ru+H]³⁺: 726.29, [C₁₁₂H₁₂₀F₃N₂₇O₁₁Ru+2H]⁴⁺: 544.97.

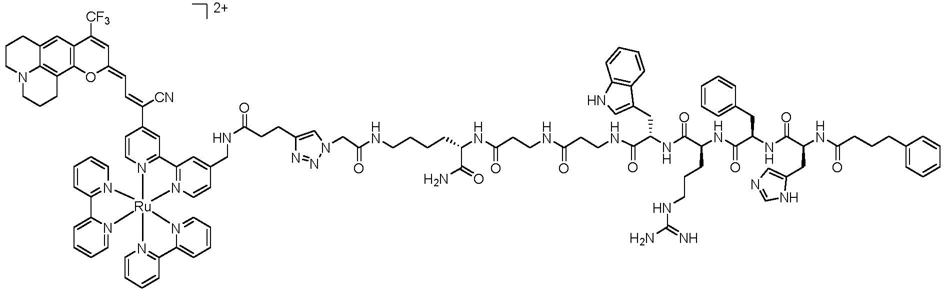

### Example 5. Phototoxicity evaluation of complex compounds of formula Ic and Im

The phototoxicity of complex compounds Ic and Im was evaluated under hypoxic (5% O₂) conditions against two human glioblastoma cancer cells, HCB-GIC20 and U87-MG, using a resazurin-based fluorometric cell viability assay. Obtained results are presented in accompanying Tables 1-2. For all experiments, the phototoxic index (PI) was calculated as: PI = IC₅₀ (dark-non-irradiated cells) / IC₅₀ (irradiated cells).

### Cell lines and cell culture conditions.

Glioblastoma-initiating cells (GICs) were isolated from glioblastoma (GB) biopsies obtained at Hospital Clinic Barcelona (HCB) with written informed consent from patients, in compliance with institutional guidelines. The HCB-GIC20 cell line was established through *in vitro* propagation of patient-derived glioblastoma cells as tumorsphere cultures in suspension. Cells were cultured in complete Neurobasal (NB) medium under hypoxic conditions (5% CO₂, 5% O₂, 37°C) in a humidified Heracell 150i incubator (ThermoFisher). Tumorspheres formed over 2-3 weeks were transferred to fresh flasks, with partial media changes every 3-4 days, gradually transitioning to a more complete Neuronal Stem Cell (NSC) medium. This medium comprised Dulbecco's Modified Eagle Medium and Nutrient Mixture F-12 (DMEM/F12, Invitrogen; 11320-033), 2 mM Glutamax, 10,000 units/mL-10,000 µg/mL P/S, N2 supplement (Invitrogen, 17502-048), 4.5% glucose (Sigma, G8769-100ML, Madrid, Spain), 1M HEPES (Sigma, H0887-100ML, Madrid, Spain), 2% BSA (Sigma, A7906-50G, Steinheim, Germany), 20 ng/mL FGF-2, and 20 ng/mL EGF. The HCB-GIC20 cell line is characterized as GB IDH-wild type, p53-wild type, and MGMT subtype. It grows as tumorspheres in non-laminin-coated plates and adheres on laminin-coated plates (7.5 mg/mL, Sigma, St. Louis, MO, USA). Human glioblastoma U87-MG cells were obtained from Cytion and cultured in DMEM supplemented with 10% (v/v) fetal bovine serum (FBS) and 1% (v/v) penicillin-streptomycin (100 U/mL penicillin, 100 µg/mL streptomycin). In both cases, the cells were maintained under hypoxic conditions (5% CO₂, 5% O₂, 37°C) in a humidified Heracell 150i incubator (ThermoFisher) for at least 2 weeks before starting the (photo)cytotoxicity experiments.

### Phototoxicity evaluation of complex compounds of formula Ic and Im, in 2D monolayer cells under hypoxia (5% O₂).

Cells were seeded in triplicate into 96-well plates at a density of 12,000 cells per well for HCB-GIC20 (on laminin-coated plates) and 4,500 cells per well for U87-MG. Each well received 100 µL of the appropriate cell culture medium (NSC medium for HCB-GIC20, and DMEM supplemented with 10% FBS and 1% P/S for U87-MG), and the plates were incubated under hypoxic conditions (5% CO₂, 5% O₂, 37°C).

Twenty-four hours after seeding, the cells were treated with serial dilutions of the test compounds in the respective culture medium, achieving final concentrations ranging from 0.01 to 150 µM. Each well was treated with 100 µL of the solution, containing less than 1% DMSO (v/v). The cells were then incubated in the dark for 2 hours (HCB-GIC20) or 4 hours (U87-MG), after which the medium was refreshed.

To evaluate the phototoxicity of the test compounds, cells were exposed to light at 660 nm (spectral half-width: 18 nm, 60 minutes, 3.8 ± 0.4 mW/cm², 13.7 J/cm²) or 760 nm (spectral half-width: 30 nm, 60 minutes, 3.1 ± 0.2 mW/cm², 11.2 J/cm²). Irradiation was performed using a device equipped with 96 AIGalnP LEDs of the specified wavelengths (LED660N-03 or LED760-03AU, Roithner Lasertechnik, Vienna, Austria). The device was positioned above the 96-well plates, with the LEDs directed towards the cells, ensuring each LED irradiated a single well from approximately 0.5 cm away. Additionally, a dark control plate was included in each experiment, where cells were not exposed to irradiation, to assess the dark cytotoxicity of the test compounds.

After a 36-hour recovery period at 5% O₂ without treatment, the medium was removed by aspiration. Each well was then filled with 100 µL of the appropriate cell culture medium containing resazurin (0.2 mg/mL), and the cells were incubated for 4 hours at 37°C under normoxic conditions (21% O₂). Following incubation, the fluorescence of the resofurin product (λₑₓ = 540 nm; λₑₘ = 590 nm) was measured using an Infinite 200 PRO Microplate Reader (TECAN). Fluorescence data were normalized and analyzed using GraphPad Prism software, and IC₅₀ values were determined by fitting the data to a non-linear regression model.

**Table 1. Phototoxicity of complex compounds of formula Ic and Im, towards U87-MG cancer cells, under and hypoxic (5% O₂) conditions.**

| | PpIX | | Complex compound of formula Ic | | Complex compound of formula Im | |
|---|---|---|---|---|---|---|
| λ (nm) | IC₅₀ (µM) | PI | IC₅₀ (µM) | PI | IC₅₀ (µM) | PI |
| Dark | 67.06 ± 8.05 | - | 181.70 ± 30.60 | - | 271.17 ± 42.71 | - |
| 660 | 1.18 ± 0.23 | 57 | 0.60 ± 0.06 | 303 | 1.74 ± 0.5 | 156 |
| 760 | 79.69 ± 7.56 | 1 | 68.88 ± 24.29 | 3 | 5.83 ± 1.43 | 47 |

**Table 2. Phototoxicity of complex compounds of formula Ic and Im, towards HCB-GIC20 cancer cells, under and hypoxic (5% O₂) conditions.**

| | PpIX | | Complex compound of formula Ic | | Complex compound of formula Im | |
|---|---|---|---|---|---|---|
| λ (nm) | IC₅₀ (µM) | PI | IC₅₀ (µM) | PI | IC₅₀ (µM) | PI |
| Dark | 146.72 ± 54.40 | - | 42.95 ± 3.86 | - | 102.82 ± 22.72 | - |
| 660 | 2.45 ± 0.42 | 60 | 0.13 ± 0.04 | 330 | 0.48 ± 0.05 | 214 |
| 760 | 159.63 ± 73.63 | 1 | 26.89 ± 1.24 | 2 | 2.82 ± 0.18 | 36 |

## Claims

1. A complex compound of formulae VI, including stereoisomers and E/Z isomers: 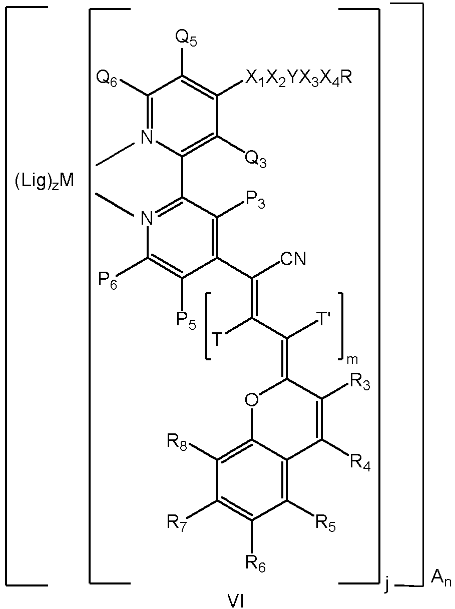
wherein M is a metal cation, and the metal cation M is Ru²⁺;
wherein radicals T and T' at each occurrence are each one a radical independently selected from the group consisting of: H and (C1-C3)-alkyl;
wherein m = 0, 1, 2, 3 or 4;
wherein when m ≥ 1 (such 1, 2, 3 or more), if radical R₃ is CH₂ it can preferably form a 6-membered ring with the T' radical closest to the coumarin backbone provided that T' = CH₂; and
wherein A is an anion from a pharmaceutically acceptable acid; n being an integer or fractional number whereby the compound of formula VI is electrically neutral; j = 1, 2, or 3; and z = 0, 1, or 2; on the proviso that j + z = 3;
wherein
X₁, X₂, X₃, X₄ are each one a radical independently selected from the group consisting of H, Ph, (CH₂)ₙ, (O-CH₂-CH₂)ₙ, (CH₂-CH₂-O)ₙ n=1-200
Y = F, Cl, Br, I, O, S, S-S, NH, C(=O), CH(=O), OC(=O), C(=O)O, NHC(=O), C(=O)NH, CN, SC(=O), C(=O)S, NHC(=O)O, OC(=O)NH, NHC(=O)NH, C(=O)NH-NH, C(=O)NH-N=CH
R=N₃, 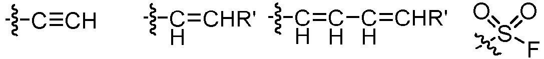 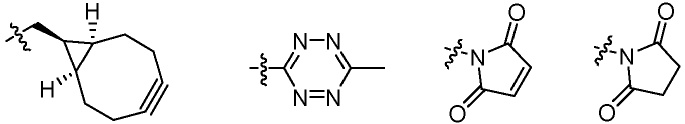 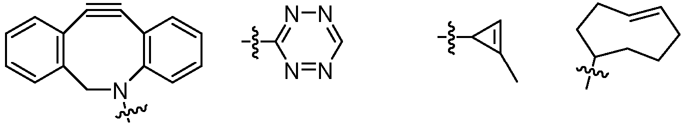 wherein any one of X₁, X₂, X₃, X₄, Y and R can be optionally absent; preferably, wherein any one of X₁, X₂, X₃, X₄, and Y can be optionally absent thus leaving radical R as mandatory for bioconjugation reactions such as any biorthogonal reaction making use of radical R, including click chemistry; also preferably any one of X₁, X₂, X₃, X₄ , Y and R can be optionally absent with the proviso that at least one of X₁, X₂, X₃, X₄, or Y is present so to allow the formation of conjugates by amide, ester, thioester, carbonate, thiocarbonate, hydrazone, oxyme, carbamate and urea bond formation, as well as by Michael addition reactions, but not limited to;
wherein radicals P₃ , P₅ , P₆ , Q₃ , Q₅ , and Q₆, at each occurrence are each one a radical independently selected from the group consisting of: H , (C1-C3)-alkyl , (C3-C6)-cycloalkyl , CH=CHR' , CF₃, CHF₂, CH₂F, CF₂CF₃ , F , CI , Br, I , OR' , C(=O)OR' , O(C=O)R' , C(=O)NR', NR'C(=O)R" , NR'R" , CN , NO₂ , phenyl, mono-substituted phenyl, di-substituted phenyl, and tri-substituted phenyl; pyridine, mono-substituted pyridine, di-substituted pyridine, and tri-substituted pyridine; wherein substituents on the phenyl and pyridine rings are attached to any of the possible substitution positions, and they are independently selected from the group consisting of: F , CI , Br, I , NO₂ , (C1-C3)-alkyl , OH , O[(C1-C3)-alkyl], CN , CF₃, CHF₂, CH₂F , CF₂CF₃, NH₂, NH[(C1-C3)-alkyl] , and N[(C1-C3)-alkyl]₂; and wherein Q₃ and P₃ can form a C=C bond when Q₃ and P₃ are both CH;
wherein Lig at each occurrence is a bidentate ligand independently selected from the group consisting of ligands of accompanying formulae: Lig 1, Lig 2, Lig 3, Lig 4, Lig 5, Lig 6, Lig 7, Lig 8, Lig 9, Lig 10, Lig 11, Lig 12, and Lig 13; 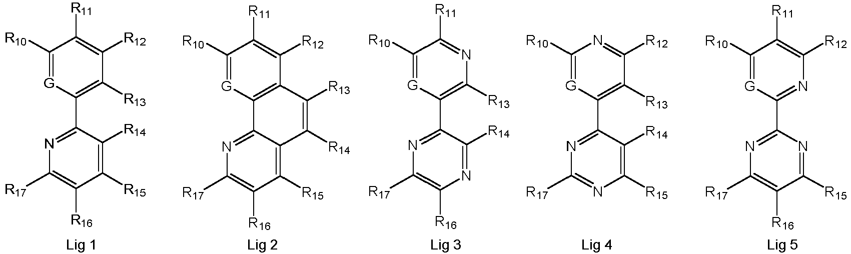 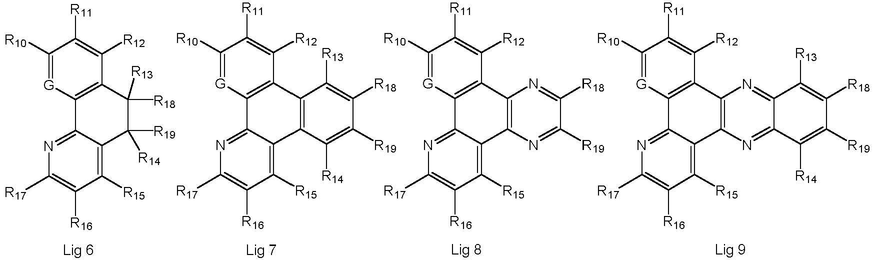 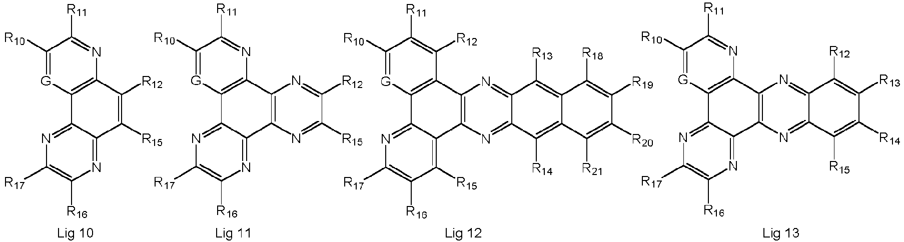
wherein G is either an N atom, or a C atom with one negative charge that is formed *in situ* from a C-H when a H⁺ is lost in the formation of the carbon-metal bond; and wherein R₁₀ , R₁₁ , R₁₂ , R₁₃ , R₁₄ , R₁₅ , R₁₆ , R₁₇ , R₁₈ , R₁₉ , R₂₀ and R₂₁ at each occurrence is each one a radical independently selected from the group consisting of: H , (C1-C3)-alkyl , (C3-C6)-cycloalkyl , CH=CHR' , CF₃, CHF₂, CH₂F, CF₂CF₃ , F , CI , Br, I , OR' , C(=O)OR' , O(C=O)R' , C(=O)NR', NR'C(=O)R" , NR'R" , CN , NO₂ , phenyl, mono-substituted phenyl, di-substituted phenyl, and tri-substituted phenyl; pyridine, mono-substituted pyridine, di-substituted pyridine, and tri-substituted pyridine; wherein substituents on the phenyl and pyridine rings are attached to any of the possible substitution positions, and they are independently selected from the group consisting of: F , CI , Br, I , NO₂, (C1-C3)-alkyl , OH , O[(C1-C3)-alkyl] , CN , CF₃, CHF₂, CH₂F , CF₂CF₃, NH₂, NH[(C1-C3)-alkyl], and N[(C1-C3)-alkyl]₂;
wherein R₃, R₅ , R₆ , and R₈ at each occurrence are each one a radical independently selected from the group consisting of: H, (C1-C3)-alkyl , (C3-C6)-cycloalkyl , CH=CHR' , CF₃, CHF₂, CH₂F, CF₂CF₃ , F , CI , Br, I , OR' , C(=O)OR' , O(C=O)R' , C(=O)NR', NR'C(=O)R" , NR'R" , CN , NO₂ , phenyl, mono-substituted phenyl, di-substituted phenyl, and tri-substituted phenyl; pyridine, mono-substituted pyridine, di-substituted pyridine, and tri-substituted pyridine; wherein substituents on the phenyl and pyridine rings are attached to any of the possible substitution positions, and they are independently selected from the group consisting of: F , CI , Br, I , NO₂, (C1-C3)-alkyl , OH , O[(C1-C3)-alkyl] , CN , CF₃, CHF₂, CH₂F , CF₂CF₃, NH₂ , NH[(C1-C3)-alkyl] , and N[(C1-C3)-alkyl]₂;
wherein radical R₇ is H, NO₂ , OR' , NR'R" , N(CH₂COOH)₂ , N(CH₂CH₂SO₃H)₂ , N(CH₂CONHCH₂CH₂NMe₂)₂ , or: an unsubstituted, a mono-(R')-substituted, a di-(R',R")-substituted, or a tri-(R',R",R‴)-substituted radical of each one of the heterocyclic radicals 1-aziridinyl, 1-azetidinyl, 1-pyrrolidinyl, 1-piperidinyl , 4-morpholinyl , or 1-piperazinyl; alternatively R₆ , R₇ and R₈ form together the bicycle system of the accompanying formula, wherein V₁ , V₂ , V₃, V₄, V₅, V₆ , V₇ , V₈ , V₉ , V₁₀ , V₁₁ , and V₁₂ are each one a radical independently selected from the group consisting of H and (C1-C3)-alkyl; 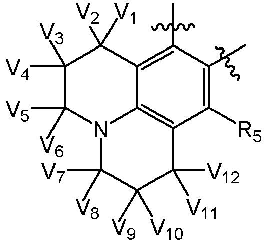
wherein radical R₄ is H , (C1-C3)-alkyl , (C3-C6)-cycloalkyl , CF₃, CHF₂, CH₂F, CF₂CF₃ , F , CI , Br , I , OR' , C(=O)OR' , O(C=O)R' , C(=O)NR' , NR'C(=O)R" , NR'R" , phenyl, a mono-(R')-substituted phenyl, a di-(R', R")-substituted phenyl, a tri-(R', R", R''')-substituted phenyl, or a radical CE₁E₂E₃ ; wherein: E₁ and E₂ are radicals independently selected from the group consisting of: H , F , CI , Br, I , OR' , NR'R" , NO₂, (C1-C3)-alkyl , phenyl, and a mono-(R')-substituted phenyl; and E₃ is OH, O[(C1-C3)-alkyl] , or
a radical of the accompanying formula wherein p = 0, 1, 2, 3 or 4; and D₁ and D₂ at each occurrence are radicals independently selected from the group consisting of H , O[CH₂]_{q}-X , S[CH₂]_{q}-X , and N[[CH₂]_{q}-X]₂ , being q = 1 or 2, and X = F, Cl, Br or I. 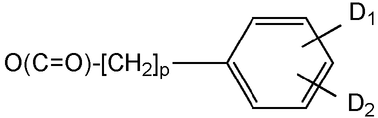
or a radical of the accompanying formula wherein p = 1, 2, 3 or 4; and D₃ is a radical independently selected from the group consisting of H , H(C=O) , [(C1-C3)-alkyl](C=O) , (OH)₂P(=O)O-CH₂-O(C=O) , (PhO)(OH)P(=O) 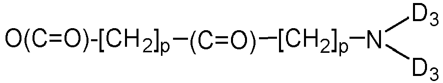 wherein R', R" and R‴ at each one of the above-mentioned occurrences are each one a radical independently selected from the group consisting of: H , F , Cl , Br, I , NO₂ , (C1-C3)-alkyl, OH, O[(C1-C3)-alkyl] , NH₂, NH[(C1-C3)-alkyl], and N[(C1-C3)-alkyl]₂.

2. The complex compound of formulae VI according to claim 1, wherein the complex compound is of formulae VII: 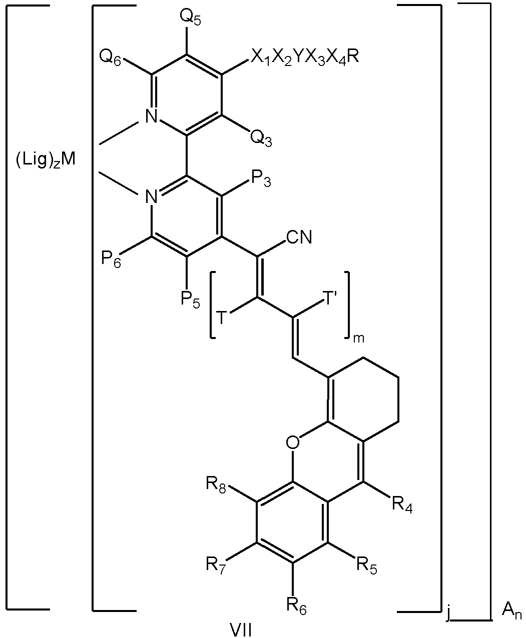 wherein Lig, A, n, j, z, M, m, X₁, X₂, X₃, X₄, Y, R, P₃, P₅, P₆, Q₃, Q₅ , Q₆, T, T' and R₄ to R₈ are as defined in claim 1.

3. The complex compound according to any one of the previous claims, wherein the compound is selected from any one of the list consisting of: 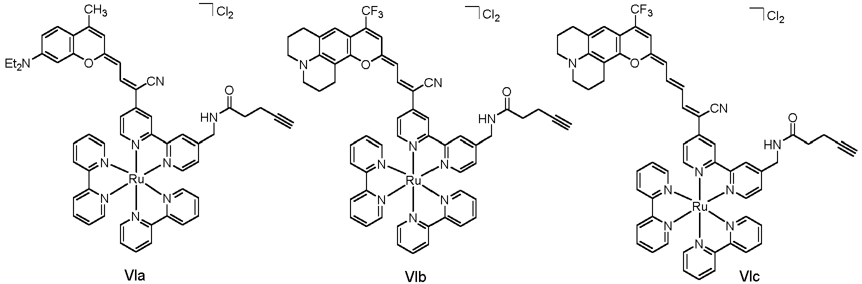 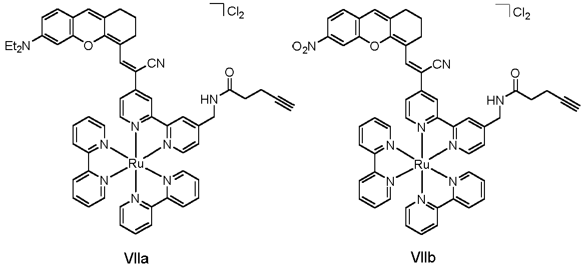 including stereoisomers and *E*/*Z* isomers thereof.

4. A complex compound of formula XVI or XVII, including stereoisomers and *E* / *Z* isomers: 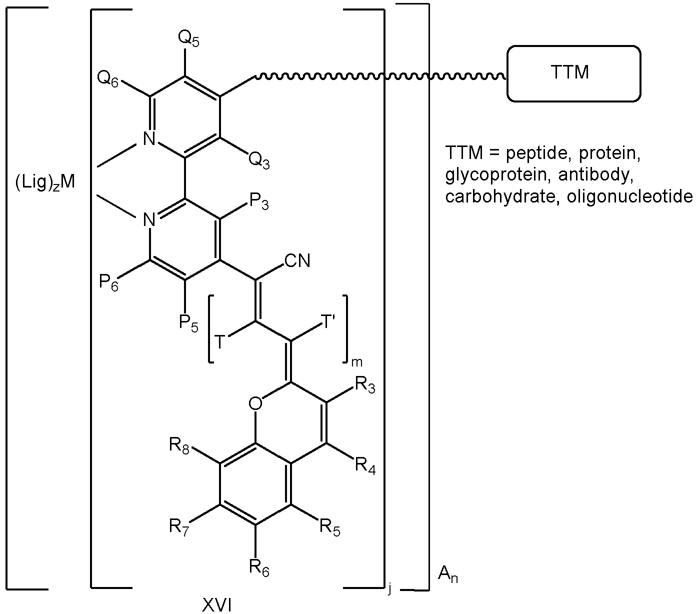 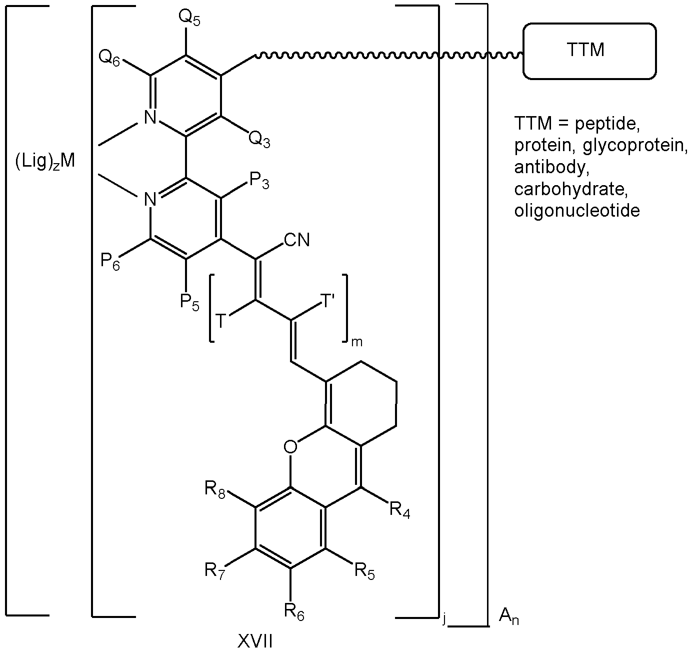 wherein Lig, M, A, n, j, z, m, P₃ , P₅ , P₆ , Q₃ , Q₅ , Q₆, T, T', R₃ (the definition of R₃ is only applicable to formula XVI as formula XVII does not comprise this specific radical), and R₄ to R₈ are as defined in any of the previous claims, and wherein TTM is a tumour-targeting moiety or biomolecule selected from the list consisting of a peptide, protein, glycoprotein, antibody, carbohydrate, oligonucleotide or analogues comprising a functional group selected from the list consisting of azide, alkene, 1,4-diene, linear or cyclic alkyne, maleimide, succinimide, tetrazine, ciclopropene, hydrazine, NH₂, COOH, CHO, or SH that can react with the complementary group of the chemical moiety X₁X₂YX₃X₄R of any one of metal complex compounds of formula VI or VII, through any suitable bioconjugation reaction including amide, ester, thioester, carbonate, thiocarbonate, hydrazone, oxyme, carbamate and urea bond formation, Michael additions and cycloaddition reactions.

5. The complex compound of formula XVI or XVII according to claim 4, wherein said compound is selected from the list consisting of:
a. Ru-RGD conjugate (XVIa) 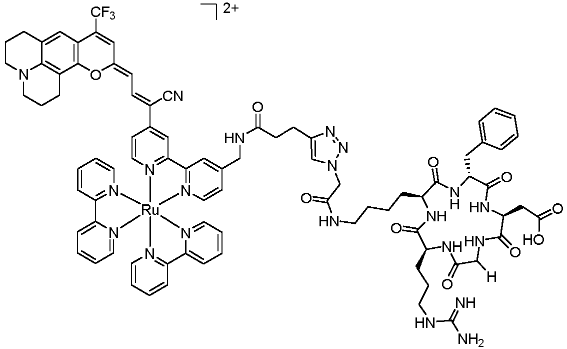
b. Ru-octreotide conjugate (XVIb) 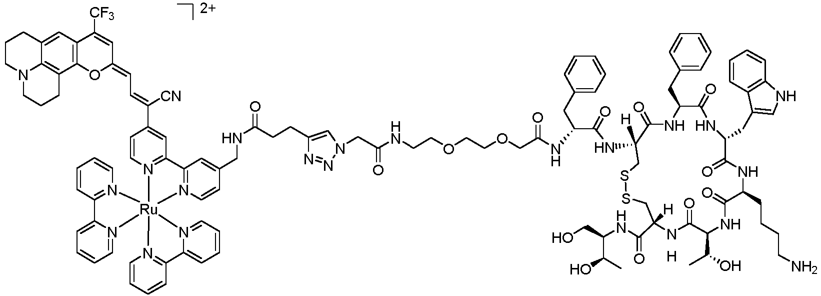
c. Ru-L1MC1R conjugate (XVIc) 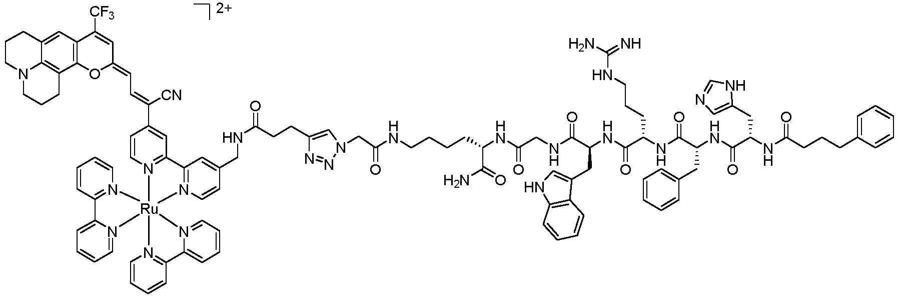
d. Ru-L2MC1R conjugate (XVId) 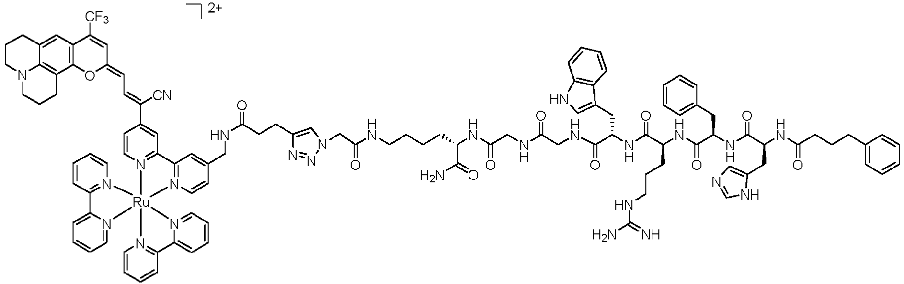
e. Ru-L3MC1R conjugate (XVIe)

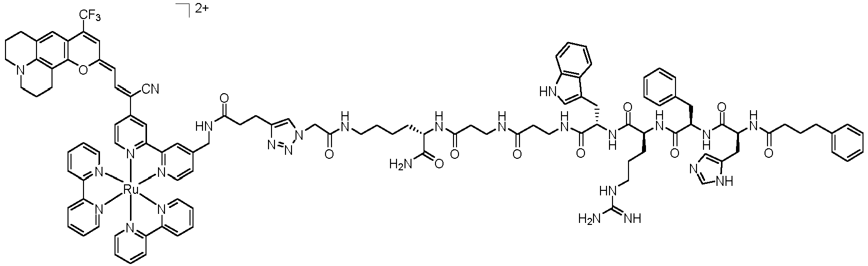

6. The complex compound of any one of claims 1 to 5, wherein the Lig is Lig 1, and the G of Lig 1 is an N atom.

7. A process for manufacturing a complex compound of formulae VI: 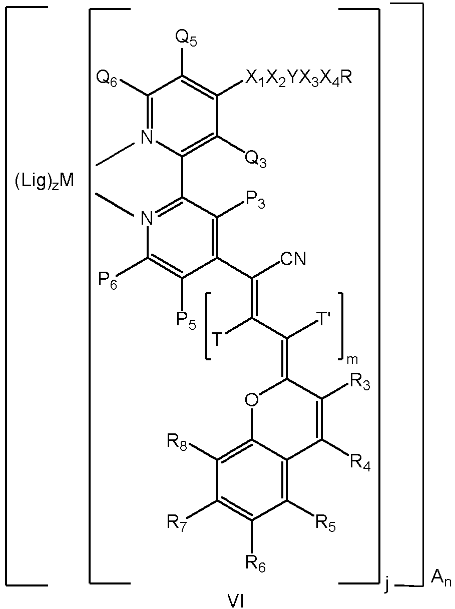
wherein the process for manufacturing complex compounds of formulae VI comprises the steps of:
a. contacting a complex compound of formula VIII as defined below, 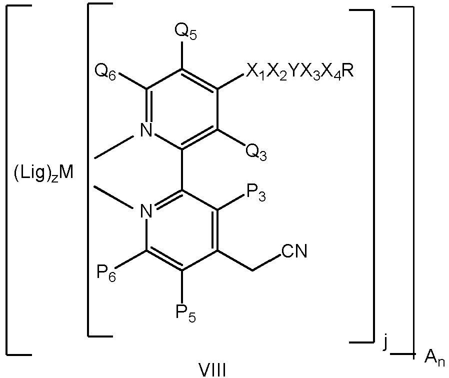 wherein the radicals of formula VIII are as defined in any of the previous claims;
with a coumarin of formula X, or XI, wherein radicals R₄, R₅, R₆, R₇, R₈, m, T and T' of any of formulae X or XI are as defined in any of the previous claims, and preferably a base (e.g. piperidine) in a solvent or solvent mixture under a normal or an inert atmosphere, and carrying out a condensation reaction to form the complex compound of formula VI; and
b. optionally filtering, concentrating and/or purifying the complex compound of formula VI; 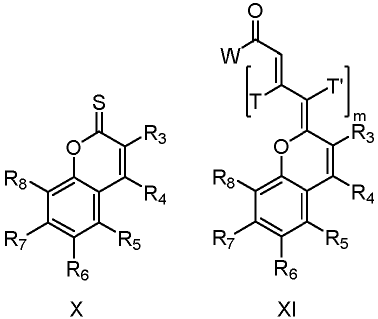 wherein W is selected from any of: H, (C1-C3)-alkyl, CF₃, or CF₂CF₃

8. A process for manufacturing a complex compound of formulae VII: 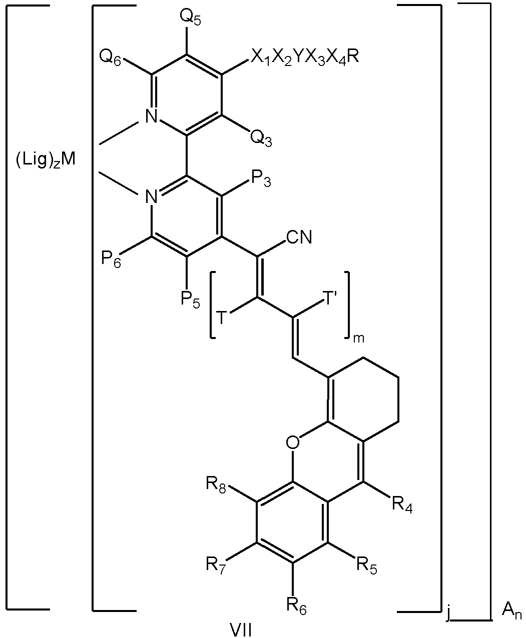 wherein the process for manufacturing complex compounds of formulae VII comprises the steps of:
a. contacting a complex compound of formula VIII as defined above, with a coumarin of formula XII, wherein radicals R₃, R₄, R₅, R₆, R₇, R₈, m, T, T' and W of formula XII are as defined in any of the previous claims, and preferably a base (e.g. piperidine) in a solvent or solvent mixture under a normal or an inert atmosphere, and carrying out a condensation reaction to form the complex compound of formula VII; and 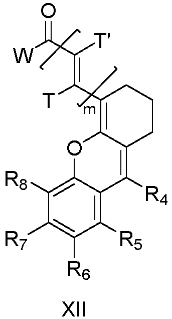
b. optionally filtering, concentrating and/or purifying the complex compound of formula VIII.

9. A process for manufacturing a complex compound of formula VIII: 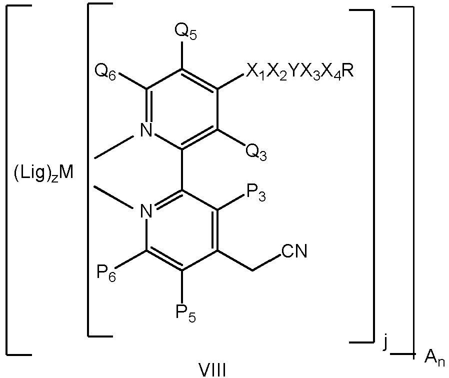 wherein the process comprises the steps of
a. Dissolving a ligand compound of formula IX 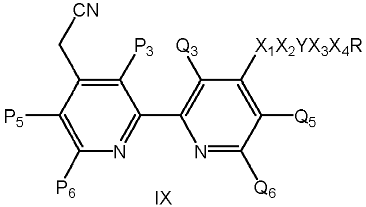 wherein X₁, X₂, X₃, X₄, Y, R, P₃ , P₅ , P₆ , Q₃ , Q₅ , and Q₆ are as defined in any of the previous claims with a compound of formula [M(Lig)_{z}]Aₙ in a solvent or solvent mixture under a normal or an inert atmosphere to carry out a coordination reaction to form the complex compound of formula VIII, in which the reactant compounds of formulae IX and [M(Lig)_{z}]Aₙ are reacted in preferably molar amount ratios; and
b. optionally filtering, concentrating and/or purifying the resulting complex compound of formula VIII.

10. An intermediate chemical compound of chemical formulae VIII or IX.

11. A method for preparing complex compounds of formula XVI or XVII, wherein these compounds are conjugated to a TTM or biomolecule, which comprises the steps of:
a. reacting a peptide, protein, glycoprotein, antibody, carbohydrate or oligonucleotide bearing a suitable functional group and linker with the complementary group of the chemical moiety X₁X₂YX₃X₄R of metal complex compounds of formula VI or VII defined above, preferably via a bioorthogonal reaction, catalysed or not by copper, in a solvent or solvent mixture under a normal o inert atmosphere, preferably in a controlled pH, and, when required, using a coupling reagent or additive; and
b. optionally filtering, concentrating and/or purifying the complex compound of formula XVI or XVII.

12. A pharmaceutical composition comprising a therapeutically effective amount of a compound as defined in any one of claims 1 to 6, together with appropriate amounts of excipients, carriers or vehicles.

13. A compound as defined in any one of claims 1 to 6 or the pharmaceutical composition of claim 12, for use in therapy, preferably for use in human therapy.

14. A compound as defined in any one of claims 1 to 6 or the pharmaceutical composition of claim 12, for use as photosensitizer in photodynamic therapy of a human condition, such as cancer, a skin condition, a fungal infection, or a microbial infection.

15. The compound for use according to claim 14, wherein the use is as a photosensitizer in photodynamic therapy for the treatment of glioblastoma, or uveal melanoma.
